(19) Europäisches Patentamt
European Patent Office
Office européen des brevets

(11) EP 3 159 013 A1

(12) EUROPEAN PATENT APPLICATION

(43) Date of publication:
**26.04.2017 Bulletin 2017/17**

(51) Int Cl.:
***A61K 47/68*** *(2017.01)*

(21) Application number: **16203350.0**

(22) Date of filing: **11.10.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2012 US 201261717710 P**
**24.10.2012 US 201261717743 P**
**12.04.2013 US 201361811285 P**
**19.06.2013 PCT/GB2013/051593**

(62) Document number(s) of the earlier application(s) in accordance with Art. 76 EPC:
**13786530.9 / 2 911 700**

(71) Applicant: **Polytherics Limited**
**Babraham**
**Cambridge**
**CB22 3AT (GB)**

(72) Inventors:
- **GODWIN, Antony**
  **Cambridge, CN22 3AT (GB)**
- **FRIGERIO, Mark**
  **Cambridge, CN22 3AT (GB)**
- **BADESCU, George**
  **Cambridge, CN22 3AT (GB)**
- **BURT, John**
  **Cambridge, CN22 3AT (GB)**

(74) Representative: **Scott, Susan Margaret et al**
**Abel & Imray**
**20 Red Lion Street**
**London WC1R 4PQ (GB)**

(54) **DRUG-PROTEIN CONJUGATES**

(57) A conjugate of the general formula:

$$(((D'_{q'}\text{-}Lk^{1'})_m\text{-}P)_p\text{-}Lk^2\text{-}Lk^3)_n\text{-}Ab \qquad (I')$$

in which D' represents a drug moiety;
q' represents an integer from 2 to 10;
$Lk^{1'}$ represents a linker;
m represents an integer from 1 to 10;
P represents a z-valent group $-P^1$-NH- where z is from 2 to 11 and $P^1$ is a polyethylene glycol group;
p represents an integer from 1 to 10;
$Lk^2$ represents a bond or a y-valent linker where y is from 2 to 11 and which consists of from 1 to 9 aspartate and/or glutamate residues;
$Lk^3$ represents a linker of the general formula:

-CO-Ph-X-Y- (II)

in which Ph is an optionally substituted phenyl group; X represents a CO group or a CH.OH group; and Y represents a group of formula:

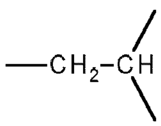
(III) or
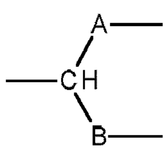
(IV)

EP 3 159 013 A1

in which each of A and B represents a $C_{1-4}$alkylene or alkenylene group;
Ab represents a binding protein or peptide capable of binding to a binding partner on a target, said binding protein being bonded to $Lk^3$ via two sulfur atoms derived from a disulfide bond in the binding protein or peptide; and
n represents an integer from 1 to s where s is the number of disulfide bonds present in the binding protein or peptide prior to conjugation to $Lk^3$;
the meanings of m, n, p, q', y and z being chosen such that the conjugate contains from 1 to 10 D' groups.

## Description

This invention relates to novel drug-protein conjugates.

The specificity of binding proteins for specific markers on the surface of target cells and molecules has led to their extensive use as carriers for a variety of diagnostic and therapeutic agents. For example, such proteins conjugated to labels and reporter groups such as fluorophores, radioisotopes and enzymes find use in labelling and imaging applications, while conjugation to cytotoxic agents and chemotherapy drugs allows targeted delivery of such agents to specific tissues or structures, for example particular cell types or growth factors, minimising the impact on normal, healthy tissue and significantly reducing the side effects associated with chemotherapy treatments. Such conjugates have extensive potential therapeutic applications in several disease areas, particularly in cancer.

Water soluble, synthetic polymers, particularly polyalkylene glycols, are widely used to conjugate therapeutically active molecules such as peptide or protein ligands, including antibodies. These therapeutic conjugates have been shown to alter pharmacokinetics favourably by prolonging circulation time and decreasing clearance rates, decreasing systemic toxicity, and in several cases, displaying increased clinical efficacy. The process of covalently conjugating polyethylene glycol, PEG, to proteins is commonly known as "PEGylation".

It is important for optimised efficacy and to ensure dose to dose consistency that the number of conjugated moieties per binding protein is the same, and that each moiety is specifically conjugated to the same amino acid residue in each binding protein. Accordingly, a number of methods have been developed to improve the homogeneity of such conjugates. Liberatore et al, Bioconj. Chem 1990, 1, 36-50, and del Rosario et al, Bioconj. Chem. 1990, 1, 51-59 describe the use of reagents which may be used to cross-link across the disulfide bonds in proteins, including antibodies. WO 2005/007197 describes a process for the conjugation of polymers to proteins, using novel conjugation reagents having the ability to conjugate with both sulfur atoms derived from a disulfide bond in a protein to give novel thioether conjugates.

Auristatins are antineoplastic agents, and are a highly potent, cell-killing class of drug. Various members of this class, including the natural product dolastatin 10 (isolated from Dolabella auricularia), monomethylauristatin E, monomethylauristatin F, monomethylauristatin D, auristatin PYE and auristatin PHE are being developed for use in the treatment of various diseases including cancer. They are highly toxic, and much research is currently being directed to the development of conjugates containing auristatins.

WO 2009/117531 is directed to drug conjugates which have auristatins linked via the C-terminus to a linker and thence to an antibody. These conjugates are stated to show efficacy without the need for a self-immolative group to release the drug. WO 2009/052431 relates to CD 19 binding agents, and discloses auristatin conjugates.

Two antibody drug conjugates have received regulatory approval: one is brentuximab vedotin, in which the drug is an auristatin, and one is trastuzumab emtansine, in which the drug is a maytansine. In both these commercially-available conjugates, the linkage of the drug to the antibody uses a linker based on maleimide. Maleimides are widely used in conjugating reagents. However, as with many other conjugating reagents, the use of maleimides presents a number of difficulties: control of the conjugation reaction is difficult, leading to products having low homogeneity, and stability of the resulting conjugates can be a problem.

Therefore there remains a need for improved auristatin-antibody conjugates with improved stability and homogeneity, which can be prepared effectively and which demonstrate the required efficacy.

Accordingly, the present invention provides an auristatin-containing conjugate which has the general formula:

$$(((Dq\text{-}Lk^1)_m\text{-}P)_p\text{-}Lk^2\text{-}Lk3)_n\text{-}Ab \qquad (I)$$

in which D represents an auristatin moiety;
q represents an integer from 1 to 10;
$Lk^1$ represents a linker;
m represents an integer from 1 to 10;
P represents a bond or a z-valent group $-P^1-NH-$ where z is from 2 to 11 and $P^1$ is a group containing at least one ethylene unit $-CH_2-CH_2-$ or ethylene glycol unit $-O-CH_2-CH_2-$;
p represents an integer from 1 to 10;
$Lk^2$ represents a bond or a y-valent linker where y is from 2 to 11 and which consists of from 1 to 9 aspartate and/or glutamate residues;
$Lk^3$ represents a linker of the general formula:

$$\text{-CO-Ph-X-Y-} \qquad (II)$$

in which Ph is an optionally substituted phenyl group; X represents a CO group or a CH.OH group; and Y represents a group of formula:

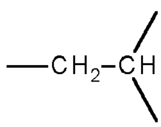 (III) or 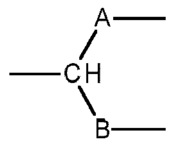 (IV)

in which each of A and B represents a $C_{1-4}$alkylene or alkenylene group;

Ab represents a binding protein or peptide capable of binding to a binding partner on a target, said binding protein being bonded to $Lk^3$ via two sulfur atoms derived from a disulfide bond in the binding protein or peptide; and

n represents an integer from 1 to s where s is the number of disulfide bonds present in the binding protein or peptide prior to conjugation to $Lk^3$;

the meanings of m, n, p, q, y and z being chosen such that the conjugate contains from 1 to 10 D groups.

D represents an auristatin moiety (i.e. the $Lk^1$ group is bonded to the residue of an auristatin). The term auristatin includes compounds such as auristatin D, auristatin E, auristatin F, monomethyl auristatin D, monomethyl auristatin E, monomethyl auristatin F, auristatin PYE auristatin PHE, the related natural product dolastatin 10, and derivatives thereof.

Preferably the auristatin is a compound containing the substructure (A)

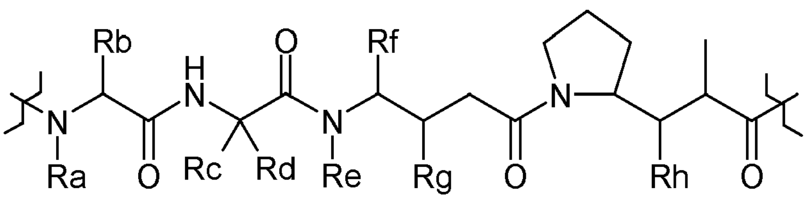

(A),

in which Ra represents $C_{1-8}$alkyl; Rb represents H, $C_{1-8}$alkyl, $C_{3-8}$ cycloalkyl or cycloalkenyl, aryl, -X-aryl, -X-$C_{3-8}$ cycloalkyl or cycloalkenyl, $C_{3-8}$heterocyclyl or -X-$C_{3-8}$heterocyclyl; Rc represents H or methyl; Rd represents H, $C_{1-8}$alkyl, $C_{3-8}$ cycloalkyl or cycloalkenyl, aryl, -X-aryl, -X-$C_{3-8}$heterocyclyl, -$C_{3-8}$heterocyclyl, -X-$C_{3-8}$heterocyclyl, -X-S-$C_{1-8}$alkyl; or Rc and Rd together form a carbocyclic ring of formula $-(CR^aR^b)_r-$, wherein $R^a$ and $R^b$ independently represent H or $C_{1-8}$alkyl and r is an integer of from 2 to 6; Re represents H or $C_{1-8}$alkyl; Rf represents H, $C_{1-8}$alkyl, $C_{3-8}$ cycloalkyl or cycloalkenyl, aryl, -X-aryl, -X-$C_{3-8}$ cycloalkyl or cycloalkenyl, $C_{3-8}$heterocyclyl or -X-$C_{3-8}$heterocyclyl; Rg represents H, -OH, -$C_{1-8}$alkyl, $C_{3-8}$ cycloalkyl or cycloalkenyl, or -O-($C_{1-8}$alkyl); Rh represents H, -OH, -$C_{1-8}$alkyl, $C_{3-8}$ cycloalkyl or cycloalkenyl, or -O-($C_{1-8}$alkyl); and each X is independently $C_{1-10}$alkylene.

Preferably Ra represents $C_{1-4}$alkyl, especially methyl. Preferably Rb represents $C_{1-4}$alkyl, especially isopropyl. Preferably Rc represents H. Preferably Rd represents $C_{1-4}$alkyl or $-CH_2CH_2SCH_3$, more preferably $-CH_2CH(CH_3)_2$, $-CH(CH_3)_2$ or $-CH_2CH_2SCH_3$, most preferably-$CH(CH_3)_2$. Preferably Re represents H or $C_{1-4}$alkyl; most preferably Re represents methyl. Preferably Rf represents $C_{1-4}$alkyl, especially 1-methylpropyl. Preferably Rg represents -OH or -O-($C_{1-4}$alkyl); most preferably Rg represents methoxy. Preferably Rh represents -OH or -O-($C_{1-4}$alkyl); most preferably Rh represents methoxy.

More preferably, the auristatin comprises the substructure (B)

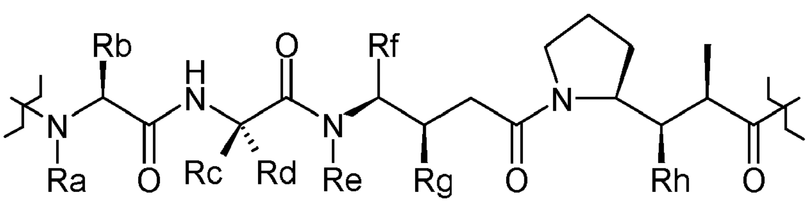

(B),

in which Ra-Rh have the meanings set out above.

Still more preferably, the auristatin comprises the substructure (C)

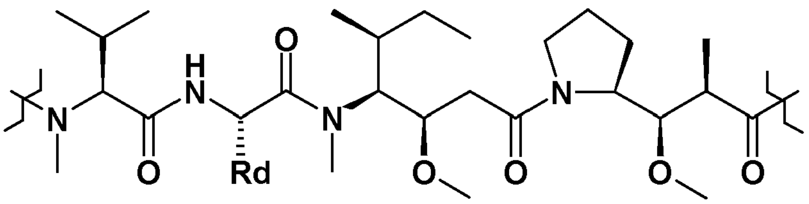

(C),

in which Rd represents $-CH(CH_3)_2$, $-CH_2CH(CH_3)_2$, or $-CH_2CH_2SCH_3$.

In some preferred embodiments, the auristatin N-terminal group is hydrogen or $C_{1-8}$alkyl, more preferably hydrogen or methyl. For example, the auristatin may be a compound that contains the substructure (D) or (E):

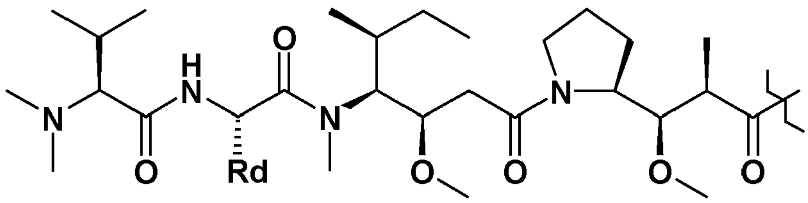

(D),

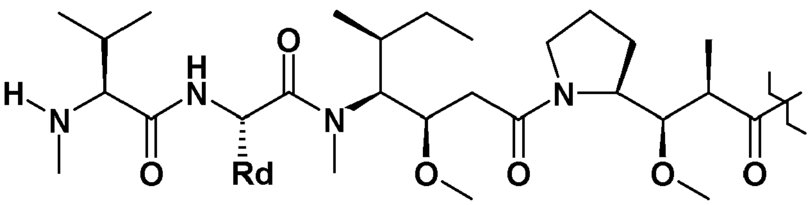

(E)

In some preferred embodiments, the auristatin C-terminal group is:

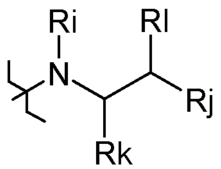

,

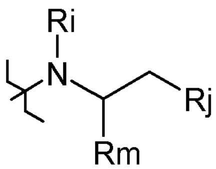

,

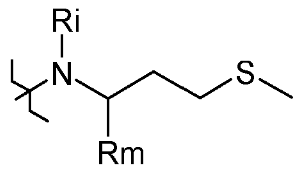

or

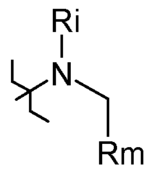

;

in which Ri represents hydrogen or $C_{1-8}$alkyl; Rj represents $C_{6-10}$aryl or $C_{5-10}$heteroaryl, said aryl or heteroaryl being optionally substituted with up to 3 substituents each independently selected from the group consisting of halogen, $C_{1-4}$alkyl optionally substituted with up to 3 halogens, hydroxy, $C_{1-4}$alkoxy and cyano; Rk represents hydrogen or $C_{1-8}$alkyl; Rl represents hydrogen or hydroxy; and Rm represents $-CO_2H$, $-CO_2C_{1-8}$alkyl, -CONH-$C_{6-10}$aryl, -CONH-$C_{5-10}$heteroaryl and $C_{5-10}$heteroaryl, said aryl or heteroaryl group being optionally substituted with up to 3 substituents each independently selected from the group consisting of halogen, $C_{1-4}$alkyl optionally substituted with up to 3 halogens, hydroxy, $C_{1-4}$alkoxy and cyano.

In some preferred embodiments the auristatin C-terminal group is

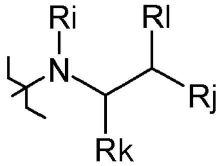

,

more preferably

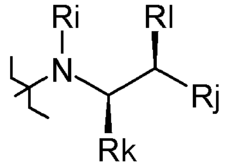

,

in which Ri represents hydrogen; Rj represents phenyl or $C_{5-10}$heteroaryl, said phenyl or heteroaryl being optionally substituted with up to 3 substituents each independently selected from the group consisting of halogen, $C_{1-4}$alkyl optionally substituted with up to 3 halogens, hydroxy, $C_{1-4}$alkoxy and cyano; and either Rk and Rl are both hydrogen, or Rk is methyl and Rj is hydroxy.

In some preferred embodiments the auristatin C-terminal group is

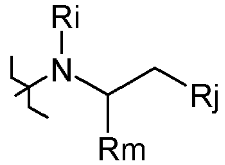

,

more preferably

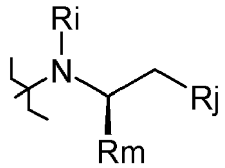

,

in which Ri represents hydrogen; Rj represents phenyl or $C_{5-10}$heteroaryl, said phenyl or heteroaryl being optionally substituted with up to 3 substituents each independently selected from the group consisting of halogen, $C_{1-4}$alkyl optionally substituted with up to 3 halogens, hydroxy, $C_{1-4}$alkoxy and cyano; and Rm represents $-CO_2H$, $-CO_2C_{1-8}$alkyl, or $C_{5-10}$heteroaryl, said heteroaryl being optionally be substituted with up to 3 substituents each independently selected from the group consisting of halogen, $C_{1-4}$alkyl optionally substituted with up to 3 halogens, hydroxy, $C_{1-4}$alkoxy and cyano.

For example, the auristatin may contain the substructure (F) or (G):

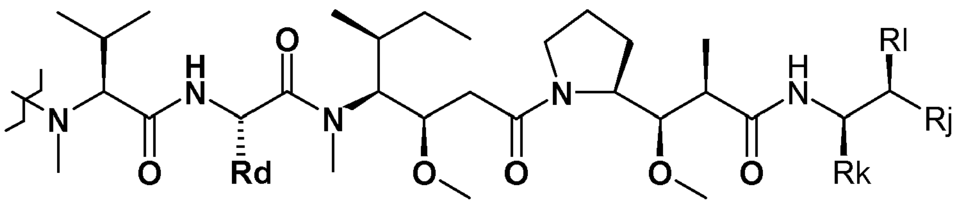

(F),

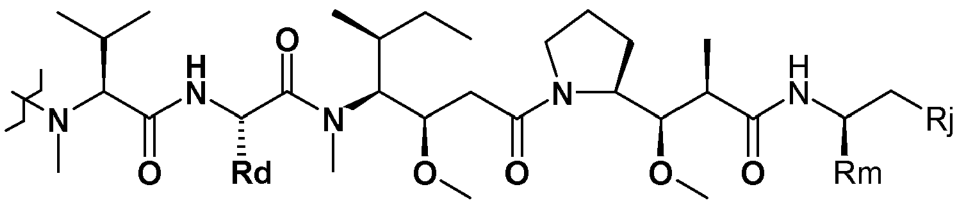

(G).

Examples of preferred specific auristatins include:

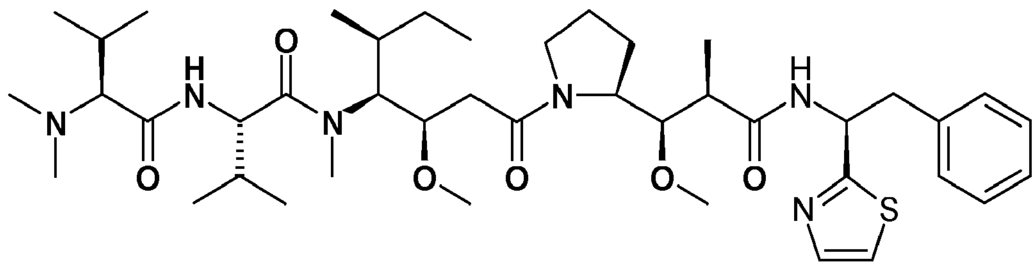

(dolastatin 10),

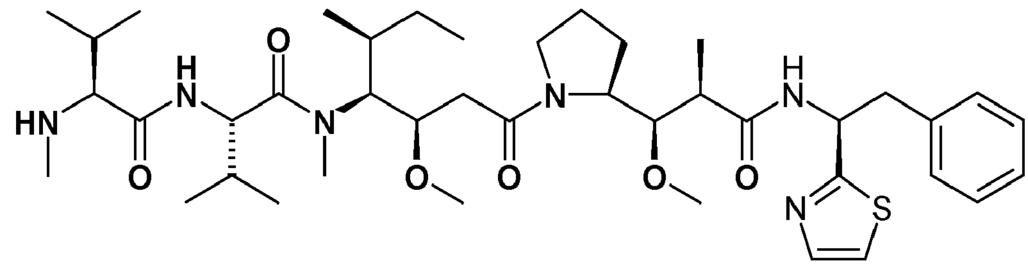

(monomethyl dolastatin 10),

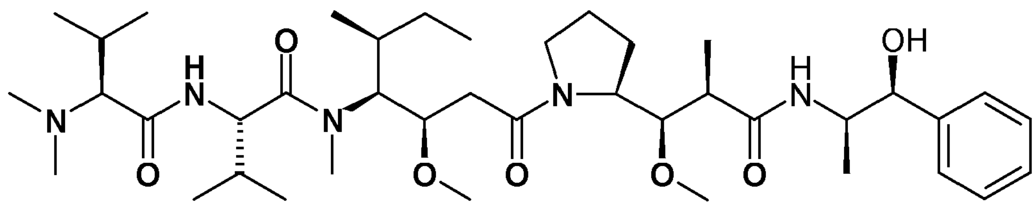

(auristatin E),

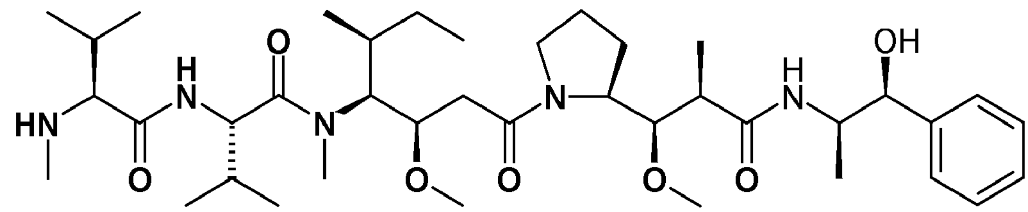

(monomethyl auristatin E),

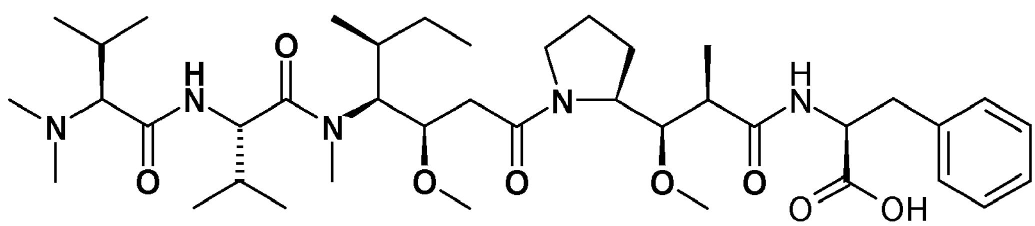

(auristatin F),

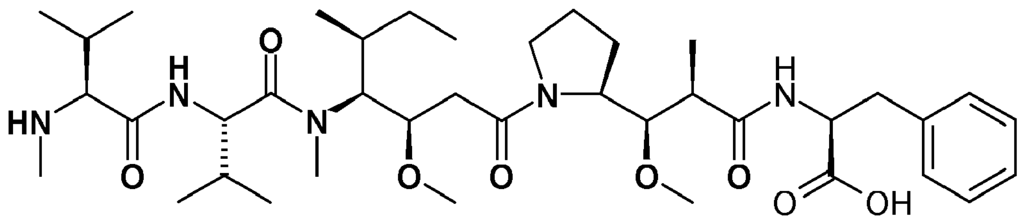

(monomethyl auristatin F),

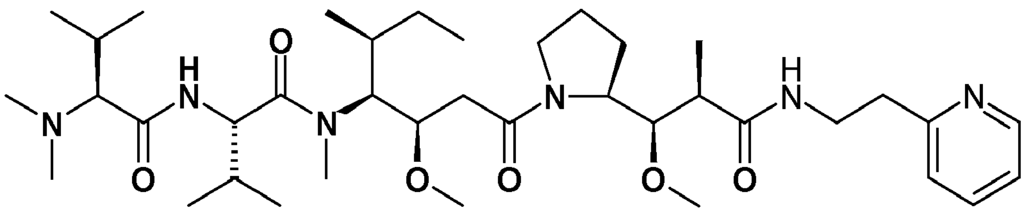

(auristatin PYE),

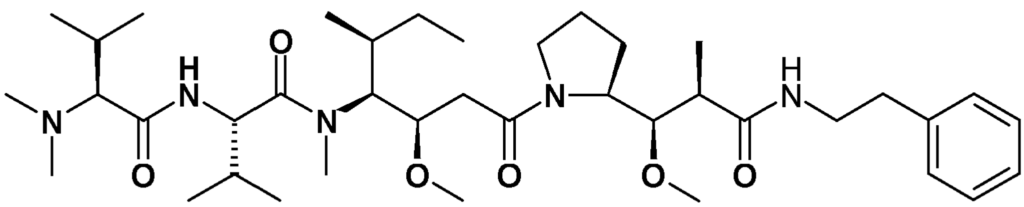

(auristatin PE),

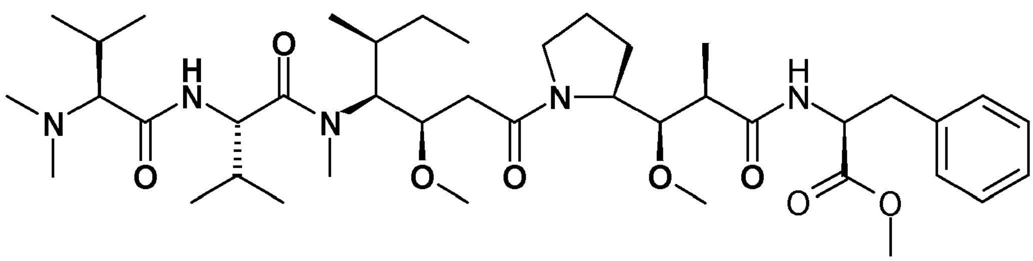

(auristatin PHE),

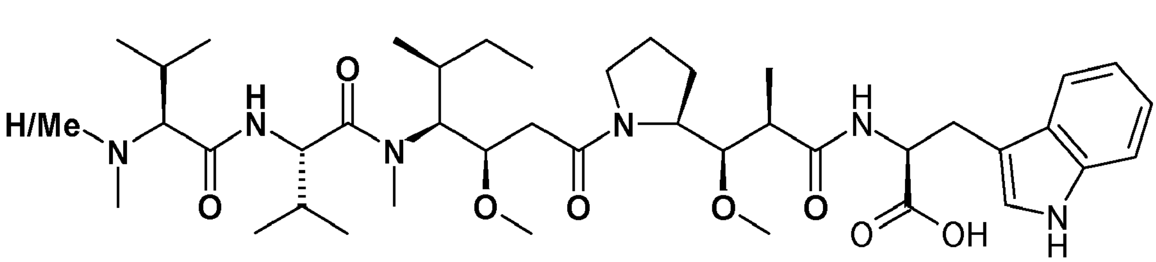

,

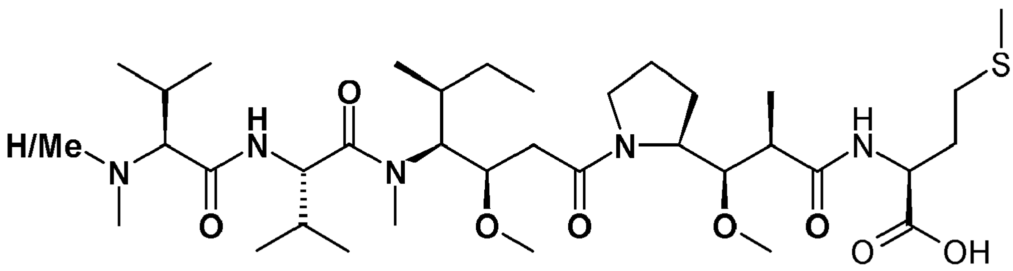

,

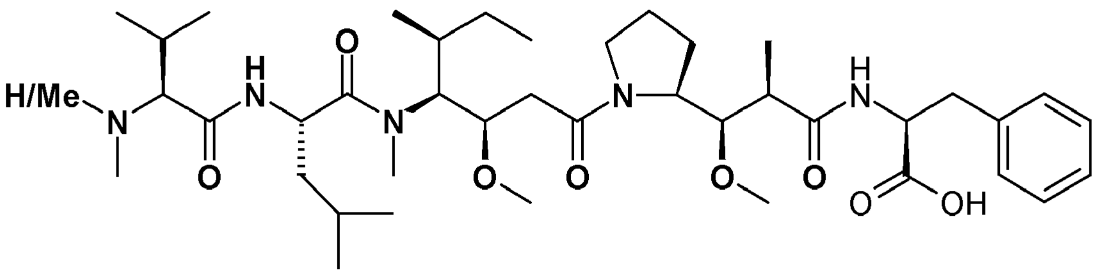

,

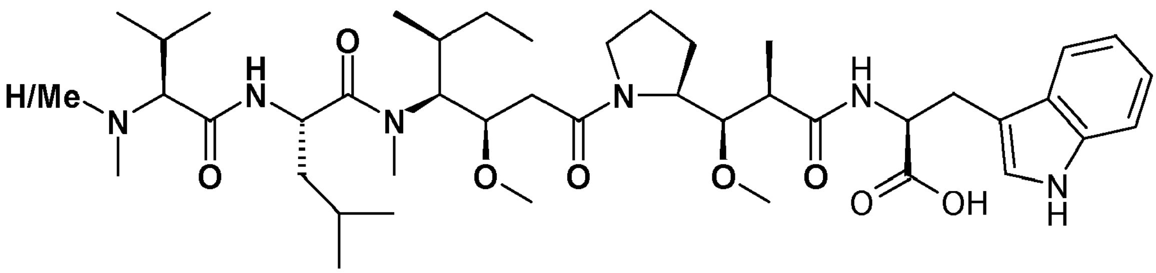

,

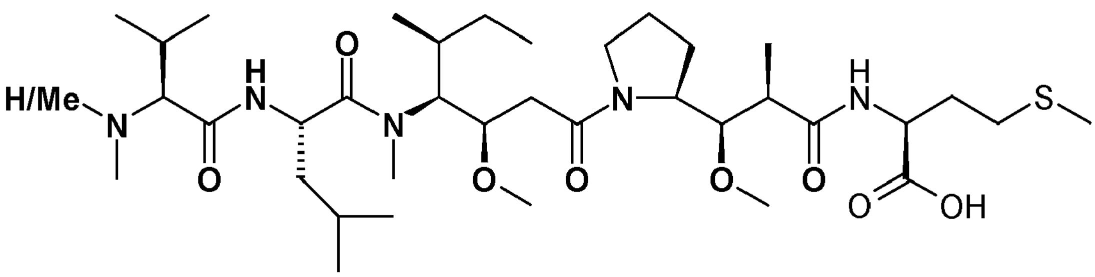

,

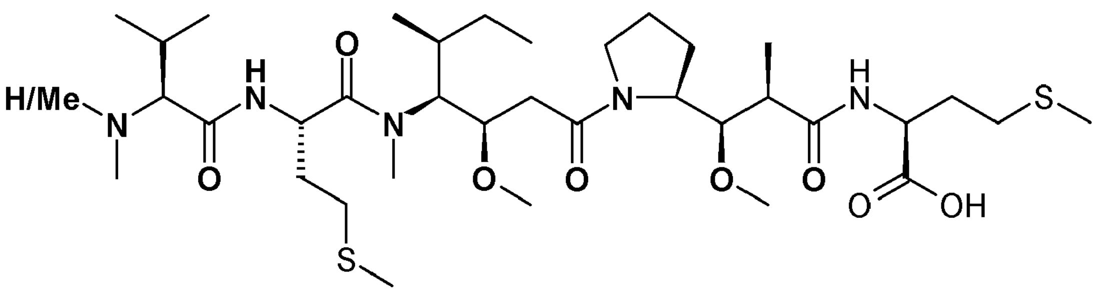

,

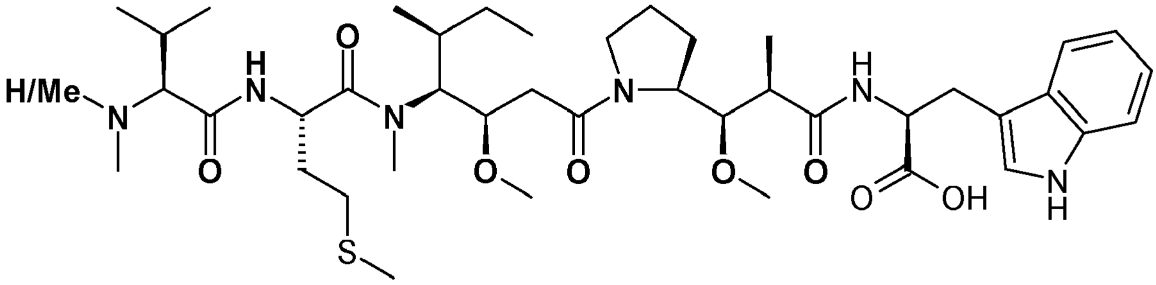

,

and

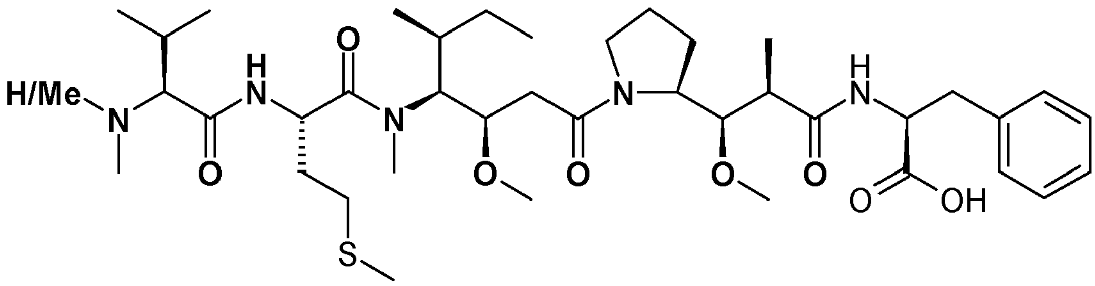

Depending on the structure of the auristatin, it may exist in the form of the free base or free acid, or in the form of a pharmaceutically acceptable salt, and/or as a solvate. The conjugates of formula I may also exist in these forms.

$Lk^1$ may be bonded to the auristatin moiety at any suitable point. Where the auristatin moiety corresponds to an auristatin having an N-terminal hydrogen, $Lk^1$ is preferably bonded to the N-terminal nitrogen, e.g.

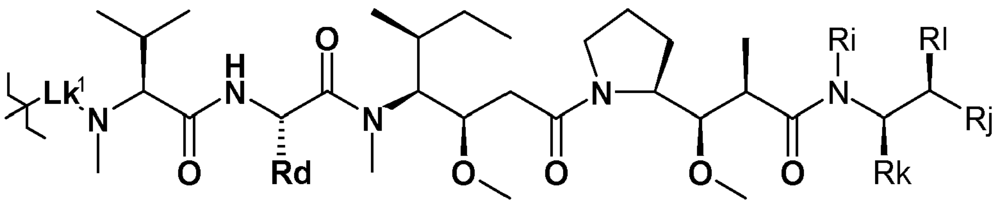

,

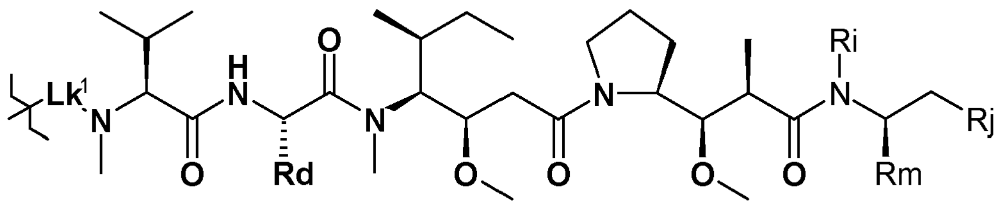

In the case of an auristatin moiety corresponding to an auristatin in which Rm represents $-CO_2H$, $Lk^1$ may for example be bonded to that C-terminal carbon, e.g.

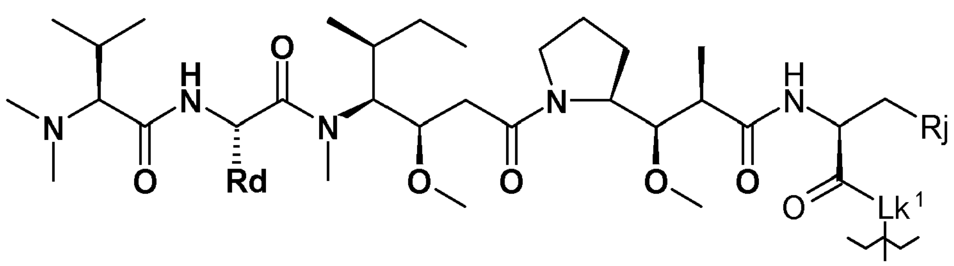

,

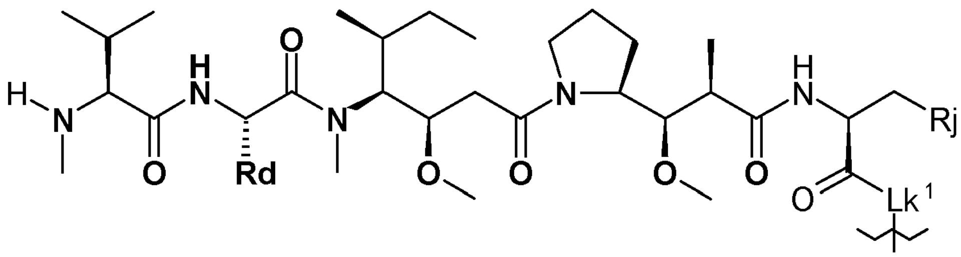

$Lk^1$ is a linker, a bond or a group which connects an auristatin moiety D to a P group. It may carry from 1 to 10 D groups. $Lk^1$ preferably contains a degradable group, i.e. $Lk^1$ is preferably a linker which breaks under physiological conditions, separating D from Ab. Alternatively, $Lk^1$ may be a linker that is not cleavable under physiological conditions. Where $Lk^1$ is a linker which breaks under physiological conditions, it is preferably cleavable under intracellular conditions. Where the target is intracellular, preferably $Lk^1$ is substantially insensitive to extracellular conditions (i.e. so that delivery to the intracellular target of a sufficient dose of the therapeutic agent is not prohibited).

Where $Lk^1$ is a degradable linker, it may contain a group that is sensitive to hydrolytic conditions. $Lk^1$ may contain a group which degrades at certain pH values (e.g. acidic conditions). Hydrolytic/acidic conditions may for example be found in endosomes or lysosomes. Examples of groups susceptible to hydrolysis under acidic conditions include hydrazones, semicarbazones, thiosemicarboazones, cis-acotinic amides, orthoesters and acetals/ketals. Examples of groups susceptible to hydrolytic conditions include:

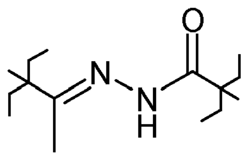

and

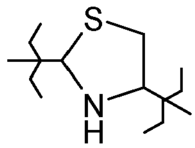

$Lk^1$ may also be susceptible to degradation under reducing conditions. For example, $Lk^1$ may contain a disulfide group that is cleavable on exposure to biological reducing agents, such as thiols. Examples of disulfide groups include:

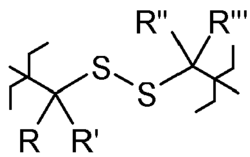

,

in which R, R', R'' and R''' are each independently hydrogen or $C_{1-4}$alkyl, and

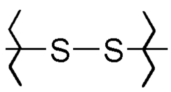

$Lk^1$ may also contain a group which is susceptible to enzymatic degradation, for example it may be susceptible to cleavage by a protease (e.g. a lysosomal or endosomal protease) or peptidase. For example, $Lk^1$ may contain a peptidyl group comprising at least one, for example at least two, or at least three, amino acid residues (e.g. Phe-Leu, Gly-Phe-Leu-Gly, Val-Cit, Phe-Lys). For example, $Lk^1$ may be an amino acid chain having from 1 to 5, for example 2 to 4, amino acids. Another example of a group susceptible to enzymatic degradation is:

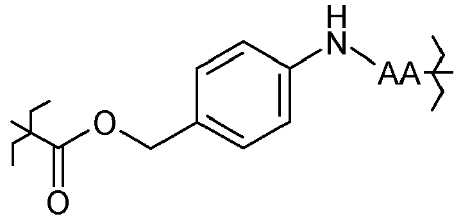

,

wherein AA represents a protease-specific amino acid sequence, such as Val-Cit.

In a preferred embodiment, $Lk^1$ is or includes:

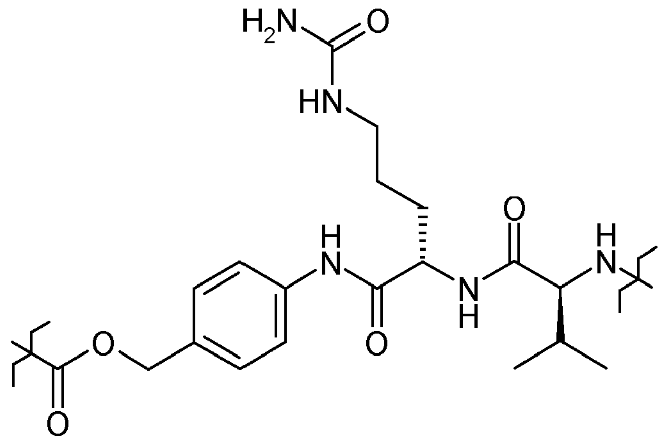

(V)

For example, $Lk^1$ may be

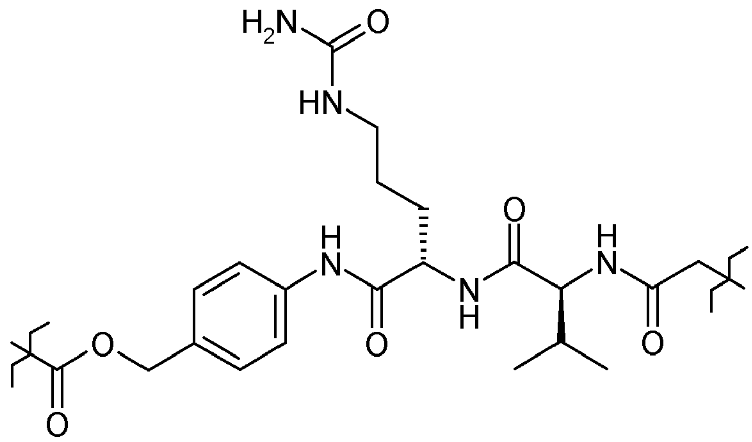

(Va)

in which case it is preferably bonded to the D and P groups as shown below

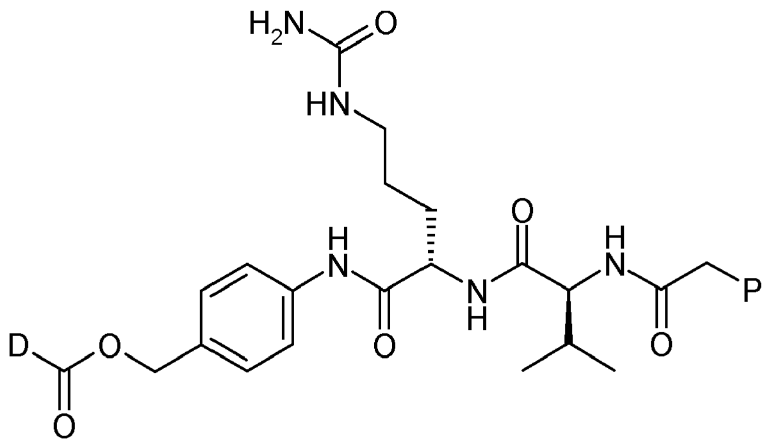

(Vb).

In that embodiment, the auristatin moiety D is preferably bonded via the N-terminal nitrogen.

In one embodiment, $Lk^1$ carries a single auristatin moiety D (i.e. q=1). The specific linker V shown above is of this type. In another embodiment, q is greater than 1, for example 2, 3 or 4, and $Lk^1$ is used as a means of incorporating more than one auristatin moiety into a conjugate of the invention. In one embodiment, this may be achieved by the use of a branching linker $Lk^1$, which may for example incorporate an aspartate or glutamate or similar residue. This introduces a branching element of formula:

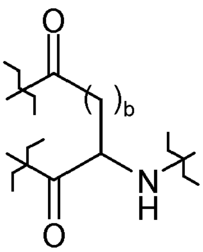

(VI)

where b is 1, 2 or 3, b=1 being aspartate and b=2 being glutamate, and b=3 representing one preferred embodiment. Each of the acyl moieties in the formula VI may be coupled to a group D via a suitable linker $Lk^{1a}$, where $Lk^{1a}$ is any suitable linker, for example a degradable linker incorporating one of the linkages mentioned above for $Lk^1$. In one particular embodiment, $Lk^{1a}$ represents the group V shown above. The amino group of the aspartate or glutamate or similar residue may be bonded to P by any suitable means, for example the linkage may be via an amide bond, e.g. the branching group above may be connected to P via a $-CO.CH_2-$ group, thus:

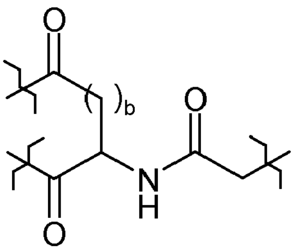

(VIa)

If desired, the aspartate or glutamate or similar residue may be coupled to further aspartate and/or glutamate and/or similar residues, for example:

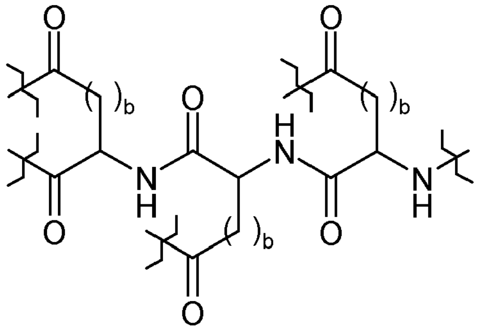

(VIIa) or

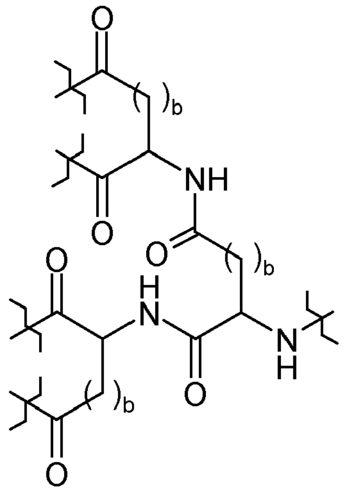

(VIIb)

and so on, up to a maximum of 9 such residues, giving the potential to incorporate up to 10 D groups. As above, each D may be attached to an aspartate/glutamate or similar residue via any suitable linker $Lk^{1a}$.

Where P represents a $-P^1$-NH- group, $P^1$ contains at least one ethylene or ethylene glycol unit ($-CH_2-CH_2-$ or $-O-CH_2-CH_2-$). If many such units are present, $P^1$ represents polyethylene, PE, or polyethylene glycol, PEG. These polymers may contain a single linear chain, or may have branched morphology composed of many chains either small or large, in which case they will contain branching groups, typically containing >CH-, as for example in $-(CH_2CH_2)_2-CH-$ or $-(O-CH_2-)_2CH-$. They may optionally be derivatised or functionalised in any desired way. They may for example carry an additional therapeutic agent, or a labelling agent. Multimeric conjugates that contain more than one molecule of therapeutic agent, for example more than one molecule of an auristatin, or a molecule of a therapeutic agent in addition to a molecule of an auristatin, can result in synergistic and additive benefits.

Where $P^1$ represents PE or PEG, the optimum molecular weight of the polymer will of course depend upon the intended application. Generally, where $P^1$ represents PE, it is preferred that the number average molecular weight is up to 2kDa, preferably up to 1kDa. Where $P^1$ represents PEG, higher molecular weights may be used, for example the number average molecular weight may be up to 75kDa, for example up to 60kDa, with the minimum number average molecular weight being for example at least 0.5kDa, for example at least 1kDa, for example 2kDa. In one preferred embodiment, PEG of number average molecular weight of from 0.5 to 2kDa may be used. However, in some preferred embodiments, P may be a bond, or P may represent $-P^1$-NH- wherein $P^1$ contains a small number of discrete ethylene or ethylene glycol units, for example from 2 to 10, for example 2 or 3, ethylene or, preferably, ethylene glycol units.

If it is desired for the conjugate of formula I to contain more than one $-(CH_2-CH_2)_a-$ or $-(O-CH_2-CH_2)_a-$ chain (where a is the number of ethylene or ethylene glycol units in any linear chain), for example so that each such chain may carry a Dq-$Lk^1$ group, this may be achieved either by bonding more than one (i.e. from 2 to 10) such chains to $Lk^2$, or by using a branched PE or PEG, in which case only one group P will be attached to $Lk^2$ , but this will contain more than one branch, for example from 1 to 9 branches (providing from 2 to 10 attachment points for D-$Lk^1$ groups).

It will be understood that where P is $-P^1$-NH-, the or each P group is coupled to adjacent groups $Lk^1$ and/or $Lk^2$ via an amide bond. For example PEG (which normally terminates with an -OH group) may be converted into the corresponding PEG amine, which terminates with an $-NH_2$ group, for amide bond formation with a $-CO_2$ group in, say, $Lk^2$; or the OH group may be reacted to form a linkage $-NH.CO.CH_2.O-$ with $Lk^1$ as described above.

In a preferred embodiment of the invention, $P^1$ represents PEG, a water-soluble, synthetic polymer, and throughout this specification, except where the context requires otherwise, any general reference to $P^1$ should be understood to include a specific reference to PEG.

$Lk^2$ represents a y-valent linker where y is from 2 to 11. It is thus capable of bonding from 1 to 10 groups P or $Lk^1$. In its simplest form, $Lk^2$ is a bond, in which case $Lk^1$ is bonded directly to a $-P^1$-NH- group or, if P is a bond, to a D-$Lk^1$ group. However, $Lk^2$ may be used as a means of incorporating more than one D group (auristatin moiety) into the conjugates of the invention. This is achieved by coupling an aspartate or glutamate residue to the -CO-group of $Lk^3$ via an amide linkage (e.g. reacting the aspartate or glutamate amine group with a suitable carboxylic acid derivative corresponding to $Lk^3$). This introduces a branching element of formula VI shown above. In that case, each of the acyl moieties may be coupled to a $-P^1$-NH- group via an amide linkage, or when P is a bond, to a D-$Lk^1$ group. Alternatively the aspartate or glutamate residue may be coupled to further aspartate and/or glutamate residues, as shown in formulae VIIa and VIIb shown above, and so on, up to a maximum of 9 such residues, giving the potential to incorporate up to 10 D groups via bonding of multiple D-$Lk^1$-P groups at different attachment points in $Lk^2$. It will be understood that the

valency of $Lk^2$ is associated with the number of D-$Lk^1$-P groups present. For example, when P is -$P^1$-NH-, for each D-$Lk^1$-P group, the valency of $Lk^2$ is associated with the number of -$P^1$-NH- groups present, i.e. p will equal y-1. When P is a bond, for each D-$Lk^1$-P group, the valency of $Lk^2$ is associated with the number of groups D-$Lk^1$ present, i.e. m will equal y-1.

$Lk^3$ is a specific linker capable of binding to a binding protein via two sulfur groups derived from a disulfide bond in the binding protein. In $Lk^3$, the phenyl group Ph may be unsubstituted or substituted. Substituents which may optionally be present on the phenyl group Ph include for example one or more of the same or different substituents selected from alkyl (preferably $C_{1-4}$alkyl, especially methyl, optionally substituted by OH or $CO_2H$), -CN, - $NO_2$, -$CO_2R^4$, -COH, -$CH_2OH$, -$COR^4$, -$OR^4$, -$OCOR^4$, -$OCO_{2R}{}^4$, -$SR^4$, -$SOR^4$, -$SO_2R^4$,-$NHCOR^4$, -$NR^4COR^4$, $NHCO_2R^4$, -$NR^4.CO_2R^4$, -NO, -NHOH, -$NR^4$.OH, -C=N-$NHCOR^4$, -C=N-$NR^4.COR^4$, -$N^+R^4_3$, -$N^+H_3$, -$N^+HR^4_2$, -$N^+H_2R^4$, halogen, for example fluorine or chlorine, -C≡$CR^4$, -C=$CR^4_2$ and -C=$CHR^4$, in which each $R^4$ independently represents a hydrogen atom or an alkyl (preferably $C_1$-$_6$alkyl), aryl (preferably phenyl), or alkyl-aryl (preferably $C_{1-6}$alkyl-phenyl) group. The presence of electron withdrawing substituents is especially preferred. Preferred substituents include for example -CN, -$NO_2$, -$OR^4$, -$OCOR^4$, -$SR^4$, -$NHCOR^4$, -NR.$COR^4$, -NHOH and -$NR^4.COR^4$. Preferably, however, the phenyl group Ph is unsubstituted.

When Y represents the group III, a single carbon bridge is formed between the linker $Lk^3$ and two sulfur atoms derived from a disulfide bond in the binding protein Ab, and when Y represents the group IV, the nature of the groups A and B determine the length of the bridge which is formed between the linker $Lk^3$ and two sulfur atoms derived from a disulfide bond in the binding protein Ab. Preferably, a 3-carbon bridge is formed, i.e. preferably Y has the formula:

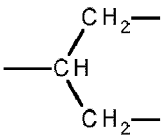

(IVa)

As mentioned above, conjugates which contain more than one auristatin moiety may have advantages. The presence of more than one auristatin moiety may be achieved in a number of different ways, for example as described above by the use of a branched PEG, by the use of a multivalent group $Lk^2$, or by the use of a multivalent group $Lk^1$. It may however also be achieved by attaching more than one linker $Lk^3$ to the binding protein Ab. In general, normal full-length antibodies have 4 interchain disulfide bonds (heavy-heavy chain or heavy-light chain for whole IgG1 antibodies), and any or all of these can be bridged by the linker $Lk^3$ according to the invention. It is also envisaged that one or more intrachain disulfide bonds in the antibody may be bridged by a linker $Lk^3$. Where more than one conjugating group is present, n is greater than 1. n may for example be 2, 3, or 4.

Alternatively or in addition, one or more additional auristatin moieties can be present linked via a linker $Lk^1$ to P or, where P is a bond, directly to $Lk^2$. In this case, m is greater than 1, for example 2, 3 or 4, up to a maximum of 10. If more than one linker $Lk^1$ is present, these may be the same as each other, or different.

Alternatively or in addition, one or more additional auristatin moieties can be present linked to a multivalent linker $Lk^1$. In this case, q is greater than 1, for example 2, 3 or 4, up to a maximum of 10.

It is envisaged that conjugates of the present invention may carry up to 10 D groups (auristatin moieties). Where it is desired for the conjugate of formula I to contain more than one D group (i.e. more than one auristatin moiety), this may be achieved in any one of a number of ways. For example, multiple $(((Dq\text{-}Lk^1)_m\text{-}P)_p\text{-}Lk^2\text{-}Lk^3)$- groups may be bonded to a single antibody (i.e. n is from 2 to s). This mode of attachment forms one preferred way of providing conjugates containing more than one group D. A second preferred way of providing conjugates containing more than one group D is by use of a multivalent linker $Lk^1$, for example a linker $Lk^1$ which contains one or more aspartate and/or glutamate or similar residues as described above (as for example in formulae VI, VIIa and VIIb), allowing multiple D groups to be present (i.e. q is from 2 to 10). It is believed that conjugates in which $Lk^1$ is a multivalent linker, especially one including formula VI above, p=1, q=2, m=1 and $Lk^2$ is a bond, are particularly effective, and such conjugates form a preferred embodiment of the invention.

Alternatively or in addition, where $Lk^2$ is a group consisting of from 1 to 9 aspartate and/or glutamate residues, multiple $((Dq\text{-}Lk^1)_m\text{-}P)$- groups may be bonded at different positions on the $Lk^2$ group (i.e. p is from 2 to 10), by amide bonding of each group through an amine moiety with a carboxyl group of an aspartate or glutamate residue in the $Lk^2$ group. Where $P^1$ contains at least one ethylene or ethylene glycol unit and also contains at least one branching unit, multiple $(Dq\text{-}Lk^1)$- groups may additionally or alternatively be bonded at different positions on the $P^1$ group (i.e. m is from 2 to 10).

Conjugates having combinations of the above are also encompassed by the invention. By way of example, a conjugate may contain an Ab group bonded to two $((D\text{-}Lk^1)_m\text{-}P)p\text{-}Lk^2\text{-}Lk^3$-groups in which, for each of those ((D-

$Lk^1)_m$-P)p-$Lk^2$-$Lk^3$- groups, $Lk^2$ is an aspartate or glutamate residue bonded to two (D-$Lk^1)_m$-P groups, and in which, for each of those (D-$Lk^1)_m$-P groups, P is -$P^1$-NH- in which $P^1$ contains at least one ethylene or ethylene glycol unit and also contains at least one branching unit, so that in total 8 D-$Lk^1$ groups are present in the conjugate. By way of further example, a conjugate may contain an Ab group bonded to two ((D-$Lk^1)_m$-P$)_p$-$Lk^2$-$Lk^3$- groups in which, for each of those ((D-$Lk^1)_m$-P$)_p$-$Lk^2$-$Lk^3$-groups, $Lk^2$ is a bond, P is -$P^1$-NH- in which $P^1$ contains at least one ethylene or ethylene glycol unit and also contains at least one branching unit, and each $Lk^1$ contains an aspartate or glutamate or similar residue bonded to two D groups, so that in total 8 D-$Lk^1$ groups are present in the conjugate.

Different $Lk^3$, $Lk^2$, P, $Lk^1$ and D groups may also be present in the same conjugate, for example where a conjugate contains an Ab group bonded to two ((D-$Lk^1)_m$-P$)_p$-$Lk^2$-$Lk^3$ groups, in one of those ((D-$Lk^1)_m$-P$)_p$-$Lk^2$-$Lk^3$-groups $Lk^2$ may be a bond and in the other of those ((D-$Lk^1)_m$-P$)_p$-$Lk^2$-$Lk^3$- groups $Lk^2$ may be an aspartate or glutamate residue. Similarly, where a conjugate contains multiple (D-$Lk^1)_m$-P groups bonded to an $Lk^2$ group, for one of those groups P may be a bond, and for another of those groups P may be -$P^1$-NH-.

In its simplest embodiment, the invention relates to conjugates which contain a single auristatin moiety (n=m=q=p=1). The conjugates may contain up to 10 auristatin moieties, for example up to 8 auristatin moieties. They may for example contain up to 4, for example 1, 2, 3 or 4, auristatin moieties. Where two D groups are present, these may for example be in conjugates of the formulae:

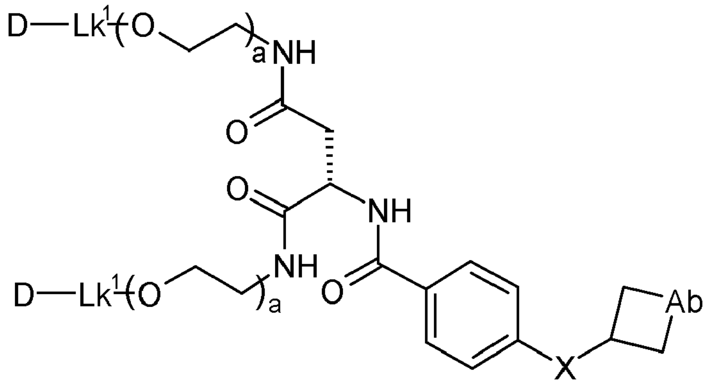

(Ia),

or

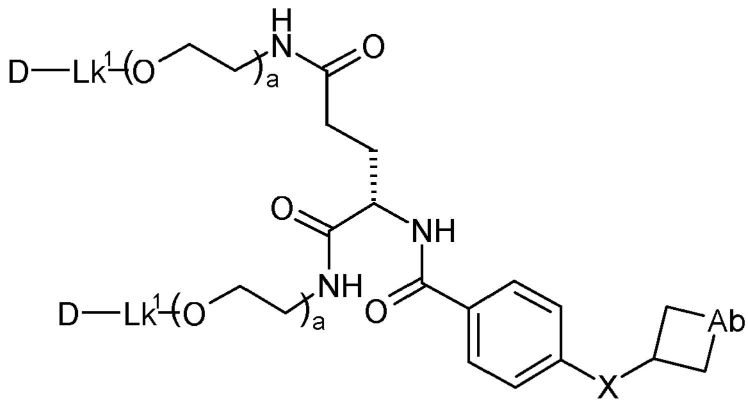

(Ib),

in which $Lk^1$ preferably comprises a group of formula (Va) as described above.

Alternatively, where two D groups are present, these may for example be in conjugates of the formulae:

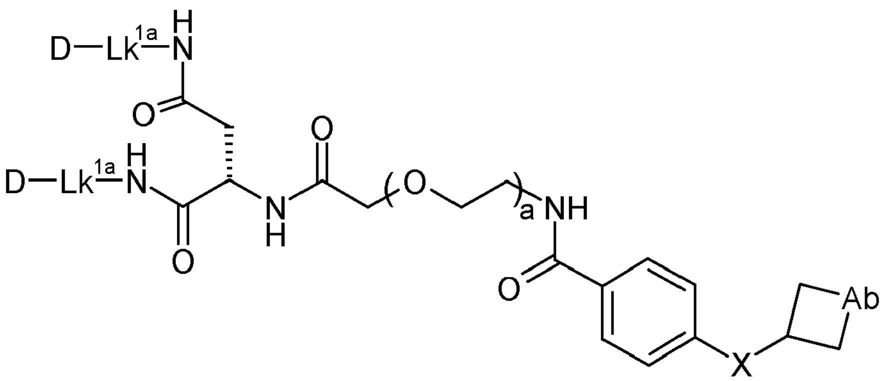

(Ic), or

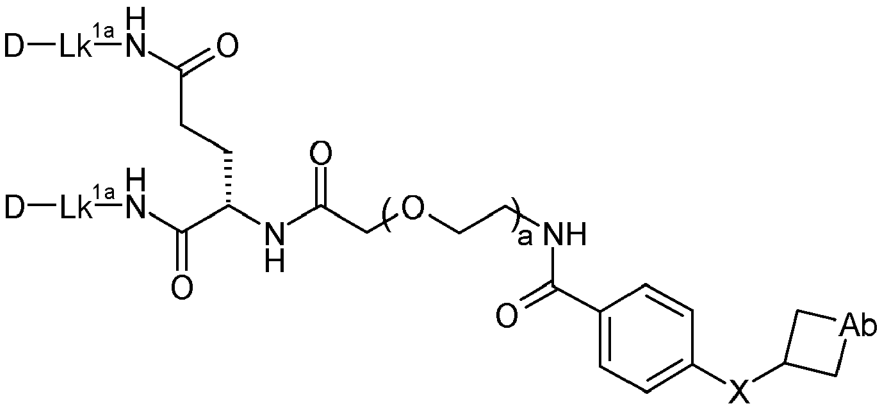

(Id),

in which $Lk^{1a}$ preferably comprises a group of formula (V) as described above.

The above formulae show the bonding of X across one of the disulfide bonds present in Ab. Antibodies may contain up to 4 inter-chain disulfide bonds, and if each of these bonds is bridged by a reagent carrying a single auristatin molecule, the resulting conjugate will have a drug:antibody ratio (DAR) of 4. If a reagent carrying two auristatin molecules is used to bridge all 4 disulfide bonds, for example a reagent carrying two PE or PEG chains or having a branched PE or PEG chain or having a branched linker $Lk^1$, then the DAR will be 8. Conjugates having such high DARs may have significant clinical advantages. Conjugates of the above formulae Ia-Ie above but in which Ab carries 2, 3 or, especially, 4, copies of the ~X- group, form one preferred embodiment of the invention.

For convenience, the term "binding protein" is used throughout this Specification to include both binding proteins and peptides, and except where the context specifically requires otherwise, should be understood to include peptides as well as proteins. Binding proteins that can be used in the conjugates of the invention include any protein, polypeptide or peptide that can serve as a binding agent for a binding partner on a target. The target may be for example a microorganism, a virus, or a cell, for example a cancer or immune cell. The binding protein thus acts to target the auristatin to the particular target. Examples of such binding proteins include full length antibodies, antibody fragments, immunoglobulin (Ig) and non-Ig protein scaffolds obtained by rational or combinatorial protein engineering techniques, and lectins. The most common binding proteins used in protein-drug conjugates are antibodies, and any reference to a binding protein or to the group Ab should, except where the context specifically requires otherwise, be understood to include a specific reference to an antibody.

Throughout this specification, the term "antibody" should be understood to mean an immunoglobulin molecule that recognises and specifically binds to a target antigen, such as a protein, polypeptide, peptide, carbohydrate, polynucleotide, lipid, or combination thereof through at least one antigen recognition site within the variable region of the immunoglobulin molecule. The term "antibody" encompasses polyclonal antibodies, monoclonal antibodies, multispecific antibodies such as bispecific antibodies, chimeric antibodies, humanised antibodies, human antibodies, fusion proteins comprising an antigen determination portion of an antibody, and any other modified immunoglobulin molecule comprising an antigen recognition site so long as the antibodies exhibit the desired biological activity. An antibody can be of any the five major classes of immunoglobulins: IgA, IgD, IgE, IgG, and IgM, or subclasses (isotypes) thereof (e.g. IgG1, IgG2, IgG3, IgG4, IgAl and IgA2), based on the identity of their heavy-chain constant domains referred to as alpha, delta, epsilon, gamma, and mu, respectively. The different classes of immunoglobulins have different and well known subunit structures and three-dimensional configurations. The use of IgG1 or IgG4 is particularly preferred.

Further, except where the context requires otherwise, the term "antibody" should be understood to encompass full length antibodies and antibody fragments comprising an antigen-binding region of the full length antibody. Antibody fragments may for example be Fab, Fab', $F(ab')_2$, scFv, Fv, diabodies, minibodies or multispecific antibodies formed from antibody fragments, for example minibodies composed of different permutations of scFv fragments or diabodies, and optionally Fc fragments or CH domains, such as scFv-Fc, scFv-Fc-scFv, Fab-scFv, $(Fab' ScFv)_2$, scDiabodies, scDiabody-Fc, scDiabody-$C_H3$, scFv- $C_H3$, scFv-$C_H2$-$C_H3$ fusion proteins and so forth. An antibody fragment can be produced by enzymatic cleavage, synthetic or recombinant techniques.

A binding protein can serve as a binding agent for a receptor, antigen or other moiety on the surface of a target, for example a cell or virus associated with a proliferative, autoimmune or infectious disease. For example, the binding protein may be an antibody that specifically binds to a cell surface antigen on a cancer cell. Methods of identification and validation of cell surface antigens for antibody targeting of cancer cells are known, for example in Carter P, et al., Endocr. Relat. Cancer. 2004 Dec; 11(4):659-87, and a number of antibody-drug conjugates for treating cancer are currently in clinical development. Examples of antibodies available for the treatment of cancer, and tumour markers of specific cancers, are also well known in the art and can be used. Alternatively, the target may be an immune cell, for example a cell that is responsible for producing autoimmune antibodies, or an activated lymphocyte that is associated with an autoimmune disease. In other embodiments, the target may be a micro-organism or virus associated with a microbial or viral infection or disease.

Conjugates of the present invention may be prepared by reducing one or more disulfide bonds in a binding protein and subsequently reacting with a conjugating reagent of the general formula:

$$((D_q\text{-}Lk^1)_m\text{-}P)_p\text{-}Lk^2\text{-}Lk^{3a} \qquad \text{(VIII)}$$

in which D, $Lk^1$, P, $Lk^2$ and m, p and q have the meanings given for the general formula I, and $Lk^{3a}$ represents a group of formula:

$$\text{-CO-Ph-}X^a\text{-}Y^a \qquad \text{(IX)}$$

in which Ph has the meaning given above, $X^a$ represents a CO group, and $Y^a$ represents a group:

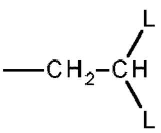
(X),
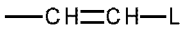
(XI),
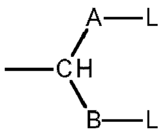
(XII)

or

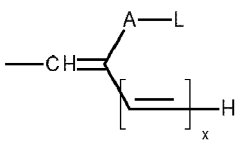
(XIII)

in which A and B have the meanings given above, each L independently represents a leaving group, and x represents an integer from 1 to 4.

Groups of formulae X, XI, XII and XIII above are chemical equivalents of each other, with groups of formula X and XI leading to a single carbon bridge across a disulfide bond of the antibody, and groups of formula XII and XIII leading to longer carbon bridges, shown below for the case where n=1:

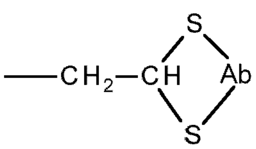
(XIV) or
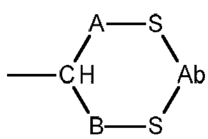
(XV)

When reacting a conjugating reagent containing a group X or XII with an antibody, the immediate step in the reaction

pathway is loss of one leaving group L leading to a conjugating reagent containing a group XI or XIII, respectively. Thus, conjugating reagents of formula XI or XIII are either prepared *in situ,* or are used *ab initio.* A key feature of using conjugation reagents containing any of groups X, XI, XII or XIII, is that an $\alpha$-methylene leaving group and a double bond are cross-conjugated with an electron withdrawing function that serves as a Michael activating moiety. If the leaving group is prone to elimination in the cross-functional reagent rather than to direct displacement and the electron-withdrawing group is a suitable activating moiety for the Michael reaction then sequential intramolecular bis-alkylation can occur by consecutive Michael and retro Michael reactions. The leaving moiety serves to mask a latent conjugated double bond that is not exposed until after the first alkylation has occurred and bis-alkylation results from sequential and interactive Michael and retro-Michael reactions. The electron withdrawing group and the leaving group are optimally selected so bis-alkylation can occur by sequential Michael and retro-Michael reactions. It is also possible to prepare cross-functional alkylating agents with additional multiple bonds conjugated to the double bond or between the leaving group and the electron withdrawing group.

A leaving group L may for example represent $-SR^4$, $-SO_2R^4$, $-OSO_2R^4$, $-N^+R^4_3$, $-N^+HR^4_2$, $-N^+H_2R^4$, halogen, or -OØ in which $R^4$ has the meaning given above, and Ø represents a substituted aryl, especially phenyl, group, containing at least one electron withdrawing substituent, for example -CN, $-NO_2$, $-CO_2R^4$ -COH, $-CH_2OH$, $-COR^4$, $-OR^4$, $-OCOR^4$, $-OCO_2R^4$, $-SR^4$,$-SOR^4$, $-SO_2R^4$, $-NHCOR^4$, $-NR^4COR^4$, $-NHCO_2R^4$, $-NR^4CO_2R^4$, -NO, -NHOH, $-NR^4OH$, $-C=N-NHCOR^4$, $-C=N-NR^4COR^4$, $-N^+R^4_3$, $-N^+HR^4_2$, $-N^+H_2R^4$, halogen, especially chlorine or, especially, fluorine, $-C\equiv CR^4$, $-C=CR^4_2$ and $-C=CHR^4$, in which each $R^4$ independently has one of the meanings given above. An especially preferred leaving group L is $-SR^4$ or $-SO_2R^4$, especially $-SO_2R^4$, where $R^4$ represents a phenyl or, especially, a tosyl group. Thus, a particularly preferred group $Y^a$ is:

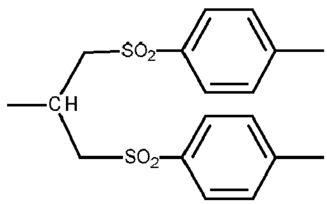

(XIIa)

Examples of preferred conjugating reagents include:

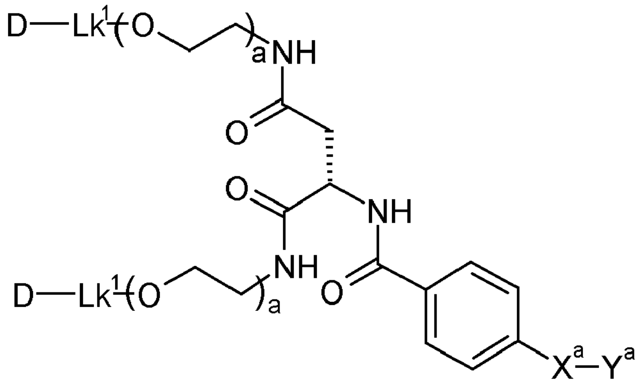

(VIIIa),

and

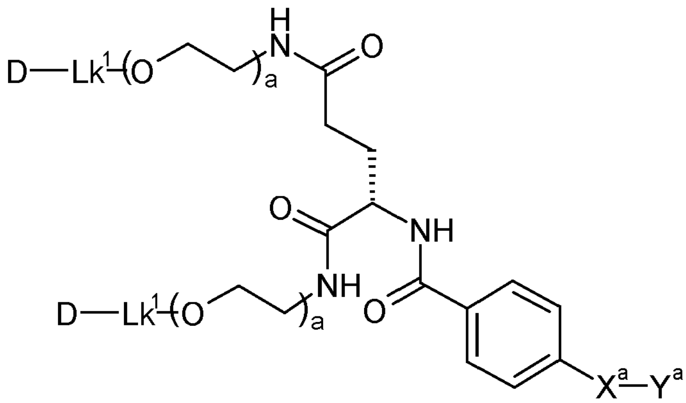

(VIIIb),

in which $Lk^1$ preferably comprises a group of formula (Va) as described above, and in which $Y^a$ is preferably a group of formula (XII), especially in which A and B are each $-CH_2-$.

Further preferred examples of conjugating agents include:

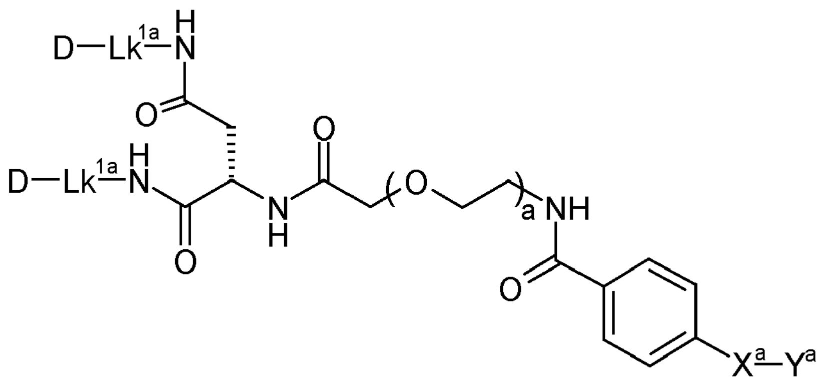

(VIIIc),

and

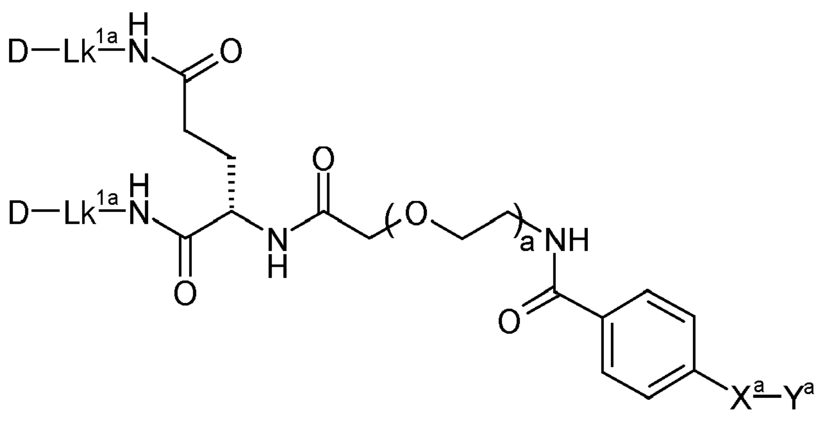

(VIIId),

in which $Lk^1$ preferably comprises a group of formula (V) as described above, and in which $Y^a$ is preferably a group of formula (XII), especially in which A and B are each $-CH_2-$.

Still further preferred examples of conjugating reagents include:

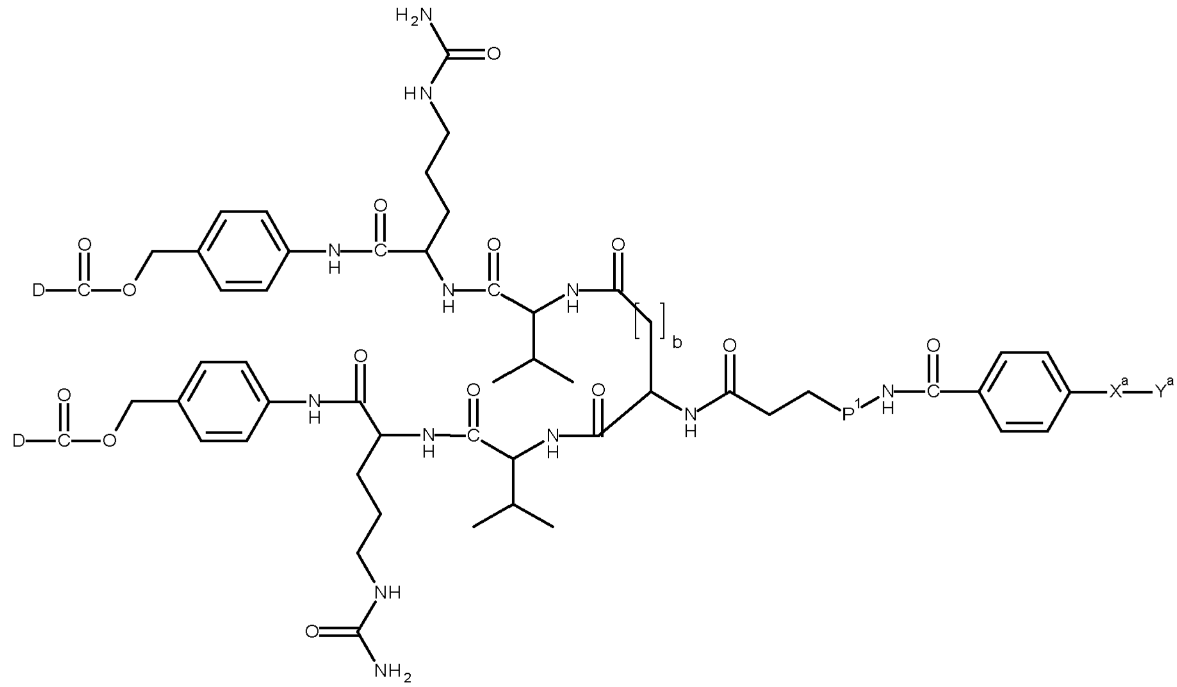

in which b is 1, 2 or 3. $Y^a$ is preferably a group of formula (XII), especially in which A and B are each $-CH_2-$. $P^1$ is preferably PEG, especially one of those mentioned above, and preferably one having from 15 to 35 repeating $-O-CH_2-CH_2-$ units.

The immediate product of the conjugation process using one of the reagents described above is an auristatin-antibody conjugate in which X represents a keto group CO. However, the process of the invention is reversible under suitable conditions. This may be desirable for some applications, for example where rapid separation of the auristatin from the antibody is required, but for other applications, rapid separation may be undesirable. It may therefore be desirable to stabilise the conjugates by reduction of the CO group X to give a CH.OH group X. Accordingly, the process described above may comprise an additional optional step of reducing the initially-formed CO group X in $Lk^3$ to give a conjugate having a CH.OH group X in $Lk^3$. The use of a borohydride, for example sodium borohydride, sodium cyanoborohydride, potassium borohydride or sodium triacetoxyborohydride, as reducing agent is particularly preferred. Other reducing agents which may be used include for example tin(II) chloride, alkoxides such as aluminium alkoxide, and lithium aluminium hydride.

Suitable reaction conditions for the process described above are given in WO 2005/007197 and WO 2010/100430, the contents of which are incorporated herein by reference. The process may for example be carried out in a solvent or solvent mixture in which all reactants are soluble. The antibody may be allowed to react directly with the conjugation reagent in an aqueous reaction medium. This reaction medium may also be buffered, depending on the pH requirements of the nucleophile. The optimum pH for the reaction will generally be at least 4.5, typically between about 5.0 and about 8.5, preferably about 6.0 to 8.2. The optimal reaction conditions will of course depend upon the specific reactants employed.

Reaction temperatures between 3-37°C are generally suitable. Reactions conducted in organic media (for example THF, ethyl acetate, acetone) are typically conducted at temperatures up to ambient.

The binding protein can be effectively conjugated with the desired reagent using a stoichiometric equivalent or an excess of reagent. Excess reagent and the product can be easily separated during routine purification, for example by standard chromatography methods, e.g. ion exchange chromatography or size exclusion chromatography, diafiltration, or, when a polyhistidine tag is present, by separation using metal affinity chromatography, e.g. based on nickel or zinc. Targeting of specific disulfide bonds in the binding protein may be carried out by known methods; for example, by partial reduction of the protein, see for example Liu et al, Anal. Chem. 2010, 82, 5219-5226.

Conjugation reagents of the general formulae VIII above may be prepared by reacting an auristatin with a compound of the general formula:

$$((Lk^{1b})_m-P)_p-Lk^2-Lk^{3a}$$

in which m, P, L, p, $Lk^2$ and $Lk^{3a}$ have the meanings given for the general formula VIII and $Lk^{1b}$ is a group of formula $Lk^1$ modified to include a group reactive with a group present in an auristatin. Typically, $Lk^{1b}$ will be reactive with the terminal nitrogen atom of a monomethyl auristatin. Typical groups and suitable reactions are well known to the skilled man.

Conjugation reagents of the general formulae VIII are novel, and the invention therefore provides these reagents *per se*. In these novel reagents, the preferred meanings for $Lk^1$, P, $Lk^2$, m, p and q are as given above for the general formula I. The invention further provides a pharmaceutical composition comprising an auristatin-protein conjugate according to the invention, together with a pharmaceutically acceptable carrier, optionally together with an additional therapeutic agent; such a conjugate for use in therapy, specifically, for use as a medicament for the treatment of a proliferative, autoimmune or infections disease, for example cancer; and a method of treating a patient which comprises administering a pharmaceutically-effective amount of such a conjugate or pharmaceutical composition to a patient. Particular conditions for which the present invention finds utility include for example leukaemia, including non-Hodgkin's Lymphoma, acute myelogenous leukaemia, multiple myeloma, lymphocytic leukaemias, and chronic myelogenous leukaemia; gastric cancer; breast cancer; ovarian cancer; liver cancer; intestinal cancer; colon cancer; renal cancer, for example renal cell carcinoma; lung cancer, for example small cell lung cancer; melanoma; bladder cancer; and sarcomas.

As mentioned above, one preferred embodiment of the invention relates to specific conjugates containing more than one molecule of an auristatin. Certain drug conjugates having a particular branching structure and containing a broad range of drugs are novel, and in a separate embodiment, the invention also provides these conjugates *per se*. Accordingly, the present invention also provides a conjugate which has the general formula:

$$(((D'_q\text{-}Lk^{1'})_m\text{-}P)_p\text{-}Lk^2\text{-}Lk^3)_n\text{-}Ab \qquad (I')$$

in which D' represents a drug moiety;
q' represents an integer from 2 to 10;
$Lk^{1'}$ represents a linker;
m represents an integer from 1 to 10;
P represents a bond or a z-valent group $-P^1$-NH- where z is from 2 to 11 and $P^1$ is a group containing at least one ethylene unit $-CH_2-CH_2-$ or ethylene glycol unit $-O-CH_2-CH_2-$;
p represents an integer from 1 to 10;
$Lk^2$ represents a bond or a y-valent linker where y is from 2 to 11 and which consists of from 1 to 9 aspartate and/or glutamate residues;
$Lk^3$ represents a linker of the general formula:

$$\text{-CO-Ph-X-Y-} \qquad (II)$$

in which Ph is an optionally substituted phenyl group; X represents a CO group or a CH.OH group; and Y represents a group of formula:

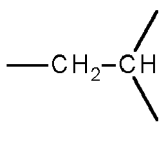
(III) or
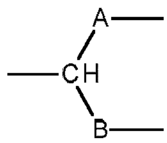
(IV)

in which each of A and B represents a $C_{1-4}$alkylene or alkenylene group;
Ab represents a binding protein or peptide capable of binding to a binding partner on a target, said binding protein being bonded to $Lk^3$ via two sulfur atoms derived from a disulfide bond in the binding protein or peptide; and
n represents an integer from 1 to s where s is the number of disulfide bonds present in the binding protein or peptide prior to conjugation to $Lk^3$;
the meanings of m, n, p, q', y and z being chosen such that the conjugate contains from 1 to 10 D' groups.

In this aspect of the invention, m, P, p, $Lk^2$, $Lk^3$, n and Ab have the meanings and the preferred meanings given above for the aspect of the invention in which D represents an auristatin moiety.

D' may represent any drug which it is desirable to conjugate to a binding protein or peptide. It may for example be a cytotoxic drug. It may for example be an auristatin, as described above, or it may be a maytansine. The term maytansine includes compounds such as maytansine itself, maytansinoids such as 15-methoxyansamitocin P-3, and derivatives thereof.

Preferably a maytansine is a compound containing substructure (A)

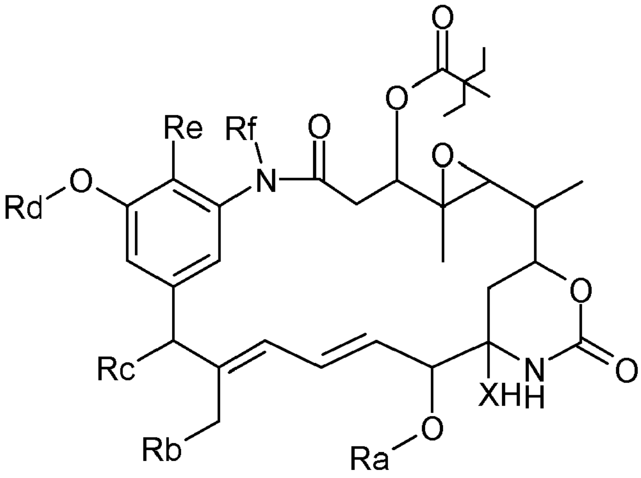

(A),

in which X represents O or S; Ra represents hydrogen or $C_{1-4}$alkyl; Rb represents hydrogen, hydroxy, $C_{1-4}$alkoxy or $C_{1-4}$alkylC(O)O-; Rc represents hydrogen, hydroxy, $C_{1-4}$alkoxy or $C_{1-4}$alkylC(O)O-; Rd represents hydrogen or $C_{1-4}$alkyl; Re represents halogen or hydrogen, and Rf represents hydrogen or $C_{1-4}$alkyl.

Preferably X represents O. Preferably Ra represents $C_{1-4}$alkyl, especially methyl. Preferably Rb represents hydrogen. Preferably Rc represents hydrogen or methoxy, more preferably hydrogen. Preferably Rd represents $C_{1-4}$alkyl, especially methyl. Preferably Re represents chlorine or hydrogen, especially chlorine. Preferably Rf represents $C_{1-4}$alkyl, especially methyl.

More preferably, a maytansine comprises substructure (B)

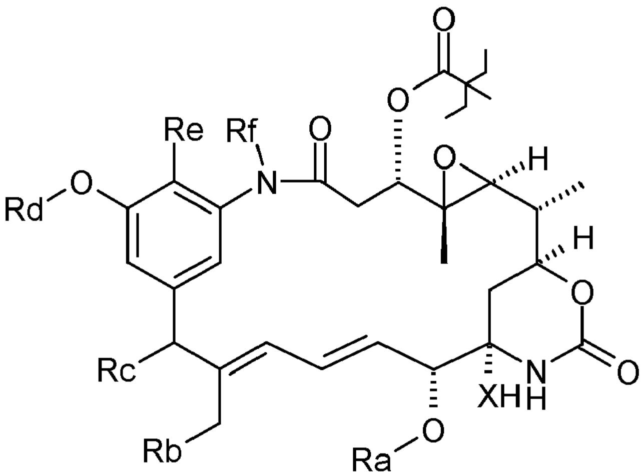

(B),

in which X and Ra-Rf have the meanings set out above.

Still more preferably, a maytansine comprises substructure (C) or (D)

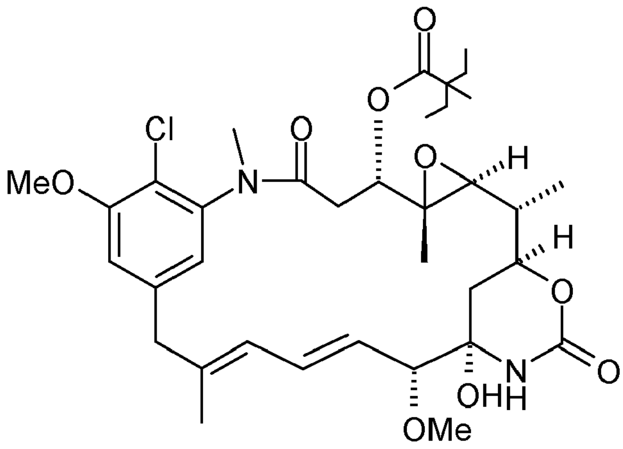

(C),

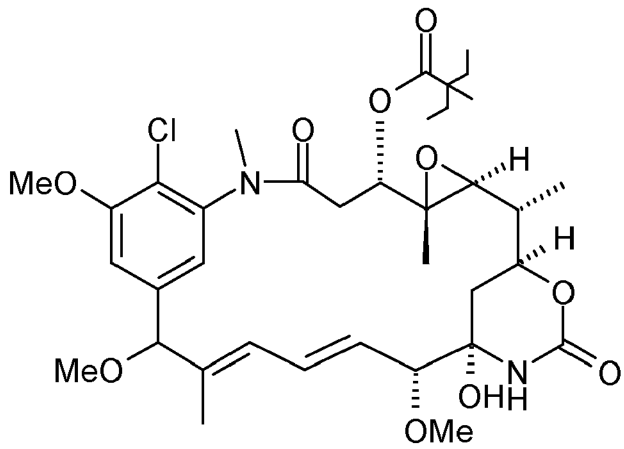

(D),

most preferably (C).

A maytansine may include the following group (E) bonded to the ester carbonyl carbon atom of substructure (A), (B), (C) or (D):

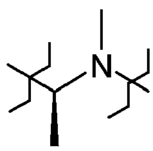

(E),

For example a maytansine may comprise substructure (F):

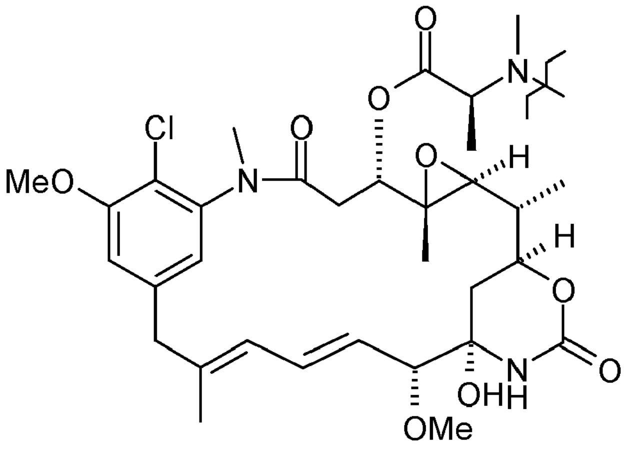

(F).

In some preferred embodiments, a maytansine includes one of the following groups bonded to the ester carbonyl carbon atom of substructure (A), (B), (C) or (D):

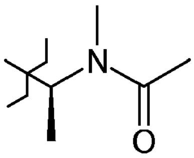

(G),

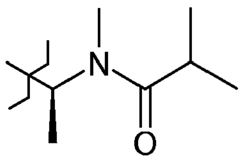

(H),

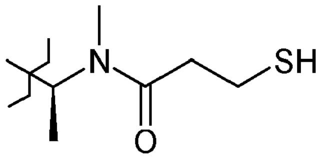

(I),

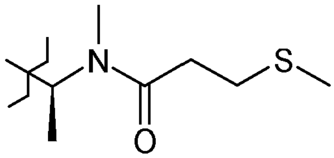

(J),

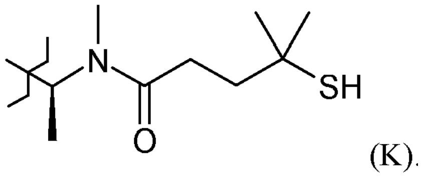

(K).

In some embodiments, a maytansine contains group (L) bonded to the ester carbonyl carbon atom of substructure (A), (B), (C) or (D):

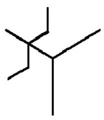

(L).

Examples of specific maytansines include:

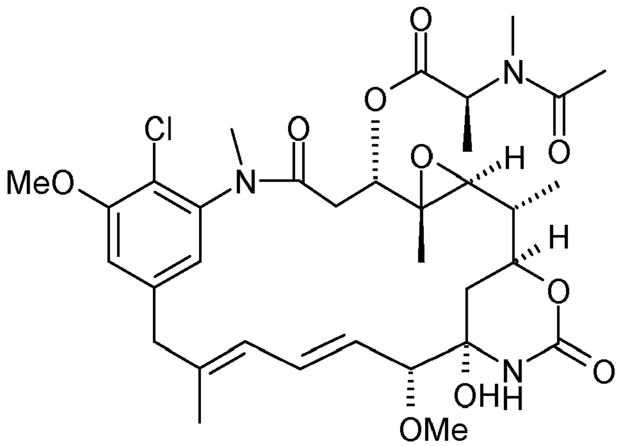

(M) (Maytansine),

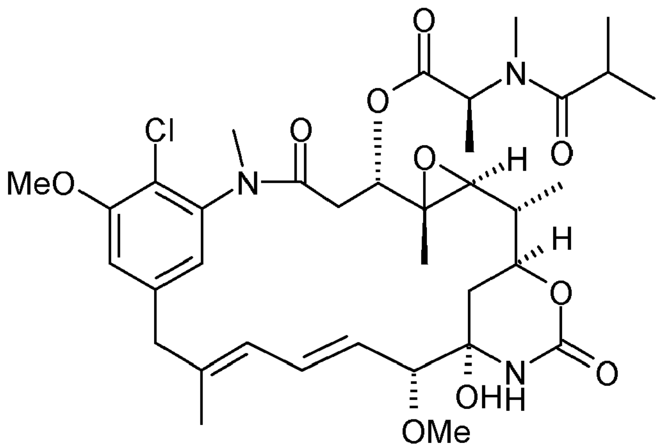

(N),

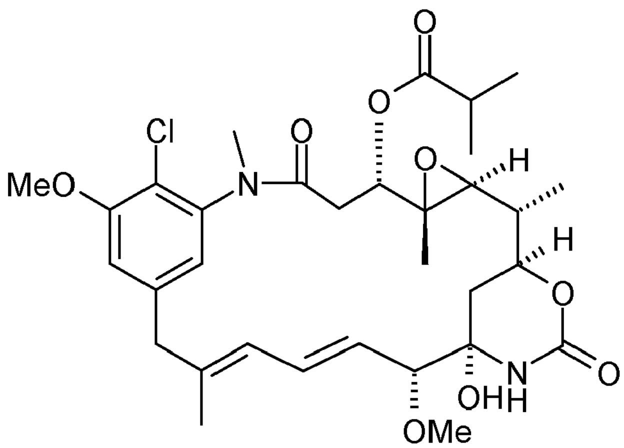

(O) (Ansamitocin P-3),

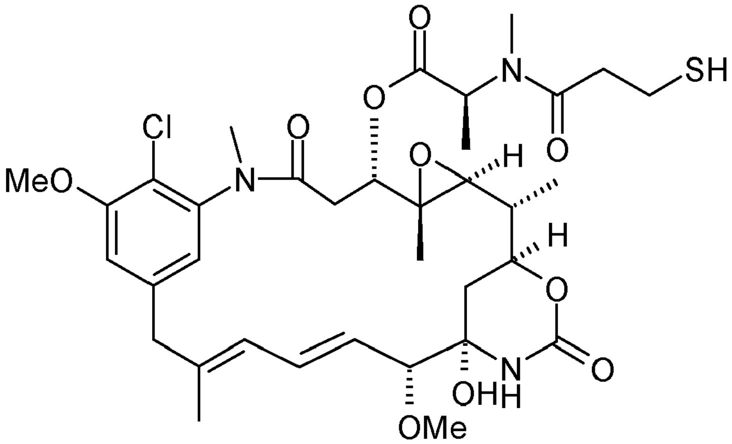

(P) (Mertansine, DM1),

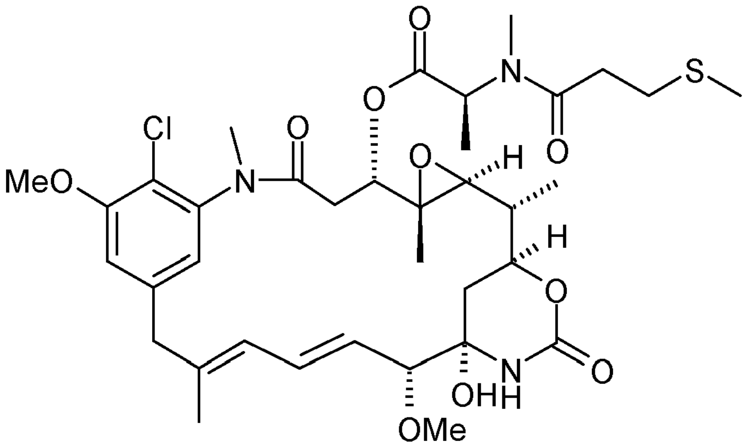

(Q) (S-methyl DM1),

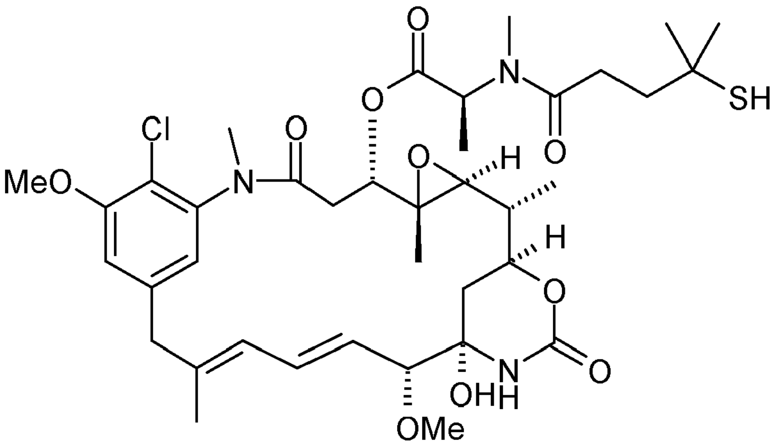

(R) (DM4),

and

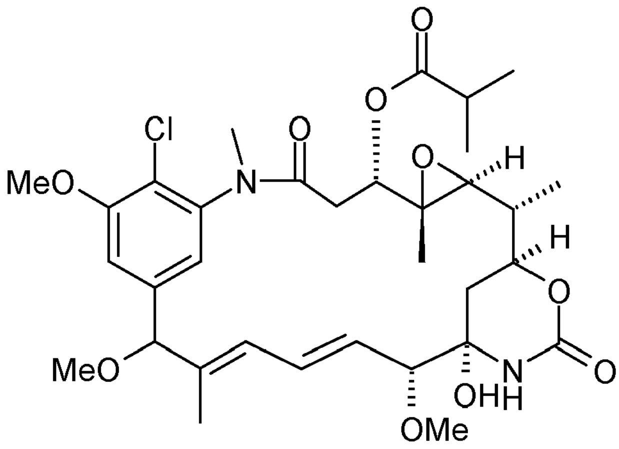

(S) (15-methoxyansamitocin P-3).

$Lk^{1'}$ may be bonded to the drug moiety at any suitable point. It may for example be bonded to an auristatin as shown above for the linker $Lk^1$. Where the D' is a maytansine moiety corresponds to a maytansine comprising group (E), $Lk^{1'}$ may for example be bonded to the nitrogen atom of group (E), e.g.:

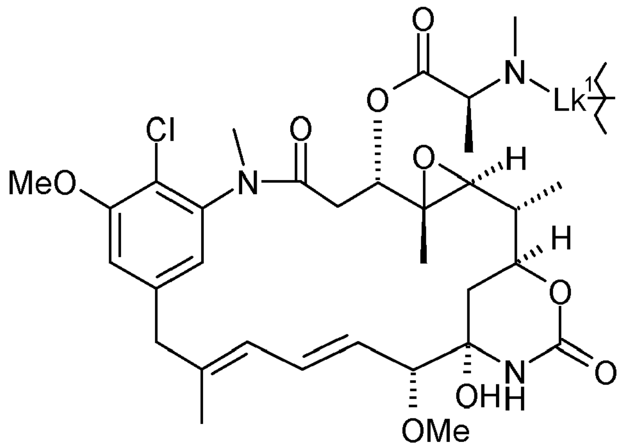

(T).

$Lk^{1'}$ is a linker which connects two or more drug moieties D' to a P group. It may carry from 2 to 10 D' groups. $Lk^{1'}$ preferably contains a degradable group, i.e. $Lk^{1'}$ is preferably a linker which breaks under physiological conditions, separating D' from Ab. Alternatively, $Lk^{1'}$ may be a linker that is not cleavable under physiological conditions. Where $Lk^{1'}$ is a linker which breaks under physiological conditions, it is preferably cleavable under intracellular conditions. Where the target is intracellular, preferably $Lk^{1'}$ is substantially insensitive to extracellular conditions (i.e. so that delivery to the intracellular target of a sufficient dose of the therapeutic agent is not prohibited).

Where $Lk^{1'}$ is a degradable linker, it may contain a group that is sensitive to hydrolytic conditions. $Lk^{1'}$ may contain a group which degrades at certain pH values (e.g. acidic conditions). Hydrolytic/acidic conditions may for example be found in endosomes or lysosomes. Examples of groups susceptible to hydrolysis under acidic conditions include hydrazones, semicarbazones, thiosemicarboazones, cis-acotinic amides, orthoesters and acetals/ketals. Examples of groups susceptible to hydrolytic conditions include:

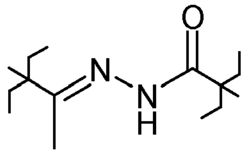

and

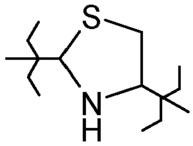

$Lk^{1'}$ may also be susceptible to degradation under reducing conditions. For example, $Lk^{1'}$ may contain a disulfide group that is cleavable on exposure to biological reducing agents, such as thiols. Examples of disulfide groups include:

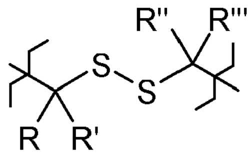

,

in which R, R', R" and R"' are each independently hydrogen or $C_{1-4}$alkyl, and

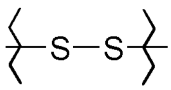

$Lk^{1'}$ may also contain a group which is susceptible to enzymatic degradation, for example it may be susceptible to cleavage by a protease (e.g. a lysosomal or endosomal protease) or peptidase. For example, $Lk^{1'}$ may contain a peptidyl group comprising at least one, for example at least two, or at least three, amino acid residues (e.g. Phe-Leu, Gly-Phe-Leu-Gly, Val-Cit, Phe-Lys). For example, $Lk^{1'}$ may be an amino acid chain having from 1 to 5, for example 2 to 4, amino acids. Another example of a group susceptible to enzymatic degradation is:

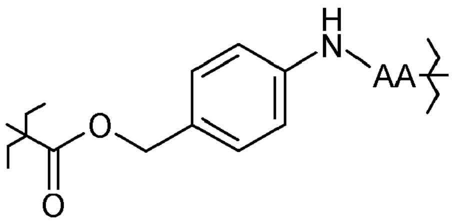

,

wherein AA represents a protease-specific amino acid sequence, such as Val-Cit.

$Lk^{1'}$ may include:

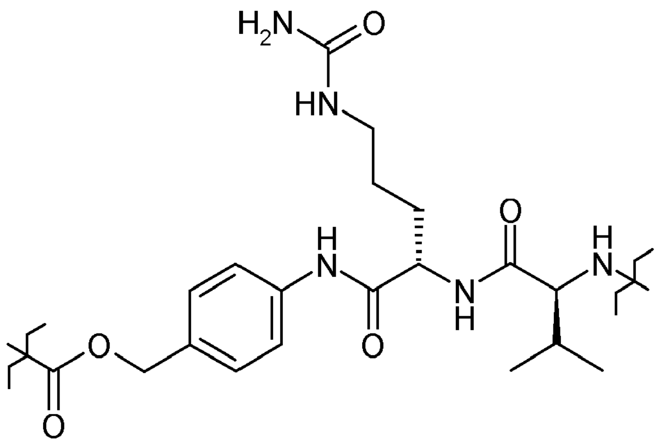

(V)

For example, $Lk^{1'}$ may include

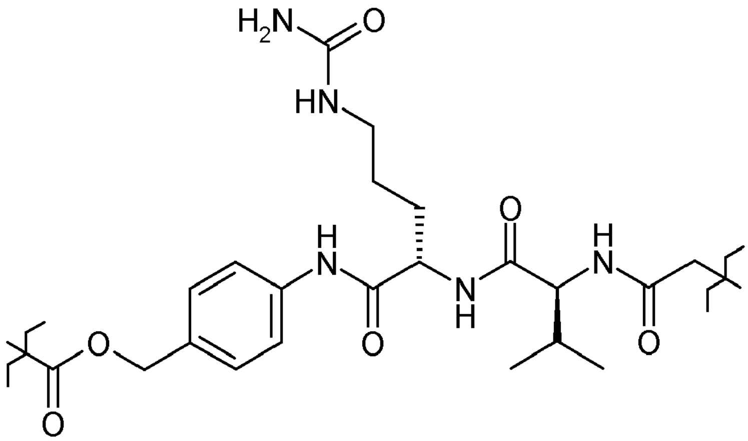

(Va)

In the embodiment of the invention represented by formula I', q' is greater than 1, for example 2, 3 or 4, and $Lk^{1'}$ is used as a means of incorporating more than one drug moiety into a conjugate of the invention. $Lk^{1'}$ is a branching linker, which may for example incorporate an aspartate or glutamate or similar residue. This introduces a branching element of formula:

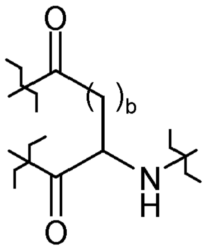

(VI)

where b is 1, 2 or 3, b=1 being aspartate and b=2 being glutamate, and b=3 forming one preferred embodiment of the invention. Each of the acyl moieties in the formula VI may be coupled to a group D' via a suitable linker $Lk^{1a}$, where $Lk^{1a}$ is any suitable linker, for example a degradable linker incorporating one of the linkages mentioned above for $Lk^{1'}$. In one particular embodiment, $Lk^{1a}$ represents the group V shown above. The amino group of the aspartate or glutamate or similar residue may be bonded to P by any suitable means, for example the linkage may be via an amide bond, e.g. the branching group above may be connected to P via a $-CO.CH_2-$ group, thus:

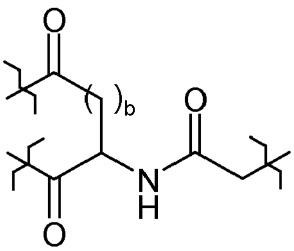

(VIa)

If desired, the aspartate or glutamate or similar residue may be coupled to further aspartate and/or glutamate and/or similar residues, for example:

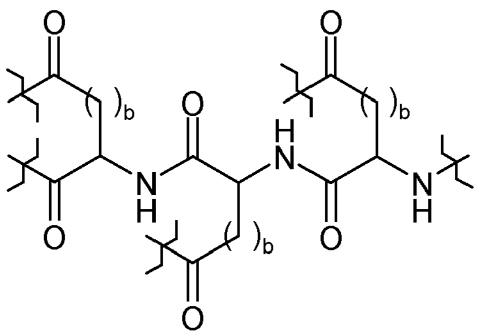

(VIIa) or

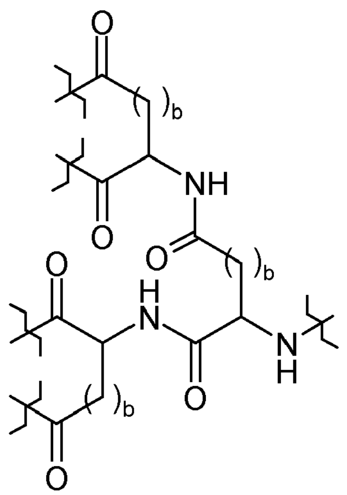

(VIIb)

and so on, up to a maximum of 9 such residues, giving the potential to incorporate up to 10 D' groups. As above, each D' may be attached to an aspartate/glutamate or similar residue via any suitable linker $Lk^{1a}$.

A preferred conjugate of formula I' has the formula:

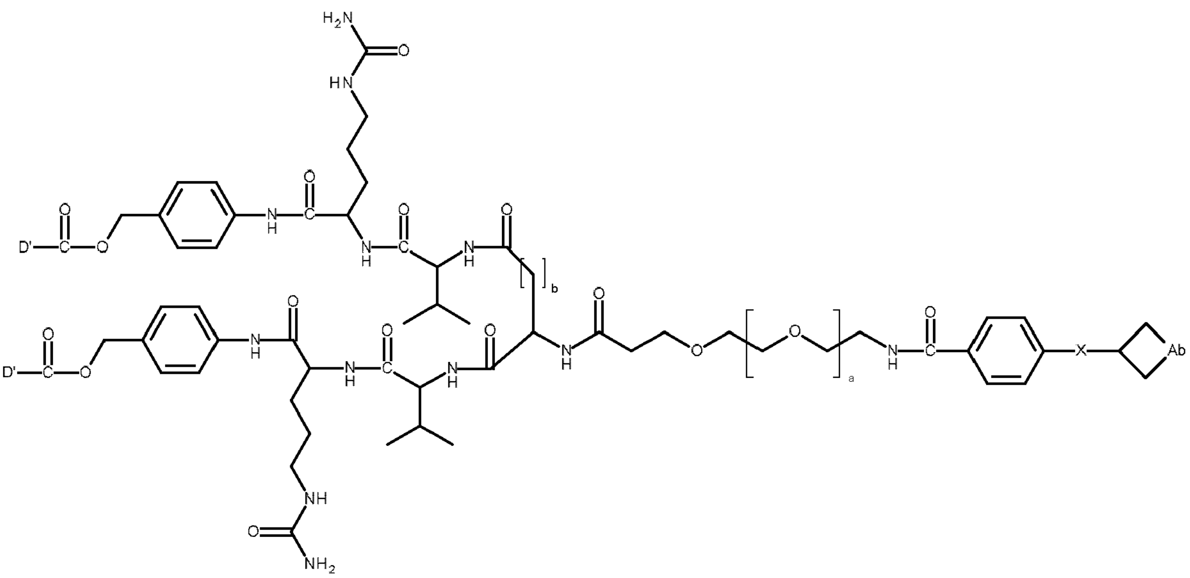

in which a, b and Ab have the meanings given above. D' may for example be a maytansine or, especially, an auristatin, as detailed above.

The conjugates of Formula I' may be prepared as described above, *mutatis mutandis,* for the conjugates of Formula I, save that $Lk^{1'}$ replaces $LK^1$, D' replaces D, and q is greater than 1.

Conjugating reagents of the formula:

$$((D'_{q'}\text{-}Lk^{1'})_m\text{-}P)_p\text{-}Lk^2\text{-}Lk^{3a} \qquad \text{(VIII')}$$

in which D', q', $Lk^{1'}$, m, P, p, $Lk^2$ and $Lk^3$ have the meanings and preferred meanings given above, are novel, and form part of this invention. They may be prepared, mutatis mutandis, by the methods described above for the reagents of formula VIII. A particularly preferred conjugating reagent according to this aspect of the invention has the formula:

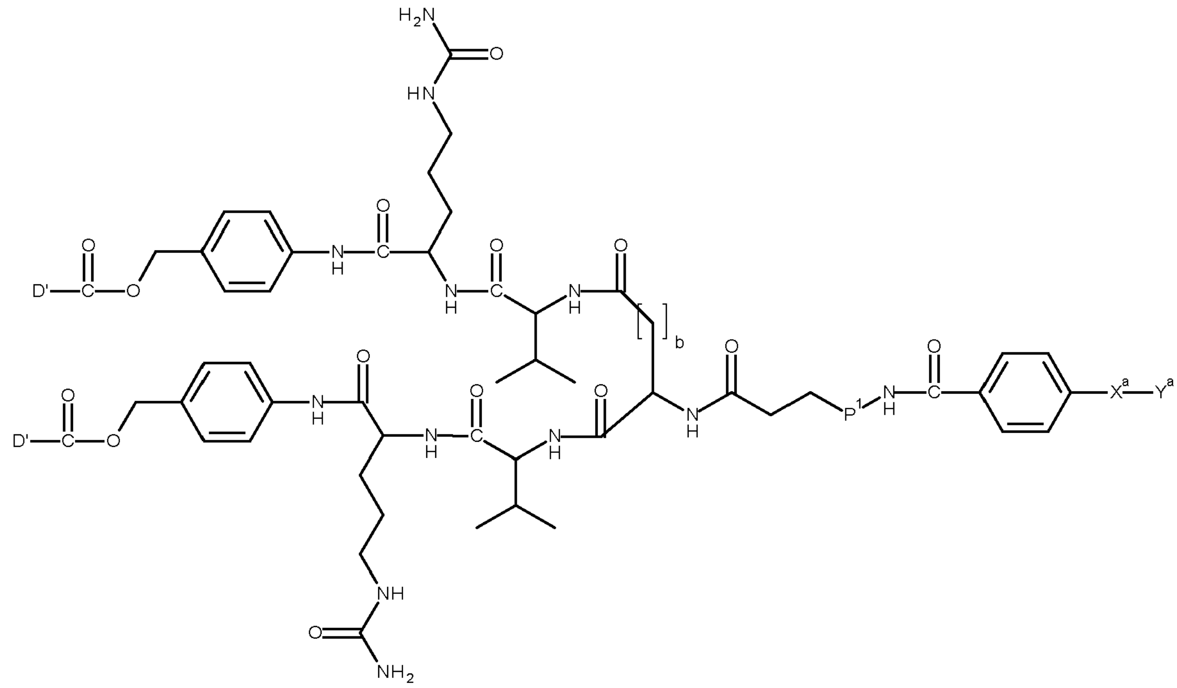

in which b is 1, 2 or 3. $Y^a$ is preferably a group of formula (XII), especially one in which A and B are each $-CH_2-$. $P^1$ is preferably PEG, especially one of those mentioned above, and preferably one having from 15 to 35 repeating $-O-CH_2-CH_2-$ units. Also preferred are equivalent structures in which Ab carries 2, 3 or, preferably, 4, copies of the ~X-group in the above formula.

The invention further provides a pharmaceutical composition comprising a conjugate of Formula I' according to the invention, together with a pharmaceutically acceptable carrier, optionally together with an additional therapeutic agent; such a conjugate for use in therapy, specifically, for use as a medicament for the treatment of a proliferative, autoimmune or infectious disease, for example cancer; and a method of treating a patient which comprises administering a pharmaceutically-effective amount of such a conjugate or pharmaceutical composition to a patient. Particular conditions for which such conjugates may be used include those mentioned above.

Surprisingly, it has been found that compounds of the formula I' have improved activity compared with corresponding conjugates containing the same number of drug molecules D' in which $Lk^{1'}$ is not a branching linker and q' is 1, but in which branching is introduced into the molecule by use of a branching linker $Lk^2$.

Specific features of the aspect of the invention relating to conjugates of the formula I' are as follows.

1. A conjugate of formula I' as defined above.
2. A conjugate as defined in clause 1, wherein D' is a cytotoxic drug.
3. A conjugate as defined in clause 1, wherein D' is an auristatin or a maytansine.
4. A conjugate as defined in clause 3, wherein D' is monomethylauristatin E or monomethylauristatin F bonded via its terminal nitrogen atom.
5. A conjugate as defined in clause 4, wherein the auristatin is monomethylauristatin E bonded via its terminal nitrogen atom.
6. A conjugate as defined in any one of the preceding clauses, in which $Lk^{1'}$ is a degradable linker.
7. A conjugate as defined in clause 6, in which $Lk^{1'}$ includes one of the following groups:

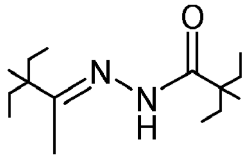

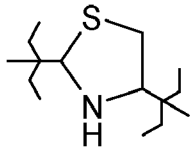

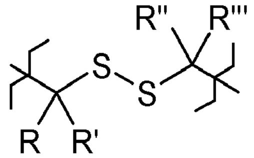

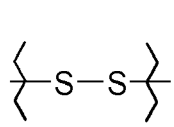

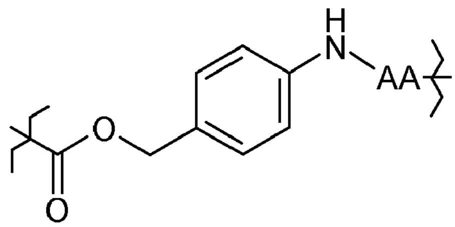

in which each of R, R', R" and R"' represents a hydrogen atom or an alkyl group and AA represents a protease-specific amino acid sequence.

8. A conjugate as defined in clause 7, in which $Lk^{1'}$ includes:

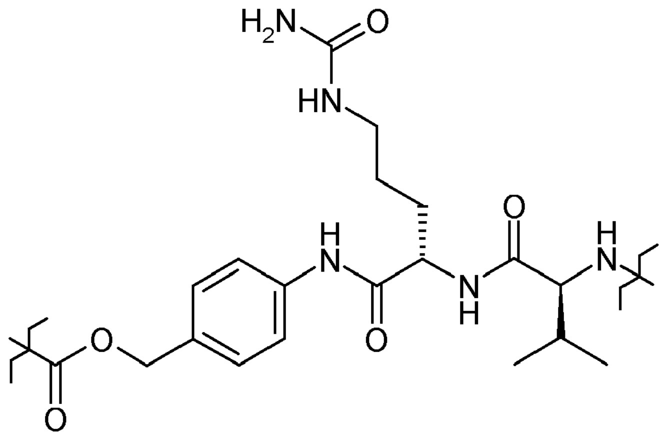

(V)

9. A conjugate as defined in any one of clauses 1 to 8, in which $Lk^{1'}$ includes an element of formula:

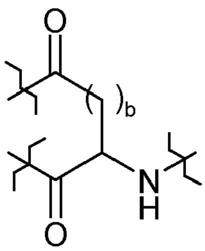

(VI)

in which b is 1, 2 or 3.

10. A conjugate as defined in any one of clauses 1 to 5, which has the formula:

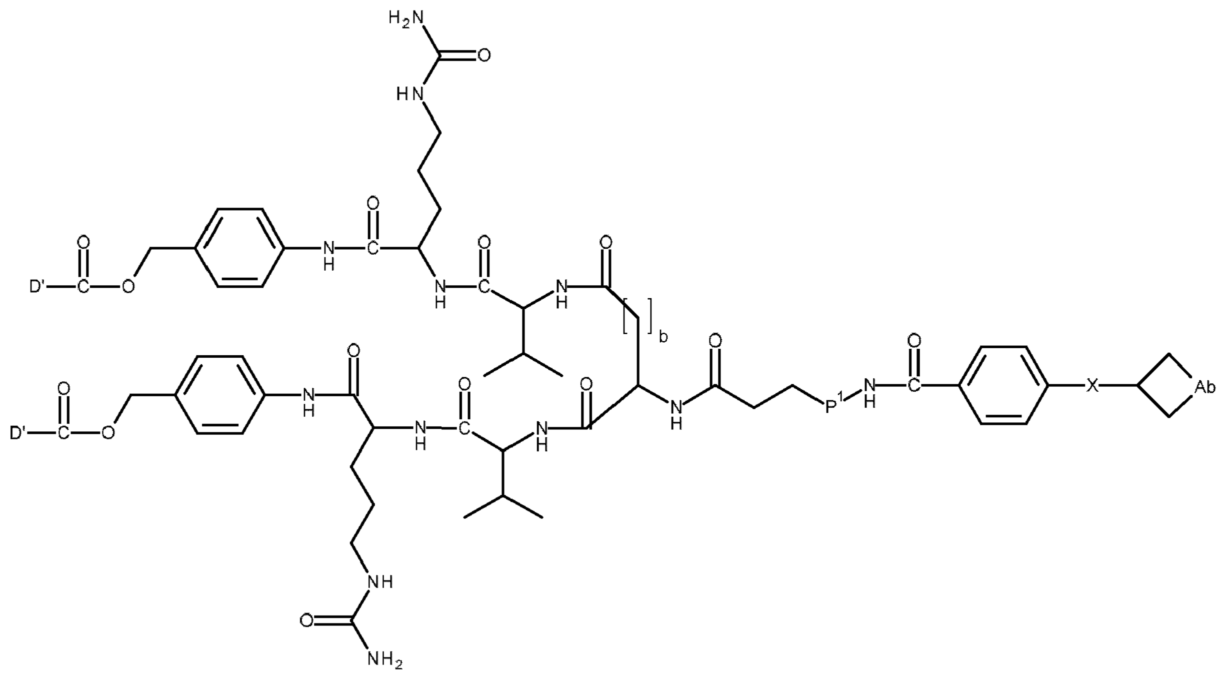

in which b is 1, 2 or 3; and equivalent structures in which Ab carries 2, 3 or 4 copies of the ~X- group.

11. A conjugate as defined in any one of the above clauses, in which P represents a bond, or P represents -$P^1$-NH- wherein $P^1$ contains from 2 to 10 ethylene glycol units.

12. A conjugate as defined in any one of the above clauses, in which P represents polyethylene glycol.

13. A conjugate as defined in any one of the above clauses, in which the phenyl group Ph in $Lk^3$ is unsubstituted.

14. A conjugate as defined in any one of the above clauses, in which Y has the formula:

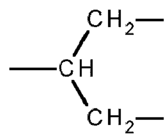

15. A conjugate as defined in any one of the above clauses, in which Ab represents a full length antibody or an antibody fragment comprising an antigen-binding region of the full length antibody.

16. A conjugate as defined in any one of the above clauses, in which Ab represents IgG1 or IgG4 or a fragment of IgG1 or IgG4.

17. A process for the preparation of a conjugate as defined in any one of the above clauses, which comprises reducing one or more disulfide bonds in a binding protein and subsequently reacting with a conjugating reagent of the general formula:

$$((D'_{q'}\text{-}Lk^1)_m\text{-}P)_p\text{-}Lk^2\text{-}Lk^{3a} \qquad \text{(VIII')}$$

in which D', $Lk^{1'}$, P, $Lk^2$ and m, p and q' have the meanings given in clause 1, and $Lk^{3a}$ represents a group of formula:

$$\text{-CO-Ph-}X^a\text{-}Y^a \qquad \text{(IX)}$$

in which Ph has the meaning given in claim 1, $X^a$ represents a CO group, and $Y^a$ represents a group:

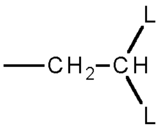 (X), 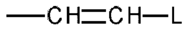 (XI), 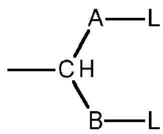 (XII)

or

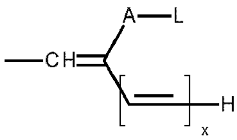 (XIII)

in which A and B have the meanings given in clause 1, each L independently represents a leaving group, and x represents an integer from 1 to 4, to produce a conjugate of formula I' in which X represents CO; and optionally reducing said initially-formed CO group X to give a conjugate having a CH.OH group X.

18. A process as defined clause 17, in which $Y^a$ represents:

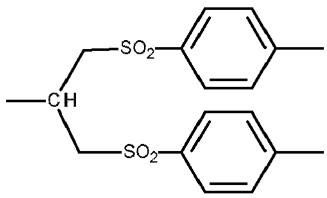 (XIIa)

19. A compound of the general formula:

$$((D'_{q'}\text{-}Lk^{1'})_m\text{-}P)_p\text{-}Lk^2\text{-}Lk^{3a} \qquad (VIII')$$

in which D' has the meaning given in any one of clauses 1 to 5; $Lk^{1'}$ has the meaning given in any one of clauses 1 and 6 to 9; P has the meaning given in any one of clauses 1,10 and 11; $Lk^2$, m, p and q have the meanings given in clause 1, and $Lk^{3a}$ represents a group of formula:

$$\text{-CO-Ph-}X^a\text{-}Y^a \qquad (IX)$$

in which Ph has the meaning given in either clause 1 or clause 10, $X^a$ represents a CO group, and $Y^a$ represents a group:

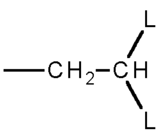 (X), 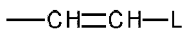 (XI), 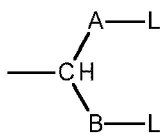 (XII)

or

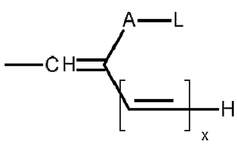 (XIII)

in which A and B have the meanings given in clause 1, each L independently represents a leaving group, and x

represents an integer from 1 to 4.

19. A compound as defined in clause 18, in which $Y^a$ represents:

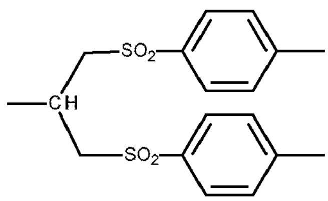

(XIIa)

20. A pharmaceutical composition comprising a conjugate as defined in any one of clauses 1 to 15, together with a pharmaceutically acceptable carrier, optionally together with an additional therapeutic agent.

21. A conjugate as defined in any one of clauses 1 to 15 or a composition as defined in clause 20 for use in therapy.

22. A method of treating a patient which comprises administering a pharmaceutically-effective amount of a conjugate as defined in any one of clauses 1 to 15 or a pharmaceutical composition as defined in clause 20 to a patient.

Conjugates of the present invention demonstrate a number of important advantages, the existence of which could not have been predicted. Compared with equivalent drug-antibody conjugates prepared using maleimide reagents, as currently used in commercially available conjugates, they demonstrate significantly increased stability. Further, their method of synthesis leads to a product with a significantly improved homogeneity in respect of drug-antibody ratio, compared with the use of maleimide. Homogeneity is advantageous for drug substances for regularity of production of the drug substance. A process which generates a greater yield of a single DAR species will be cheaper through greater efficiency. A drug product of a single DAR species produced by purification of that DAR species from a heterogeneous mixture would be prohibitively expensive to produce in large quantities.

Further, a single DAR species will demonstrate more predictable pharmacokinetics, safety, toxicity and tolerability as all the drug substance should be metabolised in a similar manner, giving the same products, as opposed to a mixed DAR substance which may be metabolised differently or at different rates, giving more heterogeneous break down products.

The following Examples illustrate the invention.

Example 1: Synthesis of valine-citrulline-paraaminobenzyl-monomethyl auristatin E (val-cit-PAB-MMAE) reagent 1 possessing a 24 repeat unit PEG with terminal bis-sulfone functionality.

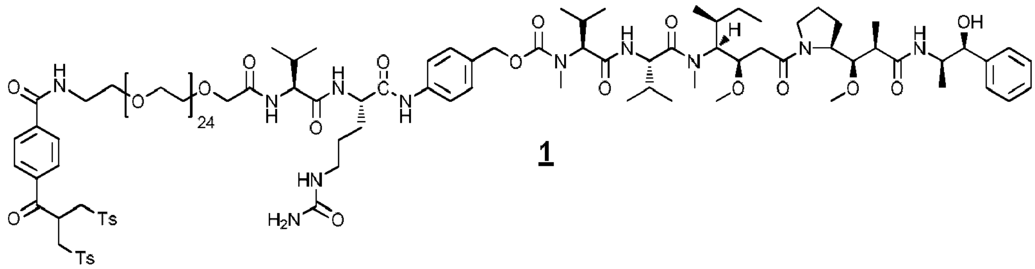

**1**

Step 1: Conjugation of 4-[2,2-bis[(p-tolylsulfonyl)-methyl]acetyl]benzoic acid-N-hydroxy succinimidyl ester (bis-sulfone) to $H_2N$-dPEG(24)-CO-OtBu.

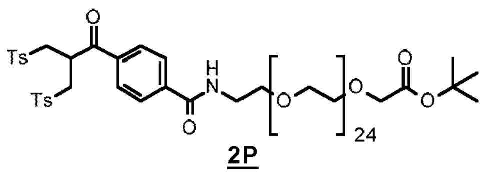

**2P**

A toluene (3 mL) solution of $H_2N$-dPEG(24)-CO-OtBu (1.057 g, Iris Biotech) was evaporated to dryness and the residue re-dissolved in dichloromethane (25 mL). Under stirring, 4-[2,2-bis[(p-tolylsulfonyl)-methyl]acetyl]benzoic acid-N-hydroxy succinimidyl ester (1.0 g; Nature Protocols, 2006, 1 (54), 2241-2252) was added and the resulting solution further stirred for 72 h at room temperature under an argon atmosphere. Volatiles were removed *in vacuo* and the solid residue was dissolved in warm acetone (30 mL) and filtered through non-absorbent cotton wool. The filtrate was cooled to -80°C to precipitate a solid which was isolated by centrifugation at -9°C, for 30 min at 4000 rpm. The supernatant was removed and the precipitation/isolation process repeated 2 additional times. Finally the supernatant was removed and the resulting solid was dried *in vacuo* to give the bis-sulfone protected acid **2P** as a colourless amorphous solid (976 mg, 68%). $^1$HNMR $\partial_H$ (400 MHz $CDCl_3$)1.45 (9H, s, O$^t$Bu), 2.40-2.45 (8H, m, Ts-Me and *$CH_2COO^tBu$),* 3.40-3.46 (2H, m, *$CH_2$-Ts),* 3.52-3.66 (m, PEG and *$CH_2$-Ts),* 4.27 (1H, q, *J6.3, CH*-COAr), 7.30 (4H, d, J8.3, Ts), 7.58 (2H, d, J8.6, Ar), 7.63 (4H, d, J8.3, Ts), 7.75 (2H, d, J 8.6, Ar).

Step 2: Removal of the tert-butyl protection group:

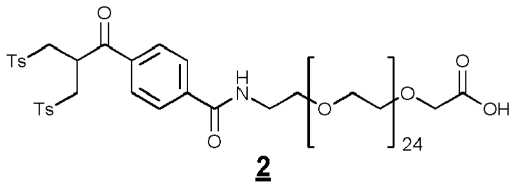

**2**

To a stirred solution of the product of step 1 (976 mg) in dichloromethane (4 mL) was added trifluoroacetic acid (4 mL) and the resulting solution was stirred for a further 2 h. Volatiles were then removed *in vacuo* and the residue was dissolved in warm acetone (30 mL). The product was isolated by precipitation from acetone as described in step 1 to give afford the product **2** as a white powder (816 mg, 85%). $^1$HNMR $\partial_H$ (400 MHz $CDCl_3$) 2.42 (6H, s, Ts-Me), 2.52 (2H, t, *J 6.1, $CH_2$-COOH),* 3.42 (4H, dd, *J* 6.3 & 14.5, *$CH_2$*-Ts), 3.50-3.64 (m, PEG), 3.68-3.73 (4H, m, PEG), 4.23-4.31 (1H, m, *CH*-COAr), 7.29 (2H, d, *J* 8.1, Ar), 7.55-7.65 (6H, m, Ar and Ts), 7.77 (2H, d, *J* 8.2, Ar)

Step 3: Conjugation of $H_2N$-val-cit-PAB-MMAE to acid terminated PEGylated bis-sulfone **2**

N-methyl morpholine (7.5 mg) was added to a stirred solution of bis-sulfone-PEG-COOH (45 mg) and HATU (13 mg) in dichloromethane-dimethylformamide (85:15 v/v, 6 mL). After stirring for 30 min at room temperature, the $H_2N$-val-cit-PAB-MMAE (38 mg, Concortis, prepared as in WO 2005/081711) was added and the mixture further stirred for 24 h at room temperature. The reaction mixture was diluted with dichloromethane and washed with 1 M HCl, aqueous $NaHCO_3$ 10% w/v, brine and then dried with $MgSO_4$. The crude material was further purified by column chromatography eluting with dichloromethane-methanol (90:10 v/v), the solvent was removed under vacuum and the bis-sulfone-PEG(24)-MMAE product **1** was isolated as a transparent colourless solid (31 mg, 41%) m/z M+Na 2758.5; diagnostic signals for $^1$HNMR $\partial_H$ (400 MHz $CDCl_3$)0.60-0.99 (m, aliphatic side chains), 2.43 (s, *Me*-Ts), 3.36-3.66 (m, PEG), 7.15-7.28 (m, Ar), 7.31 (d, J8.3, Ar), 7.54-7.62 (m, Ar), 7.79 (d, *J* 8.3, Ar).

Example 2: Synthesis of monomethyl auristatin E (MMAE) reagent **3** possessing a 5 kDa PEG with terminal bis-sulfone functionality

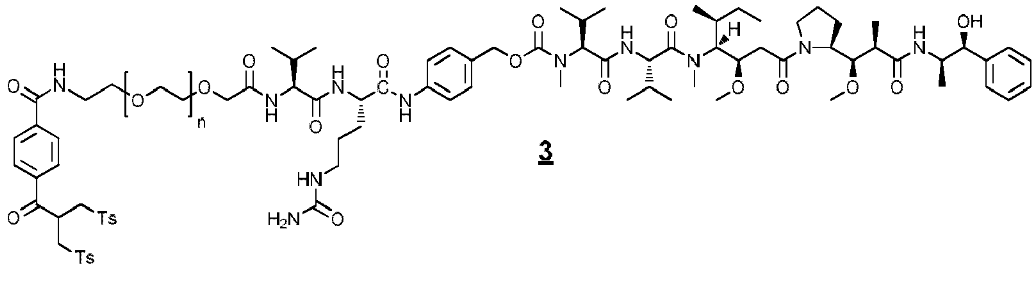

**3**

Step 1: Conjugation of 4-[2,2-bis[(p-tolylsulfonyl)-methyl]acetyl]benzoic acid-N-hydroxy succinimidyl ester (bis-sulfone) to 5kDa HCl.$H_2$N-PEG-COOH **4**.

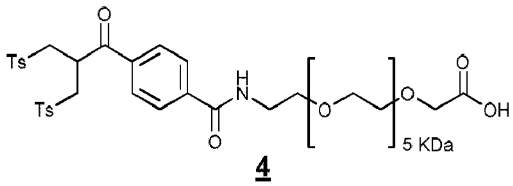

**4**

N-methyl morpholine (0.004 mL) was added to a stirred solution of 5 kDa HCl.$H_2$N-PEG-COOH (100 mg, Iris Biotech) and 4-[2,2-bis[(p-tolylsulfonyl)-methyl]acetyl]benzoic acid-N-hydroxy succinimidyl ester (49 mg) in dichloromethane (5 mL) and stirred at room temperature under an argon atmosphere for 48 h. Volatiles were removed *in vacuo* and the solid residue was dissolved in warm acetone (7 mL) and filtered through non-absorbent cotton wool. The filtrate was cooled to -80 °C to precipitate a solid which was isolated by centrifugation at -9 °C, for 30 min at 4000 rpm. The supernatant was removed and the precipitation/isolation process repeated 2 additional times. Finally the supernatant was removed and the resulting solid was dried *in vacuo* to give the 5 kDa PEG reagent **4** as a white powder (90 mg, 80 %) $^1$HNMR $\partial_H$ (400 MHz $CDCl_3$) 2.36 (2H, t, J7.0, *$CH_2$*-COOH)*,* 2.42 (6H, s, Ts-Me), 3.40-3.75 (m, PEG and *$CH_2$-Ts),* 4.24-4.28 (1H, m, *CH*-COAr), 7.30 (4H, d, J 8.2, Ts), 7.57 (2H, d, J 8.3, Ar), 7.62 (4H, d, J 8.2, Ts), 7.77 (2H, d, J 8.3, Ar)

Step 2: Conjugation of $H_2$N-val-cit-PAB-MMAE to acid terminated 5 kDa PEGylated bis-sulfone from Step 1.

HATU (5 mg) was added to a stirred suspension of $Na_2CO_3$ (3 mg), $H_2$N-val-cit-PAB-MMAE (11 mg) and the product from Step 1 (35 mg) in an anhydrous mixture of dichloromethane and DMF (1 mL) and stirred for 2 days under an argon atmosphere. The dichloromethane was removed under vacuum and the reaction mixture purified by precipitation from cold acetone (3 × 5 mL) in an analogous manner to the method described in Step 1. The solid was dried *in vacuo* to afford bis-sulfone-PEG(5 kDa)-MMAE 3 as a colourless amorphous solid (42 mg, 95 %). Diagnostic signals $^1$HNMR $\partial_H$ (400 MHz $CDCl_3$)0.65-0.99 (m, aliphatic side chains), 3.48-3.71 (m, PEG), 7.15-7.34 (m, Ar), 7.62-7.69 (m, Ar) 7.72 (d, *J*8.3, Ar), 7.81 (d, J8.3, Ar).

Example 3: Synthesis of monomethyl auristatin E (MMAE) reagent **5** directly linked to a terminal bis-sulfone functionality

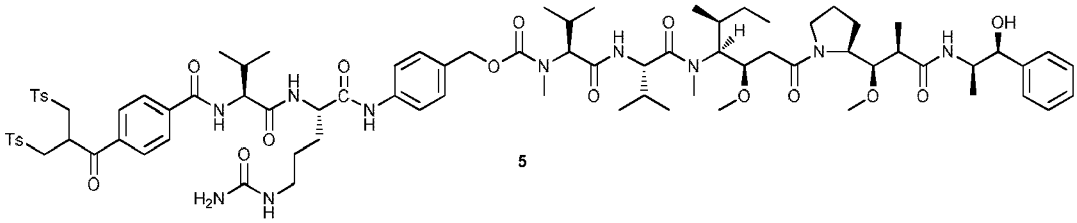

**5**

Step 1: Conjugation of 4-[2,2-bis[(p-tolylsulfonyl)-methyl]acetyl]benzoic acid-N-hydroxy succinimidyl ester (bis-sulfone) to $NH_2$-val-cit-PAB-MMAE.

4-[2,2-bis[(p-tolylsulfonyl)-methyl]acetyl]benzoic acid-N-hydroxy succinimidyl ester (35 mg) was added to a stirred suspension of sodium carbonate (3 mg) and $NH_2$-val-cit-PAB-MMAE (35 mg) in an anhydrous mixture of DCM-DMF (3:1, 1 mL) and stirred for 24 hours under an argon atmosphere. The product was purified by column chromatography eluting with dichloromethane-methanol (95:5, *v/v)* to give the bis-sulfone-MMAE 5 as a colourless amorphous solid (22 mg, 33%) m/z M+Na 1629.5; Diagnostic signals $^1$HNMR $\partial_H$ (400 MHz $CDCl_3$) 0.56-0.97 (m, aliphatic side chains) 2.36 (s, Me-Ts), 7.10-7.32 (m, Ar), 7.48-7.64 (m, Ar), 7.68 (d, *J 8.22,* Ar), 7.74-7.90 (m, Ar).

Example 4: Preparation of variant antibody-drug conjugates

Two engineered antibody variants, each having a single inter-heavy chain disulfide bond, were created by

PCR-based site-directed mutagenesis of the parent antibody sequence in order to demonstrate that antibody conjugates can be produced at high levels of homogeneity and with a low average DAR. These antibody variants and the parent antibody were reacted with a conjugating reagent (Bis-sulfone-PEG(24)-val-cit-PAB-MMAE **1**) prepared according to Example 1, which forms a bridge between the two cysteine residues derived from a disulfide bond.

Preparation of the parent antibody and antibody variants (IgGC226S and IgGC229S).

The construction of the parent antibody DNA sequence, encoding a humanised anti-Her2 receptor monoclonal antibody variant (trastuzumab) generated based on human IgG1(κ) framework, has previously been described in Carter P. et al. Proc. Natl Acad. Sci. USA, 89, 4285-4289 (1992), where the antibody is referred to as humAb4D5-8. For the purposes of the present experiments, the amino acids at positions 359 and 361 of the heavy chain amino acid sequence were replaced with Asp and Leu, respectively (E359D and M361L). The light and heavy chain amino acid sequences of the parent antibody used in the present experiments are also shown herein by SEQ ID NOs: 1 and 2, respectively. Two of the cysteines in the hinge region of the parent antibody (hinge region sequence: PKSCDKTHTCPPCP) form inter-chain disulfide bonds between the two heavy chains of the antibody. These cysteine residues correspond to positions 226 and 229 of IgG1 according to the EU-index numbering system, and are residues 229 and 232 of SEQ ID NO: 2.

The two engineered antibody variants (IgGC226S and IgGC229S) were created by PCR-based site-directed mutagenesis of the parent antibody heavy chain sequence to substitute one of the inter-heavy chain cysteine residues in the hinge region with the amino acid Ser. The PCR methodology used was primer overlapping extension, as described by Ho et al. Gene, 77 (1989) 51-59, to generate a modification in the hinge region sequence. PCR primer oligonucleotides were designed to incorporate nucleotide changes into the coding sequence of the subject antibody. In the Cys226Ser variant, the codon change was from TGC (Cys) to AGC (Ser). In the Cys229Ser variant, the codon change was from TGC (Cys) to AGT (Ser). The new sequence was cloned back into heavy chain expression vector, including other portions of the heavy chain. Final construct (after mutagenesis) was verified by full length sequencing of the insert.

The newly generated heavy chain construct was co-transfected with the corresponding light chain construct into HEK293 cells using polyethylenimine (PEI), expressed in a 6 day transient culture, and purified by a combination of Protein A and Size Exclusion Chromatography, based on the protocol from "Transient Expression in HEK293-EBNA1 Cells," Chapter 12, in Expression Systems (eds. Dyson and Durocher). Scion Publishing Ltd., Oxfordshire, UK, 2007.

Conjugation of Bis-sulfone-PEG(24)-val-cit-PAB-MMAE **1** to parent antibody and antibody variants.

Conjugation of the antibody variants with 1, 1.5 or 2 equivalents of the polymeric conjugation reagent Bis-sulfone-PEG(24)-val-cit-PAB-MMAE **1** per inter-chain disulfide bond was performed after antibody reduction. The reduction reactions were carried out at 4.7 mg/mL antibody concentration using 10 mM DTT for 1 h, at either 22°C or 40°C. Buffer exchange was performed for each antibody variant to remove excess reductant. The polymeric conjugation reagent was prepared in 50% aq. acetonitrile at pH 8 immediately before conjugation. Antibody concentrations during conjugation were 3 mg/mL and the reactions were conducted overnight (16 h), at either 40°C or 22°C. Reaction conditions for the conjugation reactions are summarised in Table 1 below:

Table 1: Conjugation conditions.

| | **Reaction 1** | **Reaction 2** | **Reaction 3** | **Reaction 4** | **Reaction 5** | **Reaction 6** |
|---|---|---|---|---|---|---|
| **mAb** | IgGC226S | IgGC226S | IgGC226S | IgGC226S | IgGC226S | IgGC226S |
| **Reagent eq. per S-S** | 1 eq. | 1.5 eq. | 2 eq. | 1 eq. | 1.5 eq. | 2 eq. |
| **Temp. / °C** | 40 | 40 | 40 | 22 | 22 | 22 |
| | | | | | | |
| | **Reaction 7** | **Reaction 8** | **Reaction 9** | **Reaction 10** | **Reaction 11** | **Reaction 12** |
| **mAb** | IgGC229S | IgGC229S | IgGC229S | IgGC229S | IgGC229S | IgGC229S |
| **Reagent eq. per S-S** | 1 eq. | 1.5 eq. | 2 eq. | 1 eq. | 1.5 eq. | 2 eq. |
| **Temp. / °C** | 40 | 40 | 40 | 22 | 22 | 22 |

The "IgGC226S" variant has a Cys to Ser substitution at position 226, and thus a single inter heavy-chain disulfide bond at position 229. The "IgGC229S" variant has a Cys to Ser substitution at position 229, and thus a single inter heavy-chain disulfide bond at position 226.

After buffer exchange, each reaction was analysed by Hydrophobic Interaction Chromatography (HIC) to determine the stoichiometry of drug loading using % area of the peaks at 280 nm, as previously described. The average Drug to Antibody Ratio (DAR) and the drug conjugate species distribution (DAR 1-3) of the antibody-drug conjugates produced are shown in Table 2.

Table 2: Average DAR and species distribution for IgGC226S (reactions 1 to 6) and IgGC229S (reactions 7 to 12)-drug conjugates.

| Reaction | Average DAR | DAR 1-3 | Reaction | Average DAR | DAR 1-3 |
|---|---|---|---|---|---|
| 1 | 1.41 | 80% | 7 | 1.43 | 78% |
| 2 | 1.99 | 89% | 8 | 1.76 | 83% |
| 3 | 2.42 | 87% | 9 | 2.65 | 76% |
| 4 | 1.33 | 79% | 10 | 1.26 | 76% |
| 5 | 1.96 | 90% | 11 | 2.11 | 88% |
| 6 | 2.40 | 89% | 12 | 2.50 | 83% |

As shown by the data in Table 2, the antibodies can be effectively conjugated at high levels of homogeneity, and with a low average DAR. Antibody-drug conjugates with low average DAR have a number of beneficial properties, including reduced clearance rate, higher therapeutic index and reduced toxicity than conjugates with higher average DAR.

Example 5: Analysis of DAR distribution

In Example 4, lowest average DAR for the single-hinge disulfide variants was obtained when using 1 equivalent of the polymeric conjugation reagent per inter-chain disulfide bond, both at 40°C (reactions 1 and 7) and at 22°C (reactions 4 and 10). To compare these results to those obtainable using the parent antibody, parent antibody was conjugated using 1 equivalent of the polymeric conjugation reagent per disulfide bond using the conditions set out in Example 4.

Average DAR for the parent antibody, IgGC226S and IgGC229S are shown in Table 3.

Table 3:

| Conjugation Temp | Parent mAb | IgGC226S | IgGC229S |
|---|---|---|---|
| 40°C | 1.91 | 1.41 | 1.43 |
| 22 °C | 1.89 | 1.33 | 1.26 |

As shown by the data in Table 3, the average DAR for the parent antibody was significantly higher than for the single-hinge disulfide variants IgGC226S and IgGC229S, at either 40°C or at 22°C.

Distribution curves of the antibody-drug conjugate species produced by the conjugation reactions were also analysed to determine DAR distribution. In addition to lower average DAR, it can be seen from Figure 1 (conjugation at 40°C) and Figure 2 (conjugation at 22°C) that the process yields antibody-drug conjugates (IgGC226S and IgGC229S) having reduced heterogeneity and improved yield than those produced using the parent antibody. Antibody-drug conjugates having improved homogeneity require less purification than mixtures of variable stoichiometry, and display reduced toxicity, and/or improved pharmacokinetics and thereby improved efficacy due to the absence of high drug-load species.

Example 6: Comparision of the artificial serum stability of antibody drug conjugates generated from bis-sulfone-PEG(24)-val-cit-PAB-MMAE **1** and from a comparable maleimide based reagent.

Four trastuzumab-MMAE conjugates were prepared. Two conjugates were prepared from bis-sulfone-PEG(24)-val-cit-PAB-MMAE **1** (Example 1) to give a DAR-2 and a DAR44 conjugate after purification, using the trastuzumab antibody (Herceptin®). The remaining two conjugates were prepared from a comparable maleimide based reagent (mal-val-cit-PAB-MMAE, prepared in a similar way to **1** from $H_2N$-val-cit-PAB-MMAE) to give purified DAR-2 and DAR-4 conjugates.

Each of the four conjugates was prepared as 1 mg/mL solutions in phosphate buffered saline, pH 7.4, containing human serum albumin at a concentration of 20 mg/mL. Sodium azide was added (final concentration 1 mM) then the mixtures were split into 4 equal aliquots. One aliquot was immediately frozen at -80 °C. The remaining aliquots were kept at 37 °C. Aliquots at 37 °C were removed after 24, 48 and 120 h and transferred to a -80 °C freezer. After the final

time point, mixtures were analysed by analytical hydrophobic interaction chromatography using a ProPac® 2.1 mm × 100 mm HIC-10 column (Fisher Scientific). The method consisted of a linear gradient from 100% buffer A (50 mM sodium phosphate pH 7.0, 1.5 M ammonium sulfate) to 100 % buffer B (50 mM sodium phosphate pH 7.0, 20% isopropanol) in 50 min. The flow rate was 1 mL/min and the temperature was set at 30 °C. Detection was carried out by following UV absorption at 248 and 280 nm. The average drug antibody ratio for each sample was determined and plotted against incubation time. The results are shown in Figure 3. In Figure 3, no significant decrease in the drug to antibody ratio is observed for the DAR-2 and DAR -4 conjugates prepared with the bis-sulfone reagent 1 signifying that the conjugates are stable to incubation in artificial serum. However, for the malemide based conjugates significant decreases in the DAR distribution over time is observed.

Example 7: Stability in IgG depleted serum

Trastuzumab-MMAE conjugates were prepared from either the bis-sulfone reagent **1** or comparable maleimide reagent (Example 6), both conjugates were purified to give four drug molecules loaded per antibody (DAR 4). The conjugates were mixed with IgG depleted serum (BBI solutions, SF142-2) at a final concentration of 1 mg/mL of conjugate. Sodium azide was added (final concentration 1 mM) then the mixtures were split into aliquots. Two aliquots were immediately frozen at -80 °C. The remaining aliquots were kept at 37 °C and after 120 h, two aliquots were transferred to a -80 °C freezer until analysis. The frozen aliquots were analysed by analytical hydrophobic interaction chromatography using the method described in Example 6 for a ProPac® 2.1 mm × 100 mm HIC-10 column (Fisher Scientific).

The HIC chromatograms for the conjugates incubated in IgG depleted serum are shown in Figure 4. For both bis-sulfone and maleimide conjugates, a series of major peaks are seen eluting between 30 and 55 min. At time zero, the bis-sulfone conjugate is observed as a peak eluting at 52.2 min (peak 1). There is no significant change in this peak after 120 h indicating the conjugate has not degraded during the study. However, for the maleimide DAR 4 conjugate, which is observed as a peak eluting at 49.1 min (peak 1) at time zero, the peak size decreases significantly after 120 h indicating that the conjugate has degraded. New peaks are observed in the 120 h chromatogram for the maleimide conjugate at 44.8 min (peak 2), 40.9 min (peak 3) and 34.0 min (peak 4), which elute with the same retention times as standards of DAR-3, DAR22 and free mAb respectively demonstrating the maleimide conjugate is undergoing loss of drug over time.

Example 8: Conjugation of bis-sulfone-PEG(24)-val-cit-PAB-MMAE **1** to an antibody with high DAR 4 yield

To a solution of trastuzumab (0.768 mL at 5.2 mg/mL in 20 mM sodium phosphate, pH 7.5, 150 mM NaCl, 20 mM EDTA) was added a 5 mM TCEP solution (0.032 mL) and the resulting mixture was incubated at 40 °C for 1 h. The TCEP treated antibody was cooled down to room temperature and diluted to 4.44 mg/mL with 20 mM sodium phosphate, pH 7.5 150 mM NaCl and 20 mM EDTA. A solution of bis-sulfone-dPEG(24u)-val-cit-PAB-MMAE solution (0.100 mL, 4.4 mg/mL) in 50 % (v/v) MeCN and 50 % (v/v) 20 mM sodium phosphate, pH 7.5, 150 mM NaCl and 20 mM EDTA was then added to the antibody solution. The resulting conjugation reaction mixture was mixed and incubated at 22 °C for 22 h. After the 22 h, a 50 mM *N*-acetyl-L-cysteine (0.064 mL, 3.0 mM) was added and the resulting mixture was incubated for a further 1 h at 22 °C. The reaction sample was analysed by analytical HIC using a TOSOH TSK-gel Buthyl-NPR 35 x 4.6 mm column. The area for each DAR variant separated, identified by the ratio of the UV absorbance maxima for drug and antibody and order of peak elution, was plotted as a bar chart and the result is shown in Figure 5. In Figure 5, the major product (>73 %) is the DAR-4 conjugate.

Example 9: In vitro analysis of a trastuzumab MMAE conjugate prepared from bis-sulfone-PEG(24u)-val-cit-PAB-MMAE reagent **1**

A trastuzumab MMAE conjugate was made as per example 8 except for the scale which was increased to 5 mg of trastuzumab and no *N*-acetyl-L-cysteine incubation step was included. The reaction mixture was purified by preparative HIC using a 1 mL column packed with ToyoPearl Phenyl 650S resin connected to an AKTA prime system and equilibrated with Buffer A: 50 mM sodium phosphate, 2.0 M NaCl, pH 7.0 and Buffer B: 80 % 50 mM sodium phosphate, 20 % isopropanol, pH 7.0. The conjugation mixture was mixed with an equal volume of 4.0 M NaCl in 50 mM sodium phosphate, pH 7.0 and injected onto the column at 1 mL/min of buffer A. DAR 4 sample was then eluted using 0-100% Buffer B gradient. The gradient was held when DAR 4 species began to elute and held until the UV trace returned to baseline, at which point the gradient was continued. Fractions of eluted peaks were collected and selected were analysed by analytical HIC using a TOSOH TSK-gel Buthyl-NPR 35 x 4.6 mm column. Fractions containing DAR 4 (at purity > 90%) were combined, buffer exchanged using a HiPrep™ 26/10 Desalting Column into PBS pH 7.4 and concentrated in VivaSpin 20 (10 kDa MWCO PES membrane) concentrators at 4000 xg, room temperature. Concentrated DAR 4 sample was then filtered under sterile conditions and flash frozen in a - 80 °C freezer. The final sample was

quantified by Bradford assay and analysed by nonreducing SDS-PAGE and analytical HIC and used for in-vitro evaluation.

The in vitro efficacy of the mAb-reagent **1** conjugates were determined by measuring the inhibitory effect on cell growth of HER-2 receptor over-expressing cancer cell lines.

Loss of tumour cell viability following treatment with cytotoxic drugs or ADCs in vitro can be measured by growing cell lines in the presence of increasing concentrations of drugs or ADCs and quantifying the loss of proliferation or metabolic activity using CellTiter Glo® Luminescence reagent (Promega Corp. Technical Bulletin TB288; Lewis Phillips G.D, Cancer Res 2008; 68:9280-9290). The protocol describes cell seeding, drug treatment and determination of the cell viability in reference to untreated cells based on ATP synthesis, which is directly related to the number of cells present in the well.

HER2-positive SK-BR-3 and BT-474 cells were trypsinised with 3 mL Trypsin EDTA for 5-15 min. Trypsinisation was stopped by adding 10 mL complete medium, and cells were transferred to a 50 mL Falcon tube. Cells were counted using a Neubauer haemocytometer and adjusted to a cell density of $1x10^5$/mL for BT-474 and $5x10^4$/mL for SK-BR-3 respectively. Cells were seeded (100 μL/well) into poly-D-lysine coated opaque-walled 96-well plates and incubated for 24 h at 37°C and 5% $CO_2$. Tumour cell lines SK-BR-3 (ATCC-HTB-30) and BT-474 (ATCC-HTB-20) were purchased from the American Type Culture Collection. SK-BR-3 cells were grown in McCoy's 5A medium (Life Technologies®), 10% fetal bovine serum, 100 u/mL Penicillin and 100 μg/mL Streptomycin. BT-474 cells were grown in DMEM/F-12 medium (Life Technologies®), 10% fetal bovine serum, 100 u/mL Penicillin and 100 μg/mL Streptomycin.

Methods for cell culture were derived from product information sheets for ATCC and references quoted therein, for example, Culture of Animal Cells: A Manual of Basic Technique by R. Ian Freshney 3rd edition, published by Alan R. Liss, N.Y. 1994, or 5th edition published by Wiley-Liss, N.Y. 2005. Serial dilutions of ADC or free drug (MMAE), were made in triplicate by pipetting across a 96 well plate from columns 3-10 with 2-fold dilutions using the relevant cell culture medium as a diluent. The HER2-positive cell lines, BT-474 and SK-BR-3 were treated with drug concentrations shown in Table 4.

Cells were then incubated with the drug (total volume 200 μL/well), at 37°C and 5% $CO_2$ for a further 96 h.

Table 4

| Cell line | Drug/drug-conj ugate | Concentration range |
|---|---|---|
| SK-BR-3 | MMAE | 2.5-0.02 nM |
| SK-BR-3 | DAR 4 antibody-reagent **1** conjugate | 0.625nM-5pM |
| BT-474 | MMAE | 2.5-0.02 nM |
| BT-474 | DAR 4 antibody-reagent **1** conjugate | 1.25nM-0.01nM |

The cell viability assay was carried out using the Cell-Titer Glo® Luminescence reagent, as described by the manufacturer's instructions, (Promega Corp. Technical Bulletin TB288; Lewis Phillips G.D, Cancer Res 2008; 68:9280-9290). Incubation times, e.g. cell lysis and incubation with luminescent reagent, were extended to 3 min and 20 min respectively, for optimal luminescent signal. Luminescence was recorded using a plate reader (e.g. MD Spectramax M3 plate reader), and data subsequently analysed using a four parameter nonlinear regression model.

The results are shown in Figure 6, which illustrates cell viability responses to treatment with either antibody-reagent **1** conjugate within SKBR-3 or BT-474 cells. Viability is expressed as % of untreated cells. The % viability (Y-axis) is plotted against the logarithm of drug concentration in nM (x-axis) to determine the $IC_{50}$ values for all conjugates as well as free drug. The IC50 values are shown in Table 5.

Table 5

| **Sample** | **SK-BR-3 $IC_{50}$ (nM)** | **BT-474 $IC_{50}$ (nM)** |
|---|---|---|
| MMAE | 0.2 | 0.75 |
| DAR-4 Antibody-reagent **1** conjugate | 0.035 | 0.12 |

As shown in Figure 6 and table 5, the antibody- reagent **1** conjugate is active in HER2-positive cell lines.

Example 10: Synthesis of bis-(valine-citrulline-paraaminobenzyl-monomethyl auristatin E) $(val\text{-}cit\text{-}PAB\text{-}MMAE)_2$ reagent **6** possessing a mono 24 repeat unit PEG with terminal bis-sulfone functionality.

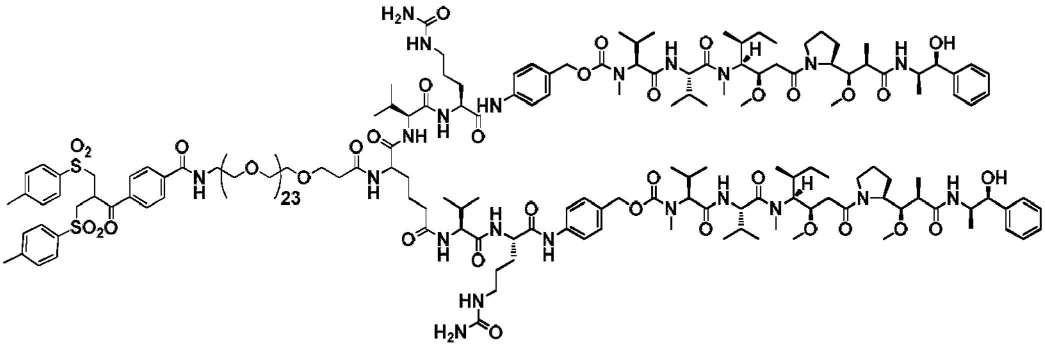

6

Step 1: Conjugation of $H_2N$-val-cit-PAB-MMAE to Fmoc-L-α-amino adipic acid.

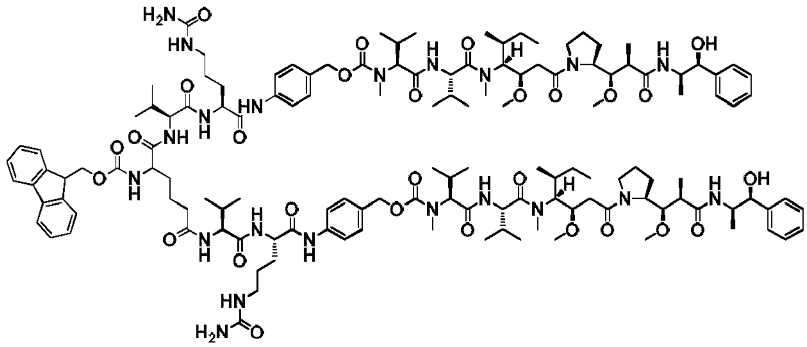

7

To a stirred solution of $H_2N$-val-cit-PAB-MMAE.TFA salt (120 mg, Concortis), Fmoc-L-α-amino adipic acid (16.9 mg) and 1-[bis(dimethylamino)methylene]-1H-1,2,3-triazolo[4,5-b]pyridinium 3-oxid hexafluorophosphate (HATU) (34.2 mg) in anhydrous dimethylformamide (1.5 mL) was added diisopropylethylamine (51 mL) at 0 °C and the reaction mixture was stirred for 2 h and allowed to warm to room temperature over 16 h. The reaction mixture was then purified directly by reverse-phase C18-column chromatography eluting with buffer A (v/v): water:5% acetonitrile:0.1% TFA and buffer B (v/v): 0.1% TFA in acetonitrile (100:0 v/v to 0:100). The organic solvent was removed *in vacuo* and the aqueous solvent was removed by lyophilisation. The Fmoc-L-α-amino adipic acid-bis-[val-cit-PAB-MMAE] 7 was isolated as a white solid (99.0 mg). m/z $[M+H]^+$ 2593.

Step 2: Deprotection of Fmoc-L-α-amino adipic acid-bis-[val-cit-PAB-MMAE] 7

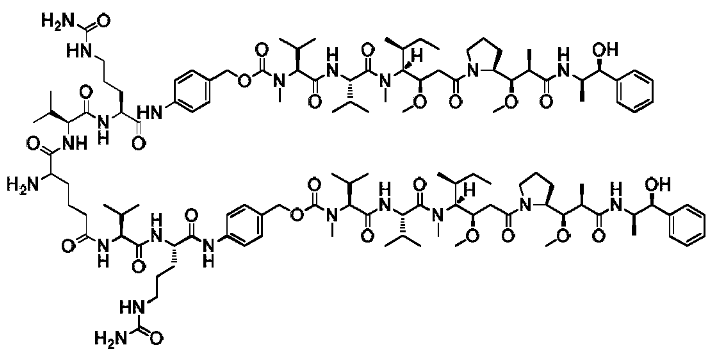

8

Fmoc-L-α-amino adipic acid-bis-[val-cit-PAB-MMAE] 7 (15 mg) was dissolved in anhydrous dimethylformamide (1 mL) and piperidine was added (0.1 mL). After 16 h stirring at room temperature, the reaction mixture was acidified by the addition of acetic acid (0.2 mL) and then purified directly by reverse-phase C18-column chromatography eluting with buffer A (v/v): Water:5% acetonitrile:0.1% TFA and buffer B (v/v): 0.1% TFA in acetonitrile (100:0 v/v to 0:100), the organic solvent was removed *in vacuo* and the aqueous solvent was removed by lyophilisation and the $H_2N$-L-α-amino adipic acid-bis-[val-cit-PAB-MMAE] 8 was isolated as a white solid (7.0 mg). m/z $[M+H]^+$ 2374.

Step 3: Conjugation of L-α-amino adipic acid-bis-[val-cit-PAB-MMAE] 8 to acid terminated PEGylated bis-sulfone 2

To a stirred solution containing the $H_2N$-L-α-amino adipic acid-bis-[val-cit-PAB-MMAE] **5c** (7 mg) and the known 4-[2,2-bis[(p-tolylsulfonyl)-methyl]acetyl]benzoic acid (6 mg) in anhydrous dimethylformamide (0.5 mL) was added 1-[bis(dimethylamino)methylene]-1H-1,2,3-triazolo[4,5-b]pyridinium 3-oxid hexafluorophosphate (HATU) (1.4 mg, 10 μL of a 14 mg in 100 μL DMF stock solution), and the solution was stirred for 5 min at 0 °C. Then N-methylmorpholine (NMM) (0.37 μL, (10 μL of a 37 μL in 963 μL DMF stock solution), was added and the solution stirred. At 1 h time intervals, additional portions of HATU (1.4 mg (10 μL of stock solution)) and NMM (0.37 μl, (10 μL of stock solution)) were added until a total of 3 additions. The material was then purified directly by reverse-phase C18-column chromatography eluting with buffer A (v/v): water:5% acetonitrile:0.1% TFA and buffer B (v/v): 0.1% TFA in acetonitrile (100:0 v/v to 0:100), the organic solvent was removed *in vacuo* and the aqueous solvent was removed by lyophilisation and the bis-sulfone-PEG(24)-$H_2N$-bis-[val-cit-PAB-MMAE] 6 was isolated as a colourless film (3.3 mg, 31%). m/z $[M+H]^{2+}$ 1992.

Example 11: Synthesis of bis-(mono 24 repeat unit PEG-valine-citrulline-paraaminobenzyl-monomethyl auristatin E) $(PEG(24u)\text{-val-cit-PAB-MMAE})_2$ reagent 9 with terminal bis-sulfone functionality.

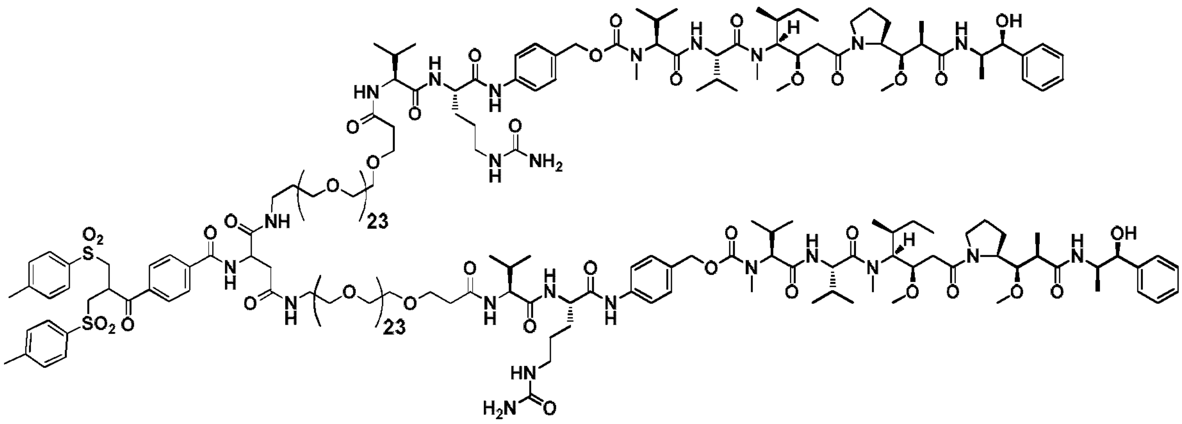

9

Step 1: Conjugation of aspartic acid to acetylbenzoic acid.

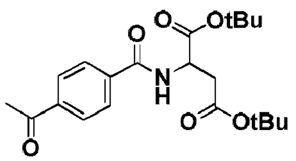

10

To a stirred solution of L-aspartic acid β-t.-butyl α-t.-butyl ester hydrochloride (3.43 g) and anhydrous dichloromethane (30 mL) was added 4-dimethylaminopyridine (DMAP) (1.786 g). After 2 h, 1-ethyl-3-(3-dimethylaminopropyl)carbodiimide (EDCI) (2.80 g) and 4-acetylbenzoic acid (2.00 g) were added and the reaction was stirred at room

temperature. After 28 h, the solution was partitioned with water (100 mL) and the aqueous phase was extracted with dichloromethane (2 × 10 mL). The combined organic phase was washed with water (2 × 50 mL), brine (100 mL), dried over magnesium sulphate, filtered and concentrated *in vacuo.* The solid residue was purified by column chromatography eluting with dichloromethane-methanol (99.5:0.5 v/v), the solvent was removed *in vacuo* and the di-tert-butyl 2-[(4-acetylbenzoyl)amino]butanedioate **10** was isolated as an off-white solid (3.856 g, 81%). $^1$H NMR $\partial_H$ (400 MHz $CDCl_3$) 1.45 (9H, s, t-Bu), 1.48 (9H, s, t-Bu), 2.52 (3H, s, C$H_3$-COAr), 2.80 (2H, dd, $CH_2CO_2$tBu), 4.80 (1H, m, *CH*-$CH_2$), 7.25 (1H, m, N*H*), 7.85 (2H, d, Ar), 8.00 (2H, d, Ar).

Step 2: Formation of bis-tolylsulfanyl butanedioate **11**

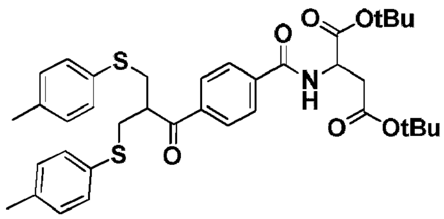

**11**

To a stirred suspension of di-tert-butyl 2-[(4-acetylbenzoyl)amino]butanedioate **10** (1.00 g) in absolute ethanol (20 mL) was added 4-methylbenzenethiol (635 mg). To the reaction mixture was added formaldehyde (37% aq. solution) (0.3 mL) Nature Protocols, 2006, 1(54), 2241-2252) then heated at reflux. After 2 h and 8 h a further portion of formaldehyde (37% aq. solution, 0.3 mL) was added. After a further 16 h, the reaction mixture was cooled to room temperature, concentrated *in vacuo,* diluted in dichloromethane (50 mL), washed with water (3 x 25 mL), dried over magnesium sulphate, filtered and concentrated *in vacuo.* The crude material was purified by column chromatography eluting with dichloromethane-methanol (99.5:0.5 v/v to 98:2 v/v), the solvent was removed *in vacuo* and the di-tert-butyl 2-[[4-[3-(p-tolylsulfanyl)-2-(p-tolylsulfanylmethyl)propanoyl]benzoyl]amino]butanedioate **11** was isolated as an pale yellow solid (0.594 g, 35%). $^1$H NMR $\partial_H$ (400 MHz $CDCl_3$) 1.45 (9H, s, t-Bu), 1.48 (9H, s, t-Bu), 2.35 (6H, s, C$H_3$-Ar), 2.85 (2H, dd, $CH_2CO_2$tBu), 3.15 (2H, dd, *$CH_2$*-SAr), 3.75 (1H, m, C*H*$CH_2$S), 4.80 (1H, m, *CH*-$CH_2$), 7.10 (4H, d, SAr), 7.15 (4H, d, SAr), 7.25 (1H, m, N*H*), 7.55 (2H, d, Ar), 75 (2H, d, Ar).

Step 3: Removal of the tert-butyl protection groups.

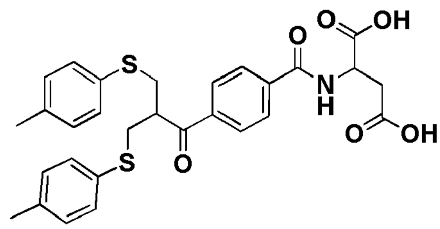

**12**

To a stirred solution of di-tert-butyl 2-[[4-[3-(p-tolylsulfanyl)-2-(p-tolylsulfanylmethyl)propanoyl]benzoyl]amino]butanedioate **11** (527 mg) in anhydrous dichloromethane (2.5 mL) was carefully added trifluoroacetic acid (2.5 mL) at room temperature under an inert atmosphere. After 16.5 h, the reaction was concentrated *in vacuo* and residual trifluoroacetic acid was removed by azeotrope distillation with toluene (2 x 3 mL). The crude product was concentrated *in vacuo* to give 2-(4-(3-(p-tolylthio)-2-((p-tolylthio)methyl)propanoyl)benzamido)succinic acid **12** as a pale yellow oil (490 mg, assumed quantitative yield) which was used without further purification. $^1$H NMR $\partial_H$ (400 MHz $CDCl_3$) 2.25 (6H, s, C$H_3$-Ar), 2.80-3.15 (6H, m, *$CH_2$*$CO_2$H and *$CH_2$*-SAr), 3.65 (1H, m, C*H*$CH_2$S), 5.00 (1H, m, *CH*-$CH_2$), 6.85 (4H, d, SAr), 6.95 (4H, d, SAr), 7.50 (2H, d, Ar), 7.55 (1H, br s, N*H*), 7.65 (2H, d, Ar), 10.95 (2H, br s, $CO_2$*H*).

Step 4: Conjugation of bis-tolylthio-Asp-acid to $H_2N$.dPEG(24)-CO-OtBu.

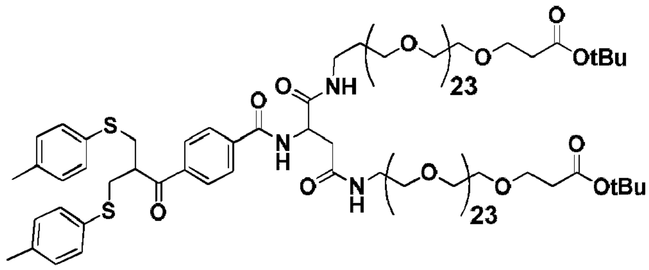

**13**

A toluene (3 mL) solution of $H_2N$-dPEG(24)-CO-OtBu (240 mg, Iris Biotech) was evaporated to dryness and the residue re-dissolved in dichloromethane (15 mL). Under stirring, 1-ethyl-3-(3-dimethylaminopropyl)carbodiimide (EDCI) (17.4 mg) and 2-(4-(3-(p-tolylthio)-2-((p-tolylthio)methyl)propanoyl)benzamido)succinic acid **12** (50 mg) were added and the reaction mixture was stirred under an inert atmosphere at room temperature. After 16 h, a further amount of $H_2N$.dPEG(24)-CO-OtBu (240 mg) and EDCI (17.4 mg) were added. After a total of 96 h, the solid residue was directly purified by column chromatography eluting with dichloromethane-methanol (99:1 v/v to 90:10 v/v), the solvent was removed *in vacuo* and the bis-tolyl-bis PEG **13** was isolated as a waxy yellow solid (179.5 mg, 68%). $^1H$ NMR $\partial_H$ (400 MHz $CDCl_3$) 1.45 (18H, s, t-Bu), 2.30 (6H, s, $CH_3$-Ar), 2.75 (2H, dd, $CH_2$CON), 3.20 (2H, dd, $CH_2$- SAr), 3.45-3.80 (m, PEG and C*H*$CH_2$S), 4.85 (1H, m, *CH*-$CH_2$), 7.05 (4H, d, SAr), 7.10 (4H, d, SAr), 7.55 (2H, d, Ar), 7.85 (2H, d, Ar), 7.90 (1H, m, NH), 8.50 (1H, m, N*H*).

Step 5: Removal of the tert-butyl protection groups.

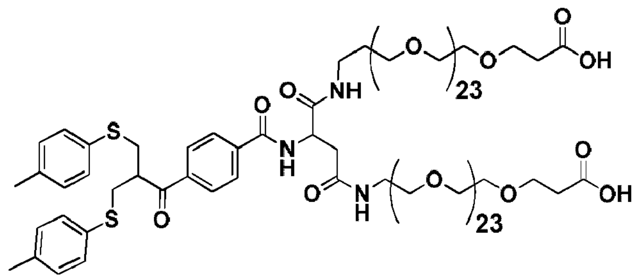

**14**

To a stirred solution of bis-toluyl-bis PEG-ester **13** (179.5 mg) in anhydrous dichloromethane (3.0 mL) was carefully added trifluoroacetic acid (3.0 mL) at room temperature under an inert atmosphere. After 2 h 45 min the reaction was concentrated *in vacuo* and residual trifluoroacetic acid was removed by azeotrope distillation with toluene (2 x 7 mL). The crude product was concentrated *in vacuo* and then purified by column chromatography eluting with dichloromethane-methanol (98:2 v/v to 85:15 v/v), the solvent was removed *in vacuo* and the bis-toluyl-bis PEG-acid **14** was isolated as a pale yellow oil (102 mg, 59%). $^1H$ NMR $\partial_H$ (400 MHz $CDCl_3$) 2.05 (6H, s, $CH_3$-Ar), 2.60 (2H, t, $CH_2CO_2H$), 2.80 (2H, dd, $CH_2$CON), 3.20 (2H, dd, $CH_2$-SAr), 3.45-3.80 (m, PEG and C*H*$CH_2$S), 4.95 (1H, m, *CH*-$CH_2$), 7.05 (4H, d, SAr), 7.10 (4H, d, SAr), 7.55 (2H, d, Ar), 7.70 (1H, m, N*H*), 7.75 (1H, m, N*H*), 7.85 (2H, d, Ar), 8.75 (1H, m, N*H*).

Step 6: Oxidation of the bis-tolylsulfanyl-bis-PEG-acid.

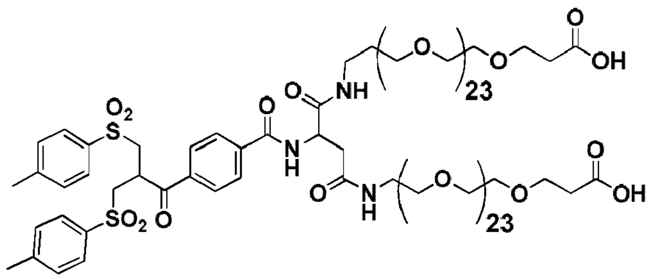

**15**

Water (3 mL) was added to a stirred solution of bis-toluyl-bis PEG-acid **14** (102 mg) in methanol (3 mL) followed by the addition of Oxone® (67.1 mg) portion-wise. The resulting suspension was allowed to stir at room temperature and after 18 h the reaction mixture was passed through a plug of cotton wool and washed with MeOH/Water 1:1 (2 mL). The combined organic washings were concentrated *in vacuo* to remove methanol and the aqueous layer extracted with dichloromethane (3 x 7 mL). The combined organic layers were washed with aqueous HCl (1x 5 mL, pH 3), dried over magnesium sulphate, filtered and concentrated *in vacuo* to yield bis-sulfone-bis-PEG-acid **15** as a pale yellow solid (79 mg, 76%). $^1$H NMR $\partial_H$ (400 MHz $CDCl_3$) 2.45 (6H, s, $CH_3$-Ar), 2.55 (2H, t, $CH_2CO_2H$), 2.80 (2H, dd, $CH_2$CON), 3.25-3.80 (m, PEG and $CH_2$-Ts), 4.25 (1H, m, *CH*-$CH_2$-Ts), 4.85 (1H, m, *CH*-$CH_2$), 7.35 (4H, d, Ts), 7.60 (1H, m, N*H*), 7.65 (2H, d, Ar), 7.70 (4H, d, Ts), 7.85 (2H, d, Ar), 7.95 (1H, m, N*H*), 8.75 (1H, m, N*H*).

Step 7: Conjugation of $H_2$N-val-cit-PAB-MMAE to bis-acid terminated PEGylated bis-sulfone.

To a stirred solution of bis-sulfone-bis-PEG-acid **15** (24.0 mg) under an inert atmosphere was added $H_2$N-val-cit-PAB-MMAE.TFA salt (37.2 mg) and anhydrous sodium carbonate (2.7 mg) in anhydrous dimethylformamide (0.8 mL). One-[bis(dimethylamino)methylene]-1H-1,2,3-triazolo[4,5-b]pyridinium 3-oxid hexafluorophosphate (HATU) (9.6 mg) was then added and the reaction mixture was stirred at room temperature. After 42 h, the reaction mixture was acidified by acetic acid (50 μL) and then purified directly by reverse-phase C18-column chromatography eluting with buffer A (v/v): Water:5% acetonitrile:0.1% TFA and buffer B (v/v): 0.1% TFA in acetonitrile (100:0 v/v to 0:100). For the product fractions, the organic solvent was removed *in vacuo* and the aqueous solvent was removed by lyophilisation. Further purification was achieved using reverse-phase silica-diol-column chromatography eluting with ethyl acetate-isopropanol (100:0 v/v to 0:100 v/v). The solvent was removed *in vacuo* and the bis-sulfone-PEG(24)-bis-[val-cit-PAB-MMAE] **9** was isolated as a colourless oil (8.0 mg, 21%). m/z M+Na 5104.

Example 12: Conjugation of diloaded bis-sulfone MMAE reagent **8** and diloaded bis-sulfone MMAE reagent **9** to an antibody fragment (Fab)

Two 5 mL aliquots of a Fab (2.14 mg/mL in PBS), derived from the papain digestion of trastuzumab, were reduced with DTT (50 μL of 1 M DTT solution added) at 22 °C for 1 h. To remove the excess DTT, the reduced Fab solutions were then buffer exchanged into 20 mM sodium phosphate, pH 7.4 (150 mM NaCl and 20 mM EDTA) using two PD-10 columns. After buffer exchange, the sample concentration was measured by UV. The buffer exchanged Fab solutions were diluted to 1.1 mg/mL (3.6 mL final volume) with 20 mM sodium phosphate, pH 7.4 (150 mM NaCl and 20 mM EDTA).

To one aliquot of reduced Fab was added a solution of bis-sulfone-PEG(24u)-val-cit-PAB-MMAE2 (1.2 mg/mL, 0.4 mL) in 50:50 v/v acetonitrile and 20 mM sodium phosphate, pH 7.4, 150 mM NaCl and 20 mM EDTA. To the second Fab aliquot was added a solution of bis-sulfone-[PEG(24u)-val-cit-PAB-MMAE]2 (0.4 mL, 1.5 mg/mL) in 50:50 v/v acetonitrile and 20 mM sodium phosphate, pH 7.4, 150 mM NaCl and 20 mM EDTA. Both reaction mixtures were incubated at 22 °C for 22 h. Upon the completion of the incubation, the reaction mixtures (4.0 mL) were buffer exchanged into PBS using a 10 mL Zeba™ spin column. Both the resulting Fab-MMAE conjugates were purified to greater than 93% purity by preparative HIC using a Toyopearl Phenyl HIC column (1 mL). Each sample was loaded in 2 M NaCl, 50 mM sodium phosphate, pH 7.0 and eluted in a 0-100% gradient of 20% propan-2-ol, 50 mM sodium phosphate, pH 7.0.

Example 13: Analysis of antibody drug conjugates (ADCs) by in vitro cell viability assay

The *in vitro* efficacy of the reagent **6** and reagent **9** antibody conjugates prepared in Example 12 were determined by measuring the inhibitory effect on cell growth of HER-2 receptor over-expressing cancer cell lines by the method of Example 9.

HER2-positive SK-BR-3 and BT-474 cells were trypsinised with 3 mL Trypsin EDTA for 5-15 min. Trypsinisation was stopped by adding 10 mL complete medium, and cells were transferred to a 50 mL Falcon tube. Cells were counted using a Neubauer haemocytometer and adjusted to a cell density of $1x10^5$/mL for BT-474 and $5x10^4$/mL for SK-BR-3 respectively. Cells were seeded (100 $\mu$L/well) into poly-D-lysine coated opaque-walled 96-well plates and incubated for 24 h at 37°C and 5% $CO_2$. Tumour cell lines SK-BR-3 (ATCC-HTB-30) and BT-474 (ATCC-HTB-20) were purchased from the American Type Culture Collection. SK-BR-3 cells were grown in McCoy's 5A medium (Life Technologies®), 10% fetal bovine serum, 100 u/mL Penicillin and 100 $\mu$g/mL Streptomycin. BT-474 cells were grown in DMEM/F-12 medium (Life Technologies®), 10% fetal bovine serum, 100 u/mL Penicillin and 100 $\mu$g/mL Streptomycin.

The HER2-positive cell lines, BT-474 and SK-BR-3 were treated with drug concentrations shown in Table 6. Cells were then incubated with the drug (total volume 200 $\mu$L/well), at 37 °C and 5% $CO_2$ for a further 96 h.

Table 6

| Cell line | Drug/Antibody drug conjugate | Concentration range (nM) |
|---|---|---|
| SK-BR-3 | MMAE | 2.5-0.02 |
| | Reagent **6** conjugate | 1.25-0.01 |
| | Reagent **9** conjugate | 2.5-0.02 |
| | | |
| BT-474 | MMAE | 5-0.04 |
| | Reagent **6** conjugate | 2.5-0.02 |
| | Reagent **9** conjugate | 5-0.04 |

The cell viability assay was carried out using the Cell-Titer Glo® Luminescence reagent, as described by the manufacturer's instructions, (Promega Corp. Technical Bulletin TB288; Lewis Phillips G.D, Cancer Res 2008; 68:9280-9290). Incubation times, e.g. cell lysis and incubation with luminescent reagent, were extended to 3 min and 20 min respectively, for optimal luminescent signal. Luminescence was recorded using a plate reader (e.g. MD Spectramax M3 plate reader), and data subsequently analysed using a four parameter nonlinear regression model.

The results are shown in Figure 7, which illustrates cell viability responses to treatment with either reagent **6** conjugate or reagent **9** conjugate within SKBR-3 or BT-474 cells. Viability is expressed as % of untreated cells. The % viability (Y-axis) is plotted against the logarithm of drug concentration in nM (x-axis) to determine the IC50 values for all conjugates as well as free drug. The $IC_{50}$ values are shown in Table 7.

Table 7

| Sample | SK-BR-3 $IC_{50}$ (nM) | BT-474 $IC_{50}$ (nM) |
|---|---|---|
| Reagent **6** conjugate | 0.14 | 0.32 |
| Reagent **9** conjugate | 0.2 | 0.42 |
| MMAE | 0.27 | 0.85 |

As shown in Figure 7 and Table 7, both antibody conjugates are active in HER2-positive cell lines. The potency of the reagent **6** conjugate is increased in comparison with the reagent **9** conjugate. Both conjugates reduce proliferation more efficiently than the free drug.

Example 14: In-vivo xenograft study for antibody drug conjugates produced using bis-sulfone-PEG(24)-val-cit-PAB-MMAE reagent **1**

Four antibody drug conjugates (ADCs) with DAR =1, DAR = 2, DAR = 3, and DAR = 4, were produced from the conjugation of bis-sulfone-PEG(24)-val-cit-PAB-MMAE reagent **1** with trastuzumab and purified by HIC to each have greater than 95% single DAR purity. Each conjugate was then used in a xenograft study as follows.

Female severe combined immunodeficient mice (Fox Chase SCID®, C.B-17/Icr-*Prkdcscid*, Charles River Laboratories) were eleven weeks old, with a body weight (BW) range of 18.0 to 23.1 grams on Day 1 of the study. The animals were fed *ad libitum* water (reverse osmosis, 1 ppm C1), and NIH 31 Modified and Irradiated Lab Diet® consisting of 18.0% crude protein, 5.0% crude fat, and 5.0% crude fibre. The mice were housed on irradiated Enrich-o'cobs™ Laboratory Animal Bedding in static micro-isolators on a 12-hour light cycle at 20-22°C (68-72°F) and 40-60% humidity.

Xenografts were initiated with BT474 human breast carcinomas maintained by serial subcutaneous transplantation in SCID mice. On the day of tumour implant, each test mouse received a 1-$mm^3$ BT474 fragment implanted subcutaneously in the right flank, and tumour growth was monitored as the average size approached the target range of 90 to 120 $mm^3$. Tumours were measured in two dimensions using calipers, and volume was calculated using the formula:

$$Tumor\ Volume\ (mm^3) = \frac{w^2 \times l}{2}$$

where w = width and *l* = length, in mm, of the tumour.

Twenty-eight days after tumour implantation, designated as Day 1 of the study, the animals were sorted into groups each consisting of ten mice with individual tumour volumes ranging from 63 to 126 $mm^3$ and group mean tumour volumes from 99 to 101 $mm^3$.

Treatment began on Day 1 in all groups of mice (n = 10) with established subcutaneous BT474 tumours (63-126 $mm^3$). Group 1 was a vehicle-treated control group. Groups 2-5 received ADCs having DAR = 1, DAR = 2, DAR = 3 and DAR = 4, respectively, each at 20 mg/kg intravenously on Days 1, 8 and 15. Tumours were measured twice per week until the study was terminated on Day 61. Mice were monitored individually, and each animal was euthanized when its tumour reached the endpoint volume of 1500 $mm^3$ or on the final day, whichever came first. The time to endpoint (TTE) was calculated for each mouse. Treatment outcome was determined from percent tumour growth delay (%TGD), defined as the percent increase in median TTE for treated versus control mice, with differences between groups deemed statistically significant at $P \leq 0.05$ using logrank survival analysis. Mice were also monitored for complete regression (CR) and partial regression (PR) responses. An animal with a CR at study end was additionally classified as a tumour free survivor. Treatment tolerability was assessed by body weight measurements and frequent observation for clinical signs of treatment-related side effects.

The median TTE for vehicle-treated controls was 34.9 days, establishing a maximum possible TGD of 26.1 days (75%) for the 61-day study. All regimens were well tolerated and could be evaluated for efficacy. ADCs having DAR = 1 and DAR = 2 produced measurable TGDs of 14.4 days (41%) and 16.9 days (48%), respectively, but no significant survival difference from controls ($P > 0.05$). The DAR = 1 group had one tumour free survivor, which was the only regression recorded among the three groups. ADCs having DAR = 3 and DAR = 4 each produced the maximum TGD (26.1 days, 75%), and a significant survival difference versus controls ($P < 0.001$), but the two regimens were distinct based on regression responses. DAR = 3 treatment produced three PRs, whereas the DAR = 4 produced 10/10 tumour free survivors. The results are shown in Figure 8.

Example 15: Conjugation of Bis-sulfone-PEG(24)-val-cit-PAB-MMAE 1 to parent antibody and antibody variant IgGC226S: Higher retention of interchain bridging with IgGC226S.

Conjugation of the parent antibody and the single-hinge disulfide variant IgGC226S described in Example 4 with 1 molar equivalent of the conjugation reagent Bis-sulfone-PEG(24)-val-cit-PAB-MMAE 1 per inter-chain disulfide bond was performed after antibody reduction (TCEP, 1 molar equivalent per interchain disulfide, 15 min, 40 °C). The conjugation reagent was prepared in DMSO (to give 5% (v/v) DMSO in reaction solution) immediately before conjugation. Antibody concentrations during conjugation were 4 mg/mL. Reactions were conducted overnight (16 h) at 40 °C, after which time the reaction mixtures were treated with 10 mM DHA for 1 h at room temperature and then analysed by SDS-PAGE. The SDS-PAGE gels were stained with InstantBlue™ and imaged using an IMAGEQUANT™ LAS 4010 instrument (GE Healthcare) to determine the % of each species present within a lane. The SDS-PAGE results are shown in Figure 9. In Figure 9, the lanes labelled M show Novex Protein Standards (Invitrogen). Lanes 1 and 2 show the migration profiles of IgGC226S pre- and post- conjugation reaction respectively. Lanes 3 and 4 show the equivalent reactions for the parent antibody. When the heavy to heavy interchain disulfides of an antibody are not covalently bridged following conjugation, for example, due to disulfide bond scrambling, a band just below the 80 kDa marker of heavy-light chain dimer (H + L) is visible by SDS-PAGE. In contrast, when the heavy to heavy interchain disulfides are bridged following conjugation, a band just above the 160 kDa marker of antibody heavy-light chain tetramer (2H + 2L) is visible. Comparing lanes 2 and 4, it can be seen that conjugating to IgGC226S, possessing a single inter heavy-chain disulfide, leads to a higher extent of bridging between the two heavy chains compared to the parent antibody, with two inter heavy-chain disulfides (80% vs 67% of antibody heavy-light chain tetramer respectively) and a lower extent of heavy-light chain dimer formation (17% v 31% respectively). The process thus improves the stability of the antibody conjugate by efficient bridging of the inter-heavy chain disulfide bond.

Sequence Listing:

<160> Number of SEQ ID NOS: 2

<170> PatentIn version 3.5

<210> SEQ ID NO: 1
<211> 214
<212> PRT
<213> Artificial Sequence

<220>
<223> sequence is synthesised

<400> 1

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> SEQ ID NO: 2
<211> 450
<212> PRT
<213> Artificial Sequence

<220>
<223> sequence is synthesised

<400> 2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

SEQUENCE LISTING

<110> Polytherics Limited

<120> Novel Drug-Protein Conjugates

<130> P020853 WO

<150> US 61/717,710
<151> 2012-10-24

<150> US 61/717,743
<151> 2012-10-24

<150> US 61/811,285
<151> 2013-04-12

<150> PCT/GB 2013/051593
<151> 2013-06-19

<160> 2

<170> PatentIn version 3.5

<210> 1
<211> 214
<212> PRT
<213> Artificial Sequence

<220>
<223> sequence is synthesised

<400> 1

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
```

```
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  2
<211>  450
<212>  PRT
<213>  Artificial Sequence

<220>
<223>  sequence is synthesised

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110
```

```
Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
```

355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

## Claims

**1.** A conjugate of the general formula:

$$(((D'_{q'}\text{-}Lk^{1'})_m\text{-}P)_p\text{-}Lk^2\text{-}Lk^3)_n\text{-}Ab \qquad (I')$$

in which D' represents a drug moiety;
q' represents an integer from 2 to 10;
$Lk^{1'}$ represents a linker;
m represents an integer from 1 to 10;
P represents a z-valent group $-P^1$-NH- where z is from 2 to 11 and $P^1$ is a polyethylene glycol group;
p represents an integer from 1 to 10;
$Lk^2$ represents a bond or a y-valent linker where y is from 2 to 11 and which consists of from 1 to 9 aspartate and/or glutamate residues;
$Lk^3$ represents a linker of the general formula:

$$\text{-CO-Ph-X-Y-} \qquad (II)$$

in which Ph is an optionally substituted phenyl group; X represents a CO group or a CH.OH group; and Y represents a group of formula:

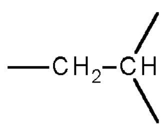 (III) or 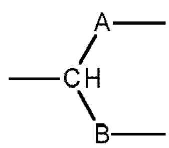 (IV)

in which each of A and B represents a $C_{1-4}$alkylene or alkenylene group;
Ab represents a binding protein or peptide capable of binding to a binding partner on a target, said binding protein being bonded to $Lk^3$ via two sulfur atoms derived from a disulfide bond in the binding

protein or peptide; and
n represents an integer from 1 to s where s is the number of disulfide bonds present in the binding protein or peptide prior to conjugation to $Lk^3$;
the meanings of m, n, p, q', y and z being chosen such that the conjugate contains from 1 to 10 D' groups.

**2.** A conjugate as claimed in claim 1, wherein D' is a cytotoxic drug.

**3.** A conjugate as claimed in claim 1, wherein D' is an auristatin or a maytansine.

**4.** A conjugate as claimed in claim 3, wherein D' is monomethylauristatin E or monomethylauristatin F bonded via its terminal nitrogen atom.

**5.** A conjugate as claimed in claim 4, wherein the auristatin is monomethylauristatin E bonded via its terminal nitrogen atom.

**6.** A conjugate as claimed in any one of the preceding claims, in which $Lk^{1'}$ is a degradable linker.

**7.** A conjugate as claimed in claim 6, in which $Lk^{1'}$ includes one of the following groups:

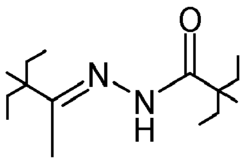
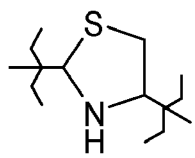
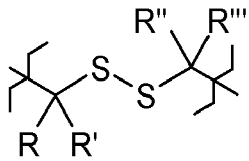
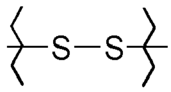

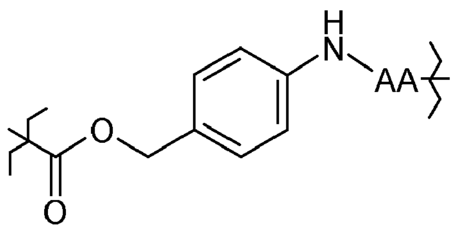

in which each of R, R', R" and R"' represents a hydrogen atom or an alkyl group and AA represents a protease-specific amino acid sequence.

**8.** A conjugate as claimed in claim 7, in which $Lk^{1'}$ includes:

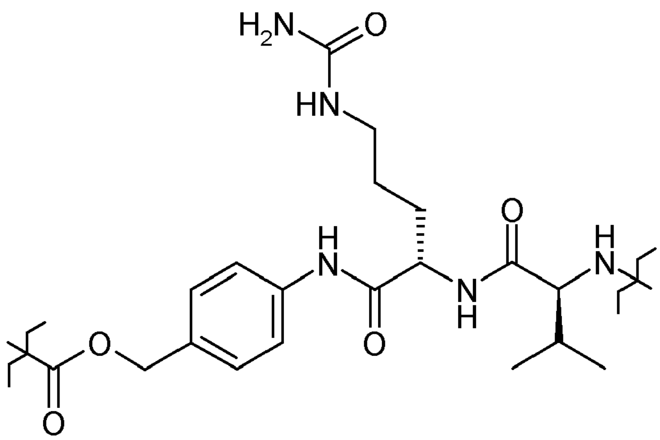

(V)

**9.** A conjugate as claimed in any one of the preceding claims, in which $Lk^{1'}$ includes an element of formula:

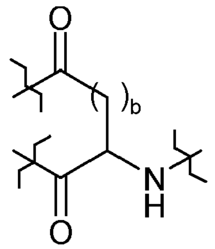

(VI)

in which b is 1, 2 or 3.

**10.** A conjugate as claimed in any one of claims 1 to 5, which has the formula:

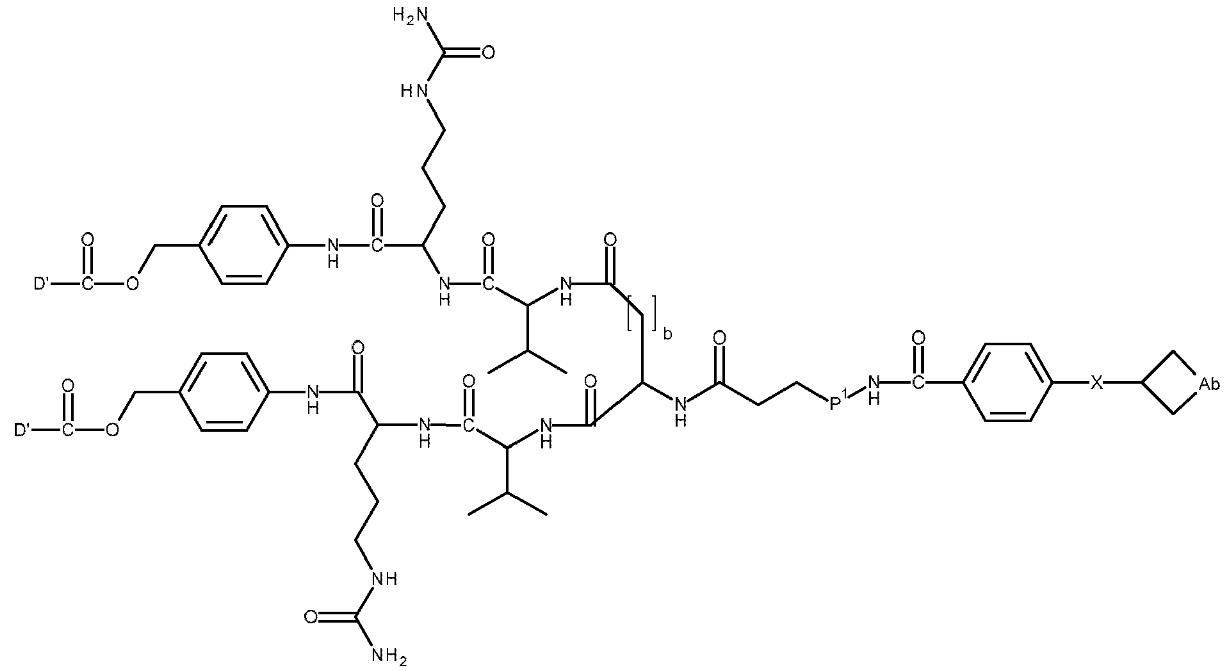

in which b is 1, 2 or 3; and equivalent structures in which Ab carries 2, 3 or 4 copies of the ~X- group.

**11.** A conjugate as claimed in any one of the preceding claims, in which $P^1$ contains from 2 to 10 ethylene glycol units.

**12.** A conjugate as claimed in any one of the preceding claims, in which the phenyl group Ph in $Lk^3$ is unsubstituted.

**13.** A conjugate as claimed in any one of the preceding claims, in which Y has the formula:

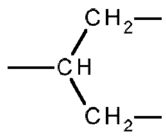

**14.** A conjugate as claimed in any one of the preceding claims, in which Ab represents a full length antibody or an antibody fragment comprising an antigen-binding region of the full length antibody.

**15.** A conjugate as claimed in any one of the preceding claims, in which Ab represents IgG1 or IgG4 or a fragment of IgG1 or IgG4.

**16.** A process for the preparation of a conjugate as claimed in any one of the preceding claims, which comprises reducing

one or more disulfide bonds in a binding protein and subsequently reacting with a conjugating reagent of the general formula:

$$((D'_{q'}\text{-}Lk^{1'})_m\text{-}P)_p\text{-}Lk^2\text{-}Lk^{3a} \qquad (VIII')$$

in which D', $Lk^{1'}$, P, $Lk^2$ and m, p and q' have the meanings given in claim 1, and $Lk^{3a}$ represents a group of formula:

$$\text{-CO-Ph-}X^a\text{-}Y^a \qquad (IX)$$

in which Ph has the meaning given in claim 1, $X^a$ represents a CO group, and $Y^a$ represents a group:

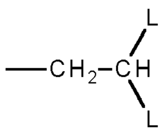
(X),
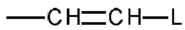
(XI),
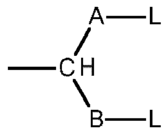
(XII)

or

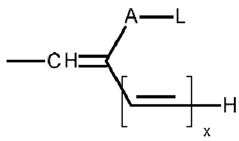
(XIII)

in which A and B have the meanings given in claim 1, each L independently represents a leaving group, and x represents an integer from 1 to 4, to produce a conjugate of formula I' in which X represents CO; and optionally reducing said initially-formed CO group X to give a conjugate having a CH.OH group X.

**17.** A process as claimed in claim 16, in which $Y^a$ represents:

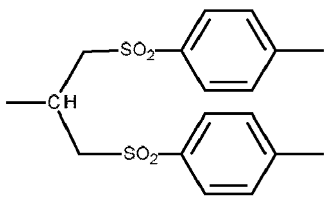
(XIIa)

**18.** A compound of the general formula:

$$((D'_{q'}\text{-}Lk^{1'})_m\text{-}P)_p\text{-}Lk^2\text{-}Lk^{3a} \qquad (VIII')$$

in which D' has the meaning given in any one of claims 1 to 5; $Lk^{1'}$ has the meaning given in any one of claims 1 and 6 to 9; P, $Lk^2$, m, p and q have the meanings given in claim 1, and $Lk^{3a}$ represents a group of formula:

$$\text{-CO-Ph-}X^a\text{-}Y^a \qquad (IX)$$

in which Ph has the meaning given in either claim 1 or claim 11, $X^a$ represents a CO group, and $Y^a$ represents a group:

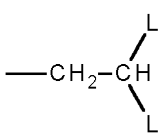
(X),
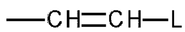
(XI),
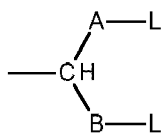
(XII)

or

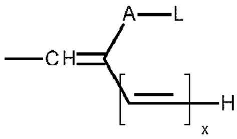

(XIII)

in which A and B have the meanings given in claim 1, each L independently represents a leaving group, and x represents an integer from 1 to 4.

**19.** A compound as claimed in claim 18, in which $Y^a$ represents:

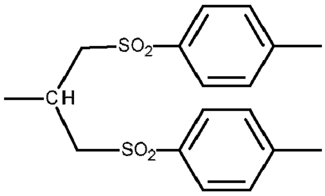

(XIIa)

**20.** A pharmaceutical composition comprising a conjugate as defined in any one of claims 1 to 14, together with a pharmaceutically acceptable carrier, optionally together with an additional therapeutic agent.

**21.** A conjugate as claimed in any one of claims 1 to 14 or a composition as claimed in claim 20 for use in therapy.

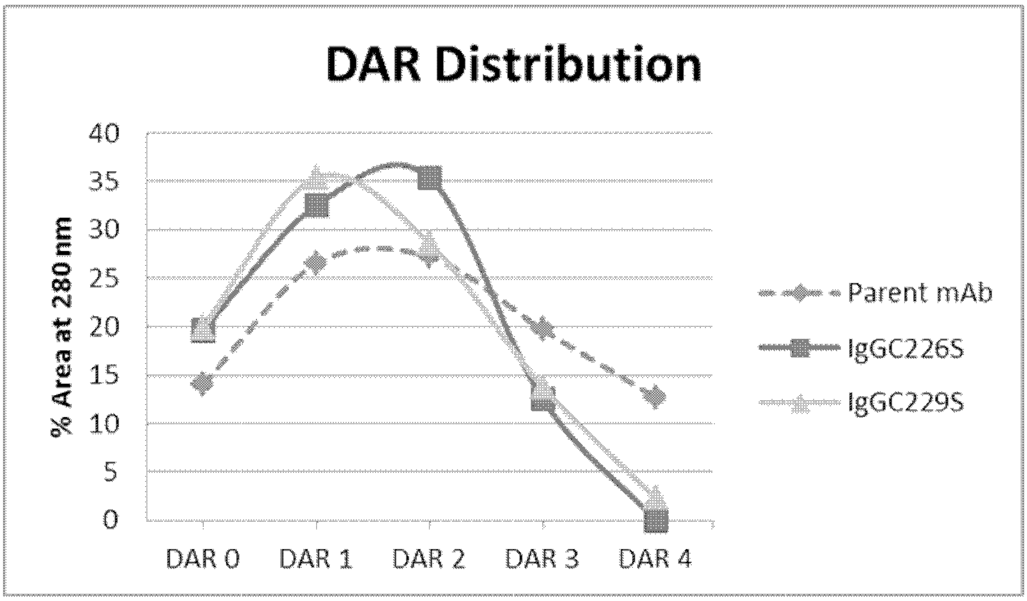


Figure 1

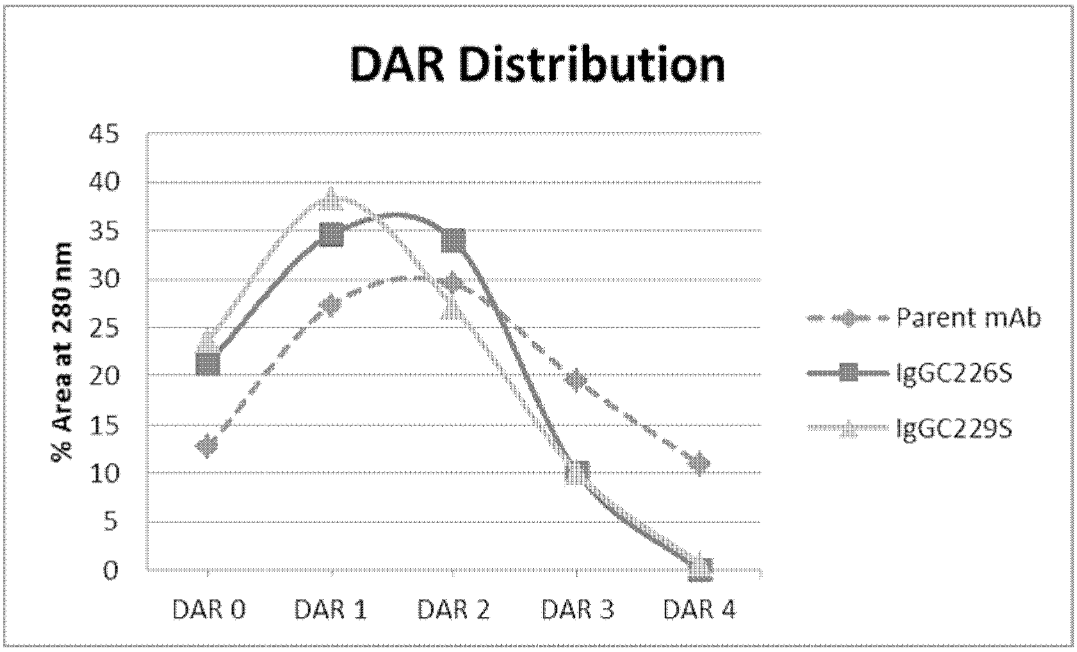


Figure 2

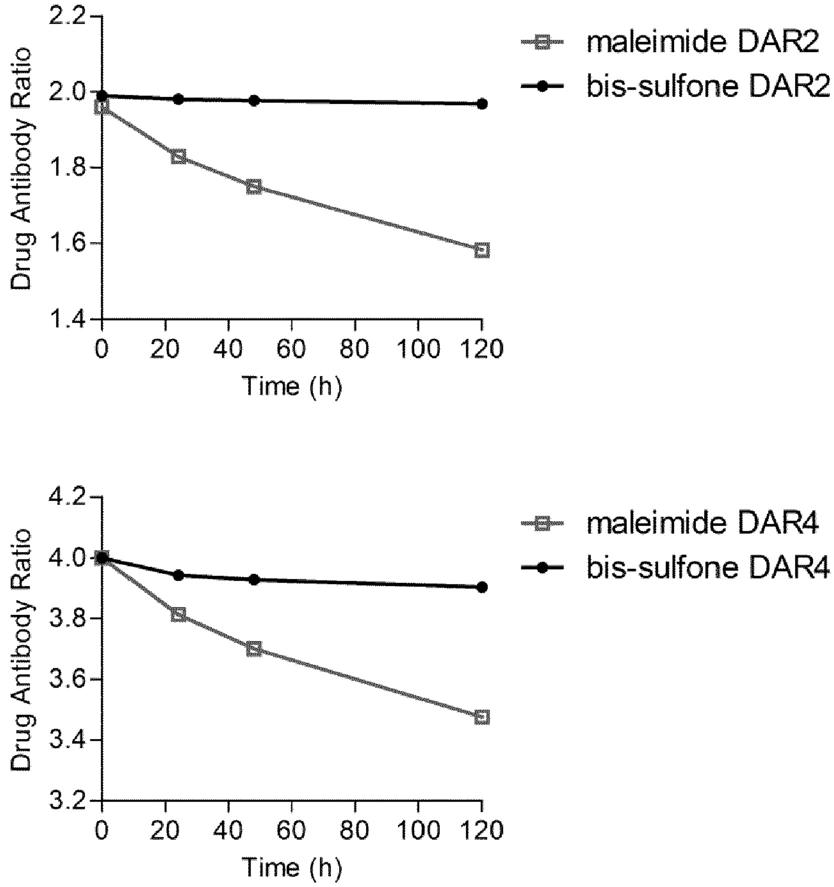


Figure 3

**Bis-sulfone reagent 1 conjugate** **Maleimide conjugate**

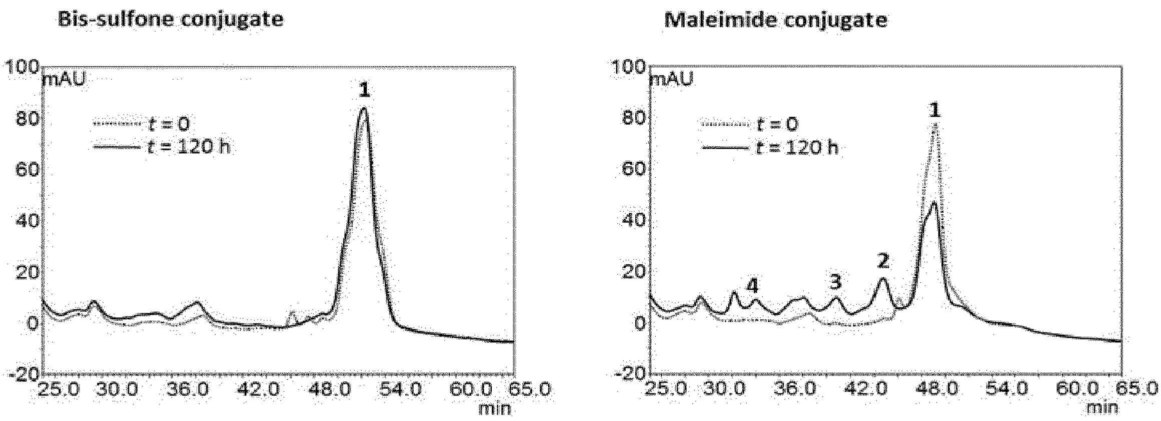


Figure 4

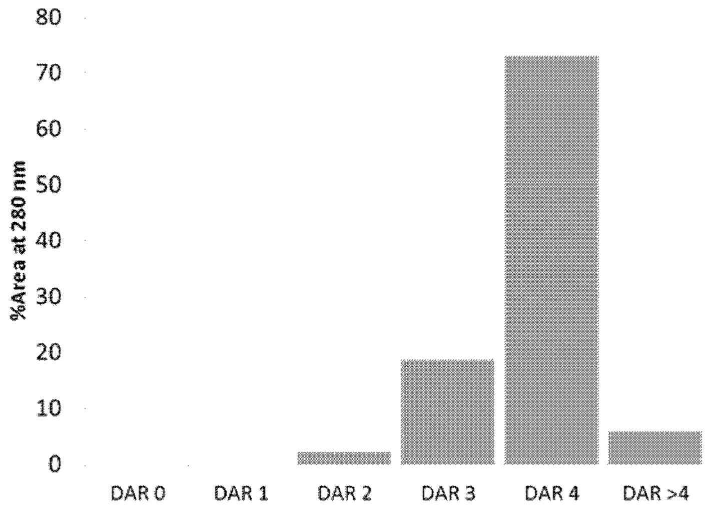
80
70
60
50
40
30
20
10
0
%Area at 280 nm
DAR 0
DAR 1
DAR 2
DAR 3
DAR 4
DAR >4

Figure 5

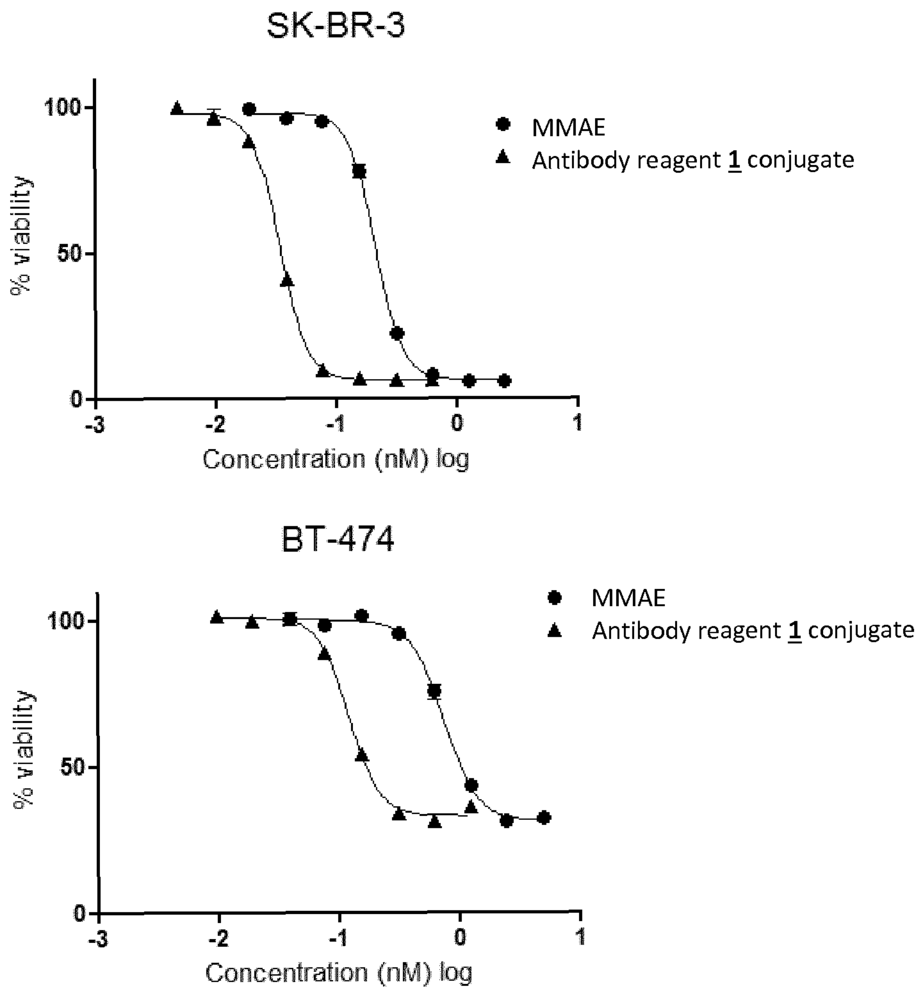
SK-BR-3
100
50
0
% viability
-3
-2
-1
0
1
Concentration (nM) log
MMAE
Antibody reagent 1 conjugate
BT-474
100
50
0
% viability
-3
-2
-1
0
1
Concentration (nM) log
MMAE
Antibody reagent 1 conjugate

Figure 6

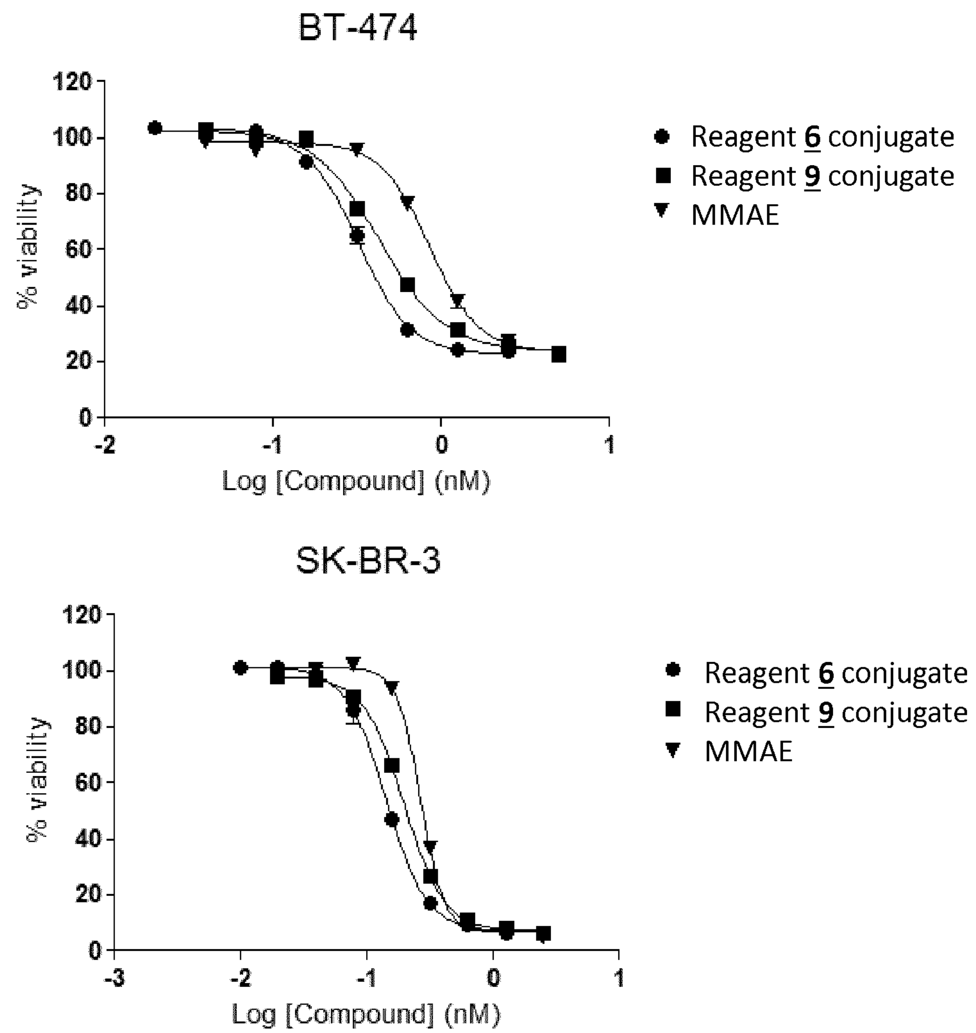
BT-474
120
100
80
60
40
20
0
% viability
-2
-1
0
1
Log [Compound] (nM)
Reagent 6 conjugate
Reagent 9 conjugate
MMAE
SK-BR-3
120
100
80
60
40
20
0
% viability
-3
-2
-1
0
1
Log [Compound] (nM)
Reagent 6 conjugate
Reagent 9 conjugate
MMAE

Figure 7

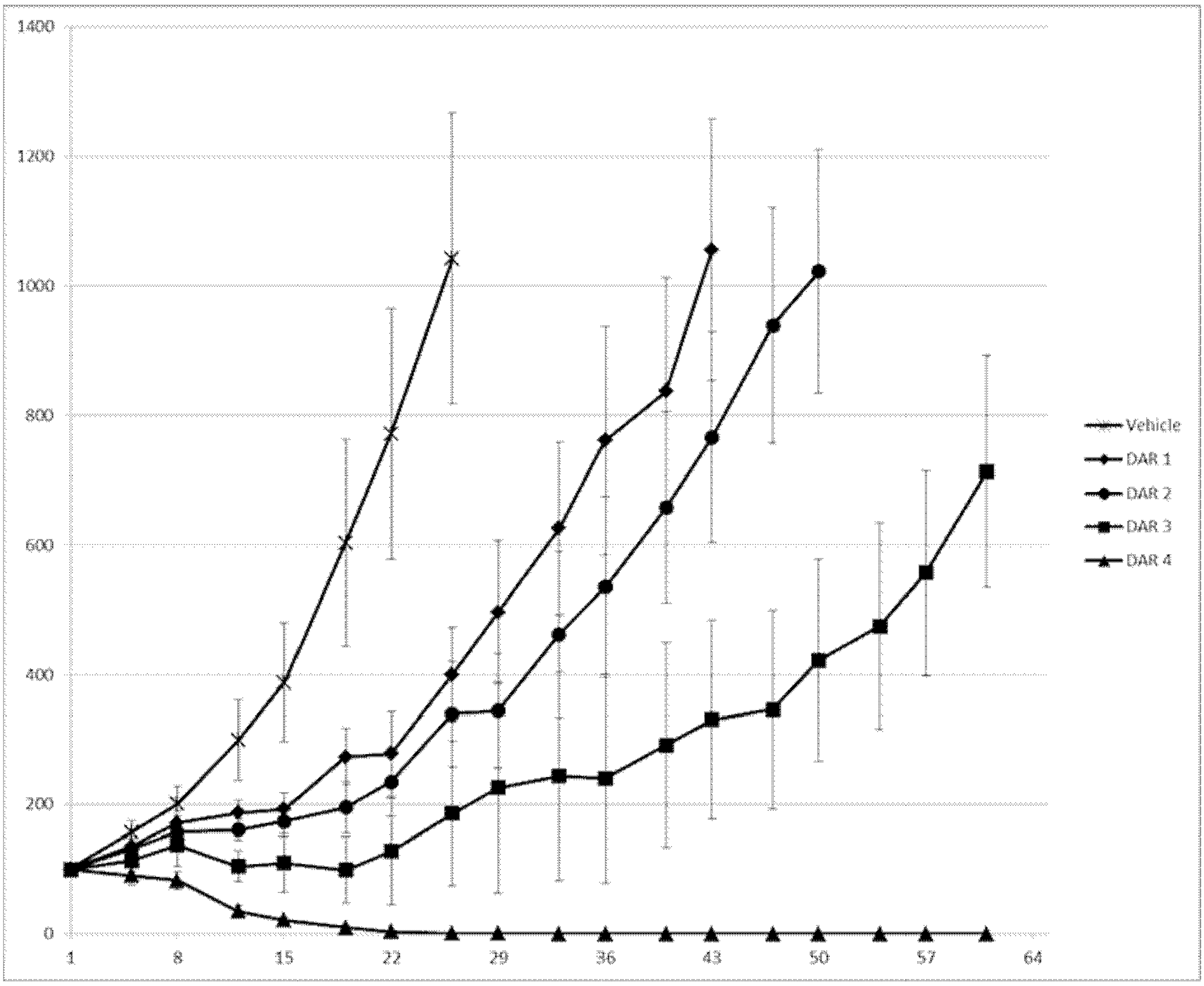
1400
1200
1000
800
600
400
200
0
1
8
15
22
29
36
43
50
57
64
Vehicle
DAR 1
DAR 2
DAR 3
DAR 4

Figure 8

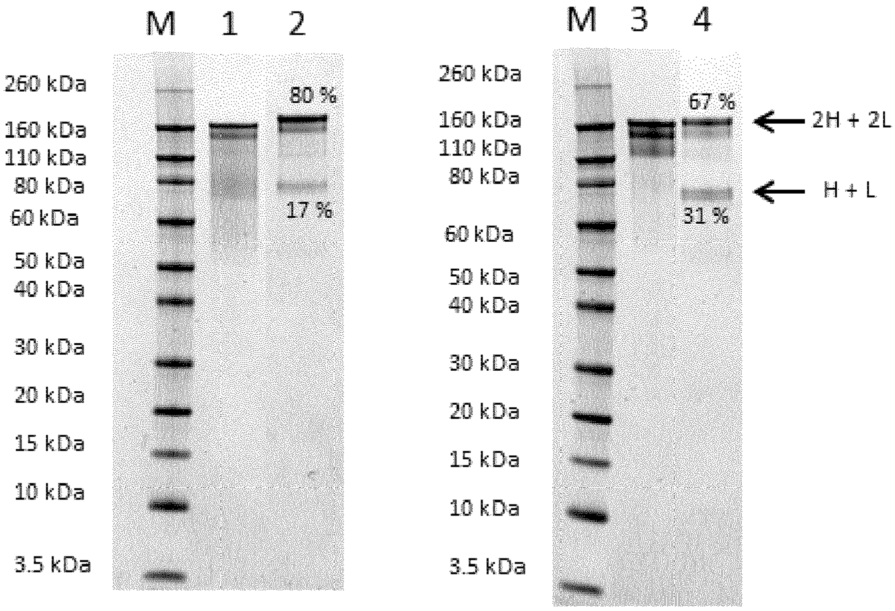
M
1
2
260 kDa
160 kDa
110 kDa
80 kDa
60 kDa
50 kDa
40 kDa
30 kDa
20 kDa
15 kDa
10 kDa
3.5 kDa
80 %
17 %
M
3
4
260 kDa
160 kDa
110 kDa
80 kDa
60 kDa
50 kDa
40 kDa
30 kDa
20 kDa
15 kDa
10 kDa
3.5 kDa
67 %
31 %
2H + 2L
H + L

Figure 9

Europäisches Patentamt
European Patent Office
Office européen des brevets

# EUROPEAN SEARCH REPORT

Application Number
EP 16 20 3350

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 354 163 A2 (MEDAREX INC [US]) 10 August 2011 (2011-08-10) * paragraphs [0043], [0198], [0199], [0245] - [0258] * ----- | 1-21 | INV. A61K47/68 |
| A,D | WO 2009/052431 A2 (SEATTLE GENETICS INC [US]; MCDONAGH CHARLOTTE [US]; CERVENY CHARLES G) 23 April 2009 (2009-04-23) * paragraphs [0233], [0333] * ----- | 1-21 | |
| A | WO 2009/134976 A1 (IMMUNOGEN INC [US]; SINGH RAJEEVA [US]; KOVTUN YELENA [US]; WILHELM SH) 5 November 2009 (2009-11-05) * paragraphs [0084] - [0094] * * figures 1-20 * ----- | 1-21 | |
| A | WO 2011/039721 A1 (SANOFI AVENTIS [FR]; BOUCHARD HERVE [FR]; COMMERCON ALAIN [FR]; FROMON) 7 April 2011 (2011-04-07) * page 1, line 5 - page 3, line 15 * * page 6, line 10 - page 8, line 18 * * examples 1-3 * ----- | 1-21 | TECHNICAL FIELDS SEARCHED (IPC) |
| A | HANIEH KHALILI ET AL: "Comparative Binding of Disulfide-Bridged PEG-Fabs", BIOCONJUGATE CHEMISTRY, vol. 23, no. 11, 21 September 2012 (2012-09-21), pages 2262-2277, XP055096650, ISSN: 1043-1802, DOI: 10.1021/bc300372r * whole document and especially: abstract; page 2262-2264, "Introduction"; Schemes 1-3 * ----- | 1-21 | A61K |

The present search report has been drawn up for all claims

1

| Place of search | Date of completion of the search | Examiner |
|---|---|---|
| The Hague | 14 March 2017 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 20 3350

This annex lists the patent family members relating to the patent documents cited in the above-mentioned European search report. The members are as contained in the European Patent Office EDP file on
The European Patent Office is in no way liable for these particulars which are merely given for the purpose of information.

14-03-2017

| Patent document cited in search report | Publication date | Patent family member(s) | Publication date |
|---|---|---|---|
| EP 2354163 A2 | 10-08-2011 | AU 2006294554 A1 | 05-04-2007 |
| | | BR PI0617546 A2 | 26-07-2011 |
| | | CA 2623652 A1 | 05-04-2007 |
| | | CN 101312748 A | 26-11-2008 |
| | | EA 200800952 A1 | 27-02-2009 |
| | | EP 1940470 A2 | 09-07-2008 |
| | | EP 2354163 A2 | 10-08-2011 |
| | | ES 2416136 T3 | 30-07-2013 |
| | | HK 1117410 A1 | 24-01-2014 |
| | | IL 190187 A | 31-05-2015 |
| | | JP 5290756 B2 | 18-09-2013 |
| | | JP 2009509977 A | 12-03-2009 |
| | | KR 20080057310 A | 24-06-2008 |
| | | NZ 566982 A | 30-06-2011 |
| | | US 2008279868 A1 | 13-11-2008 |
| | | WO 2007038658 A2 | 05-04-2007 |
| | | ZA 200803153 B | 25-02-2009 |
| WO 2009052431 A2 | 23-04-2009 | AU 2008311815 A1 | 23-04-2009 |
| | | CA 2702555 A1 | 23-04-2009 |
| | | CN 101903403 A | 01-12-2010 |
| | | DK 2211904 T3 | 24-10-2016 |
| | | EP 2211904 A2 | 04-08-2010 |
| | | ES 2599172 T3 | 31-01-2017 |
| | | HR P20161345 T1 | 18-11-2016 |
| | | JP 5727786 B2 | 03-06-2015 |
| | | JP 2011500725 A | 06-01-2011 |
| | | JP 2015129131 A | 16-07-2015 |
| | | KR 20100097655 A | 03-09-2010 |
| | | LT 2211904 T | 25-11-2016 |
| | | RU 2010115886 A | 27-10-2011 |
| | | SI 2211904 T1 | 30-12-2016 |
| | | US 2009136526 A1 | 28-05-2009 |
| | | US 2011312088 A1 | 22-12-2011 |
| | | US 2012294853 A1 | 22-11-2012 |
| | | US 2015283255 A1 | 08-10-2015 |
| | | WO 2009052431 A2 | 23-04-2009 |
| WO 2009134976 A1 | 05-11-2009 | AU 2009243009 A1 | 05-11-2009 |
| | | CA 2722109 A1 | 05-11-2009 |
| | | CN 102083461 A | 01-06-2011 |
| | | CN 104258413 A | 07-01-2015 |
| | | EP 2276506 A1 | 26-01-2011 |
| | | JP 5980989 B2 | 31-08-2016 |
| | | JP 2011523628 A | 18-08-2011 |
| | | JP 2015163622 A | 10-09-2015 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 16 20 3350

This annex lists the patent family members relating to the patent documents cited in the above-mentioned European search report. The members are as contained in the European Patent Office EDP file on
The European Patent Office is in no way liable for these particulars which are merely given for the purpose of information.

14-03-2017

| Patent document cited in search report | Publication date | Patent family member(s) | Publication date |
|---|---|---|---|
| | | KR 20100137585 A | 30-12-2010 |
| | | NZ 588851 A | 31-05-2013 |
| | | RU 2010148740 A | 10-06-2012 |
| | | SG 189817 A1 | 31-05-2013 |
| | | UA 108598 C2 | 25-05-2015 |
| | | US 2010129314 A1 | 27-05-2010 |
| | | US 2012226026 A1 | 06-09-2012 |
| | | US 2016114052 A1 | 28-04-2016 |
| | | WO 2009134952 A2 | 05-11-2009 |
| | | WO 2009134976 A1 | 05-11-2009 |
| | | ZA 201007806 B | 30-01-2013 |
| WO 2011039721 A1 | 07-04-2011 | AR 078471 A1 | 09-11-2011 |
| | | AU 2010302247 A1 | 26-04-2012 |
| | | BR 112012007305 A2 | 06-12-2016 |
| | | CA 2774916 A1 | 07-04-2011 |
| | | CN 102741260 A | 17-10-2012 |
| | | CR 20120147 A | 01-06-2012 |
| | | EA 201270473 A1 | 28-02-2013 |
| | | EC SP12011756 A | 31-07-2012 |
| | | EP 2483279 A1 | 08-08-2012 |
| | | JP 2013506653 A | 28-02-2013 |
| | | KR 20120091166 A | 17-08-2012 |
| | | MA 33702 B1 | 01-10-2012 |
| | | NZ 599045 A | 27-09-2013 |
| | | PE 15342012 A1 | 03-12-2012 |
| | | TW 201117814 A | 01-06-2011 |
| | | US 2012276124 A1 | 01-11-2012 |
| | | UY 32913 A | 29-04-2011 |
| | | WO 2011039721 A1 | 07-04-2011 |
| | | ZA 201202328 B | 25-06-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005007197 A
- WO 2009117531 A
- WO 2009052431 A
- WO 2010100430 A
- WO 2005081711 A


### Non-patent literature cited in the description


- **LIBERATORE et al.** *Bioconj. Chem,* 1990, vol. 1, 36-50
- **DEL ROSARIO et al.** *Bioconj. Chem.,* 1990, vol. 1, 51-59
- **CARTER P et al.** *Endocr. Relat. Cancer.,* December 2004, vol. 11 (4), 659-87
- **LIU et al.** *Anal. Chem.,* 2010, vol. 82, 5219-5226
- *Nature Protocols,* 2006, vol. 1 (54), 2241-2252
- **CARTER P. et al.** *Proc. Natl Acad. Sci. USA,* 1992, vol. 89, 4285-4289
- **HO et al.** *Gene,* 1989, vol. 77, 51-59
- Transient Expression in HEK293-EBNA1 Cells. Expression Systems. Scion Publishing Ltd, 2007
- **LEWIS PHILLIPS G.D.** *Cancer Res,* 2008, vol. 68, 9280-9290
- **R. IAN FRESHNEY.** Culture of Animal Cells: A Manual of Basic Technique. Alan R. Liss, 1994
- CULTURE OF ANIMAL CELLS: A MANUAL OF BASIC TECHNIQUE. Wiley-Liss, 2005